# EUROPEAN PATENT SPECIFICATION

(11) **EP 2 532 750 B1**
(45) Date of publication and mention of the grant of the patent: **27.04.2016**
(21) Application number: 12183100.2
(22) Date of filing: 29.06.2006
(51) Int. Cl.: C12N 15/82, A01H 5/00, C12N 9/88

(54) **Herbicide-resistant sunflower plants, polynucleotides encoding herbicide-resistant acetohydroxyacid synthase large subunit proteins, and methods of use**
Herbizidresistente Sonnenblumenpflanzen, für große herbizidresistente Acetohydroxysäuresynthase-Untereinheitenproteine codierende Polynucleotide und Verwendungsverfahren
Plantes de tournesol résistantes aux herbicides, polynucléotides codant pour la résistance aux herbicides, protéines à grandes sous-unités d'acétohydroxyacide synthase et procédés d'utilisation

(30) Priority: 01.07.2005 US 695952 P
(43) Date of publication of application: 12.12.2012
(62) Divisional of application: 11180363.1
(73) Proprietor: BASF SE, 67056 Ludwigshafen am Rhein (DE); Nidera S.A., Buenos Aires (AR)
(72) Inventor: Sala, Carlos Alberto, 2600 Santa Fe (AR); Echarte, Adriana Mariel, 2600 Santa Fe (AR); Bulos, Mariano, 2600 Santa Fe (AR); Whitt, Sherry R., Raleigh, NC North Carolina 27614 (US); Ascenzi, Robert, Cary, NC North Carolina 27511 (US)
(74) Representative: Hoffmann Eitle

(56) References cited:
- WO-A-2005/020673
- WO-A-2006/024351
- WO-A-2006/060634
- US-A1- 2003 097 692
- WHITE A D ET AL: "ISOLATION OF ACETOLACTATE SYNTHASE HOMOLOGS IN COMMON SUNFLOWERS", WEED SCIENCE, WEED SCIENCE SOCIETY OF AMERICA, CHAMPAIGN, IL, US, vol. 51, no. 6, November 2003 (2003-11), pages 845-853, XP009058786, ISSN: 0043-1745
- MILLIMAN LESLIE D ET AL: "CHARACTERIZATION OF TWO BIOTYPES OF IMIDAZOLINONE-RESISTANT EASTERN BLACK NIGHTSHADE (SOLANUM PTYCANTHUM)", WEED SCIENCE, WEED SCIENCE SOCIETY OF AMERICA, CHAMPAIGN, IL, US, vol. 51, no. 2, 10 March 2003 (2003-03-10) , pages 139-144, XP009078200, ISSN: 0043-1745
- KOLKMAN JUDITH M ET AL: "Acetohydroxyacid synthase mutations conferring resistance to imidazolinone or sulfonylurea herbicides in sunflower.", TAG. THEORETICAL AND APPLIED GENETICS. THEORETISCHE UND ANGEWANDTE GENETIK OCT 2004, vol. 109, no. 6, October 2004 (2004-10), pages 1147-1159, XP002417954, ISSN: 0040-5752
- AL-KHATIB K ET AL: "IMAZETHAPYR RESISTANCE IN COMMON SUNFLOWER (HELIANTHUS ANNUUS)", WEED SCIENCE, WEED SCIENCE SOCIETY OF AMERICA, CHAMPAIGN, IL, US, vol. 46, no. 4, 1998, pages 403-407, XP001024427, ISSN: 0043-1745

## Description

### FIELD OF THE INVENTION

This invention relates to the field of agricultural biotechnology, particularly to herbicide-resistant sunflower plants and novel polynucleotide sequences that encode herbicide-resistant sunflower acetohydroxyacid synthase large subunit proteins.

### BACKGROUND OF THE INVENTION

Acetohydroxyacid synthase (AHAS; EC 4.1.3.18, also known as acetolactate synthase or ALS), is the first enzyme that catalyzes the biochemical synthesis of the branched chain amino acids valine, leucine and isoleucine (sough (1999) "Biosynthesis of valine, leucine and isoleuane," in Plant Amino Acid, Singh, B.K., ed., Marcel Dekker Inc. New York, New York, pp. 227-247). AHAS is the site of action of five structurally diverse herbicide families including the sulfonylureas (Tan et al. (2005) Pest Manag. Sci. 61:246-57; Mallory-Smith and Retzinger (2003) Weed Technology 17:620-626; LaRossa and Falco (1984) Trends Biotechnol. 2:158-161), the imidazolinones (Shaner et al. (1984) Plant Physiol. 76:545-546), the triazolopyrimidines (Subramanian and Gerwick (1989) "Inhibition of acetolactate synthase by triazolopyrimidines," in Biocatalysis in Agricultural Biotechnology, Whitaker, J.R. and Sonnet, P.E.. eds., ACS Symposium Series, American Chemical Society, Washington, D.C., pp. 277-288), the pyrimidinyloxybenzoates (Subramanian et al. (1990) Plant Physiol. 94: 239-244) and the sulfonylamino-carbonyltriazolinones (Tan et al. (2005) Pest Manag. Sci. 61:246-57; Mallory-Smith and Retzinger (2003) Weed Technology 17:620-626). Imidazolinone and sulfonylurea herbicides are widely used in modern agriculture due to their effectiveness at very low application rates and relative non-toxicity in animals. By inhibiting AHAS activity, these families of herbicides prevent further growth and development of susceptible plants including many weed species. Several examples of commercially available imidazolinone herbicides are PURSUIT® (imazethapyr), SCEPTER® (imazaquin) and ARSENAL® (imazapyr). Examples of sulfonylurea herbicides are chlorsulfuron, metsulfuron methyl, sulfometuron methyl, chlorimuron ethyl, thifensulfuron methyl, tribenuron methyl, bensulfiiron methyl, nicosulfuron, ethametsulfuron methyl, rimsulfuron, triflusulfuron methyl, triasulfiuron, primisulfuron methyl, cinosulfuron, amidosulfuron, flazasulfuron, imazosulfuron, pyramsulfuron ethyl and halosulfluron.

Due to their high effectiveness and low-toxicity, imidazolinone herbicides are favored for application by spraying over the top of a wide area of vegetation. The ability to spray a herbicide over the top of a wide range of vegetation decreases the costs associated with plant establishment and maintenance and decreases the need for site preparation prior to use of such chemicals. Spraying over the top of a desired tolerant species also results in the ability to achieve maximum yield potential of the desired species due to the absence of competitive species. However, the ability to use such spray-over techniques is dependent upon the presence of imidazolinone-resistant species of the desired vegetation in the spray over area.

Among the major agricultural crops, some leguminous species such as soybean are naturally resistant to imidazolinone herbicides due to their ability to rapidly metabolize the herbicide compounds (Shaner and Robinson (1985) Weed Sci. 33:469-471). Other crops such as corn (Newhouse et al. (1992) Plant Physiol. 100:882-886) and rice (Barrett et al. (1989) Crop Safeners for Herbicides, Academic Press, New York, pp. 195-220) are somewhat susceptible to imidazolinone herbicides. The differential sensitivity to the imidazolinone, herbicides is dependent on the chemical nature of the particular herbicide and differential metabolism of the compound from a toxic to a non-toxic form in each plant (Shaner et al. (1984) Plant Physiol. 76:545-546; Brown at al., (1987) Pestic. Biochem. Physiol. 27:24-29). Other plant physiological differences such as absorption and translocation also play an important role in sensitivity (Shaner and Robinson (1985) Weed Sci. 33:469-471).

Plants resistant to imidazolinone, sulfonylureas, triazolopyrimidines, and pyrimidinyloxybenzoates have been successfully produced using seed, microspore, pollen, and callus mutagenesis in *Zea mays, Arabidopsis thaliana, Brassica napus* (*i.e.,* canola) *Glycine max, Nicotiana tabacum,* sugarbeet (*Beta vulgaris)* and *Oryza satitva* (Sebastian et al (1989) Crop Sci. 29:1403-1408; Swanson et al., 1989 Theor Appl. Ganet. 78:525-530; Newhouse et al. (1991) Theor. Appl. Genet. 83:65-70; Sathasivan et a/. (1991) Plant Physiol. 97:1044-1050; Mourand et al. (1993) J. Heredity 84:91-96; Wright and Penner (1998) Theor. Appl. Genet. 96:612-620; U.S. Patent No. 5,545,822). In all cases, a single, partially dominant nuclear gene conferred resistance. Four imidazolinone resistant wheat plants were also previously isolated following seed mutagenesis of *Triticum aestivum* L. cv. Fidel (Newhouse et al. (1992) Plant Physiol. 100:882-886). Inheritance studies confirmed that a single, partially dominant gene conferred resistance. Based on allelic studies, the authors concluded that the mutations in the four identified lines were located at the same locus. One of the Fidel cultivar resistance genes was designated FS-4 (Newhouse et al. (1992) Plant Physiol. 100:882-886).

Naturally occurring plant populations that were discovered to be resistant to imidazolinone and/or sulfonylurea herbicides have also been used to develop herbicide-resistant sunflower breeding lines. Recently, two sunflower lines that are resistant to a sulfonylurea herbicide were developed using germplasm originating from a wild population of common sunflower (*Helianthus annuus*) as the source of the herbicide-resistance trait (Miller and Al-Khatib (2004) Crop Sci. 44:1037-1038). Previously, White et al. ((2002) Weed Sci. 50:432-437) had reported that individuals from a wild population of common sunflower from South Dakota, U.S.A were cross-resistant to an imidazolinone and a sulfonylurea herbicide. Analysis of a portion of the coding region of the acetohydroxyacid synthase large subunit (*AHASL*) genes of individuals from this population revealed a point mutation that results in an Ala-to-Val amino acid substitution in the sunflower AHASL protein that corresponds to Ala₂₀₅ in the wild-type *Arabidopsis thaliana* AHASL protein (White et al. (2003) Weed Sci. 51:845-853). Earlier, Al-Khatib and Miller ((2000) Crop Sci 40:869) reported the production of four imidazolinone-resistant Sunflower breeding lines.

Computer-based modeling of the three dimensional conformation of the AHAS-inhibitor complex predicts several amino acids in the proposed inhibitor binding pocket as sites where induced mutations would likely confer selective resistance to imidazolinone (Ott et al. (1996) J. Mol. Biol. 263:359-368). Tobacco plants produced with some of these rationally designed mutations in the proposed binding sites of the AHAS enzyme have in fact exhibited specific resistance to a single class of herbicides (Ott et al. (1996) J. Mol. Biol. 263:359-368).

Plant resistance to imidazolinone herbicides has also been reported in a number of patents. U.S. Patent Nos. 4,761,373, 5,331,107, 5,304,732, 6,211,438, 6,211,439 and 6,222,100 generally describe the use of an altered AHAS gene to elicit herbicide resistance in plants, and specifically discloses certain imidazolinone, resistant corn lines. U.S. Patent No. 5,013,659 discloses plants exhibiting herbicide resistance due to mutations in at least one amino acid in one or more conserved regions. The mutations described therein encode either cross-resistance for imidazolinones and sulfonylureas or sulfonylurea-specific resistance, but imidazolinone-specific resistance is not described. U.S. Patent No. 5,731,180 and U.S. Patent No. 5,767,361 discuss an isolated gene having a single amino acid substitution in a wild-type monocot AHAS amino acid sequence that results in imidazolinone-specific resistance. In addition, rice plants that are resistant to herbicides that interfere with AHAS have been developed by mutation breeding and also by the selection of herbicide-resistant plants from a pool of rice plants produced by anther culture. *See,* U.S. Patent Nos. 5,545,822, 5,736,629, 5,773,703, 5,773,704, 5,952,553 and 6,274,796.

In plants, as in all other organisms examined, the AHAS enzyme is comprised of two subunits: a large subunit (catalytic role) and a small subunit (regulatory role) (Duggleby and Pang (2000) J. Biochem. Mol. Biol. 33:1-36). The AHAS large subunit (also referred to herein as AHASL) may be encoded by a single gene as in the case of *Arabidopsis,* and sugar beet or by multiple gene family members as in maize, canola, and cotton. Specific single-nucleotide substitutions in the large subunit confer upon the enzyme a degree of insensitivity to one or more classes of herbicides (Chang and Duggleby (1998) Biochem J. 333:765-777).

For example, bread wheat, *Triticum aestivum* L., contains three homoeologous acetohydroxyacid synthase large subunit genes. Each of the genes exhibit significant expression based on herbicide response and biochemical data from mutants in each of the three genes (Ascenzi et al. (2003) International Society of Plant Molecular Biologists Congress, Barcelona, Spain, Ref. No. S10-17). The coding sequences of all three genes share extensive homology at the Nucleotide level (WO 03/014357). Through sequencing the *AHASL* genes from several varieties of *Triticum aestivum*, the molecular basis of herbicide tolerance in most IMI tolerant (imidazolinone-tolerant) lines was found to be the mutation S653(*At*)N, indicating a serine to asparagine substitution at a position equivalent to the serine at amino add 653 in *Arabidopsis thaliana* (WO 03/01436; WO 03/014357). This mutation is due to a single nucleotide polymorphism (SNP) in the DNA sequence encoding the AHASL protein.

Multiple *AHASL* genes are also know to occur in dicotyledonous plants species. Recently, Kolkman et al. ((2004) Theor. Appl. Genet. 109: 1147-1159) reported the identification, cloning, and sequencing for three *AHASL* genes (*AHASL1, AHASL2,* and *AHASL3)* from herbicide-resistant and wild type genotypes of sunflower (*Helianthus annuus* L.). Kolkman *et al.* reported that the herbicide-resistance was due either to the Pro197Leu (using *the Arabidopsis* AHASL amino acid position nomenclature) substitution or the Ala205Val substitution in the AHASL1 protein and that each of these substitutions provided resistance to both imidazolinone and sulfonylurea herbicides.

Given their high effectiveness and low-toxicity, imidazolinone herbicides are favored for agricultural use. However, the ability to use imidazolinone herbicides in a particular crop production system depends upon the availability of imidazolinone-resistant varieties of the crop plant of interest. To produce such imidazolinone-resistant varieties, plant breeders need to develop breeding lines with the imidazoinone-resistance trait. Thus, additional imidazolinone-resistant breeding lines and varieties of crop plants, as well as methods and compositions for the production and use of imidazolinone-resistant breeding lines and varieties, are needed.

### SUMMARY OF THE INVENTION

The present invention is defined in the claims.

As set forth in claim 1, the invention provides a sunflower acetohydroxyacid synthase large subunit (*AHASL*) polynucleotide, wherein said *AHASL* polynucleotide encodes an imidazolinone herbicide-tolerant AHASL1 protein comprising a substitution from alanine to threonine at amino acid position 7 of SEQ ID NO:2 or, equivalently, at position 107 of SEQ ID NO:12; wherein said AHASL1 protein comprises a polypeptide that is at least 95% identical to SEQ ID NO:12; and wherein said AHASL1 protein confers upon a plant increased tolerance to an imidazolinone herbicide as compared to a wild-type variety of said plant.

Further aspects of the invention are defined in the other claims.

The following description sets out details relating to both embodiments covered by the claims and subject matter not covered by the claims. The description of subject matter not covered by the claims is disclosed for information only.

Disclosed herein are sunflower plants having increased resistance to herbicides when compared to a wild-type sunflower plant. In particular, the sunflower plants of the disclosure have increased resistance to imidazolinone herbicides, when compared to a wild-type sunflower plant. The herbicide-resistant sunflower plants of the disclosure comprise at least one copy of a gene or polynucleotide that encodes a herbicide-resistant acetohydroxyacid synthase large subunit (AHASL). Such a herbicide-resistant AHASL protein comprises a threonine at amino acid position 107 or equivalent position. A herbicide-resistant sunflower plant of the disclosure can contain one, two, three, four, five, six, or more copies of a gene or polynucleotide encoding a herbicide-resistant AHASL protein of the disclosure. The sunflower plants of the disclosure also include seeds and progeny plants that comprise at least one copy of a gene or polynucleotide encoding a herbicide-resistant AHASL protein of the disclosure.

The present: disclosure describes herbicide-resistant sunflower plants of a. sunflower line that is designated herein as S4897 and progeny and derivatives thereof that comprise the herbicide resistance characteristics of S4897. A genetic line designated herein as GM40 was derived from, and possesses the herbicide resistance characteristics of S4897. A sample of seeds of the GM40 line has been deposited with the American Type Culture Collection (ATCC) as ATCC Patent Deposit No. PTA-6716. Thus, a sunflower plant of the disclosure that comprises the herbicide resistance characteristics of GM40 or a sunflower plant having ATCC Patent Deposit No. PTA-6716 also comprises the herbicide resistance characteristics of S4897. S4897 sunflower plants, GM40 sunflower plants, and sunflower plants having ATCC Patent Deposit No. PTA-6716 and progeny and derivatives thereof comprising the herbicide resistance characteristics of S4897, GM40, or a sunflower plant having ATCC Patent Deposit No. PTA-6716, comprise in their genomes *an AHASL1* gene that comprises the nucleotide sequence set forth in SEQ ID NO: 1 and that encodes the AHASL1 protein comprising, the amino acid sequence set forth in SEQ ID NO: 2. When compared to the amino acid sequence of the AHASL1 protein (SEQ ID NO: 4) that is encoded by an *AHASL1* gene (SEQ ID NO: 3) from a wild-type sunflower plant, the amino acid sequence set forth in SEQ ID NO: 2 has a single amino acid difference from the wild-type amino acid sequence. In the amino acid sequence set forth in SEQ ID NO: 2, there is threonine at amino acid position 7. This position corresponds to position 107 in the full-length sunflower AHASL1 protein encoded by the nucleotide sequence set forth in SEQ ID NO: 12 (Accession No. AY541451). In the wild-type AHASL1 amino acid sequence of the disclosure (SEQ ID NO: 4), this equivalent amino acid position has an alanine. Unless otherwise indicated, the amino acid positions referred to herein for sunflower AHASL proteins correspond to the amino acid positions of the full-length amino acid sequence set forth in SEQ ID NO: 12.

The present disclosure includes herbicide-resistant sunflower plants that are sunflower line that is designated herein as GM1606. A sample of seeds of genetic material of GM1606 has been deposited with the ATCC as ATCC Patent Deposit No. PTA-7606. Thus, the present disclosure discloses herbicide-resistant sunflower plants that having ATCC Patent Deposit No. PTA-7606 and progeny and derivatives thereof that comprise the herbicide resistance characteristics of the sunflower plants having ATCC Patent Deposit No. PTA-7606. Like the S4897 and M40 sunflower plants, M1606 and progeny and derivatives thereof comprising the herbicide resistance characteristics of GM1606 comprise in their genomes an *AHASL1* gene encodes an AHASL1 protein comprising a threonine at position 107 in the full-length sunflower AHASL1 protein. Similarly, sunflower, plants having ATCC Patent Deposit No. PTA-7606, and progeny and deriviatives thereof comprising the herbicide resistance characteristics of the sunflower plants having ATCC Patent Deposit No. PTA-7606 comprise in their genomes an *AHASL1* gene encodes an AHASL1 protein comprising a threonine at position 107 in the full-length sunflower AHASL1 protein.

The present disclosure further describes isolated polynucleotides and isolated polypeptides for sunflower (*Helianthus annus*) AHASL proteins. The polynucleotides herein encompass nucleotide sequences that encode herbicide-resistant and wild-type AHASL proteins, including, but not limited to, the proteins encoded by the sunflower *AHASL1, AHASL2,* and AHASL3 genes. The herbicide-resistant sunflower AHASL proteins include imidazolinone-resistant AHASL1 proteins that comprise threonine at position 107 of a full-length sunflower AHASL1 protein or equivalent position.

The polynucleotides of the disclosure encompass the nucleotide sequences set forth in SEQ ID NOS: 1 and 3, nucleotide sequences encoding the amino acid sequences set forth in SEQ ID NOS: 2 and 4, and fragments and variants of said nucleotide sequences that encode proteins comprising AHAS activity.

The present disclosure describes expression cassettes for expressing the polynucleotides of the: disclosure in plants, plant cells, and other, non-human host cells. The expression cassettes comprise a promoter expressible in the plant, plant cell, or other host cells of interest operably linked to a polynucleotide of the disclosure that encodes either a wild-type or herbicide-resitant AHASL protein. If necessary for targeting expression to the chloroplast, the expression cassette can also comprise an operably linked chloroplast-targeting sequence that encodes of a chloroptast transit peptide to direct an expressed AHASL protein to the chloroplast The expression cassettes of the disclosure find use in a method for enhancing the herbicide tolerance of a plant and a host cell. The method involves transforming the plant or host cell with an expression cassette of the disclosure, wherein the expression cassette comprises a promoter that is expressible in the plant or host cell of interest and the promoter is operably linked to a polynucleotide of the disclosure that encodes an herbicide-resistant AHASL protein of the disclosure. The method further comprises regenerating a transformed plant from the transformed plant cell.

The present disclosure describes a method for increasing AHAS activity in a plant comprising transforming a plant cell with a polynucleotide construct comprising a nucleotide sequence operably linked to a promoter that drives expression in a plant cell and regenerating a transformed plant from the transformed plant cell. The nucleotide sequence is selected from those nucleotide sequences that encode the herbicide-resistant or wild-type AHASL proteins of the disclosure, particularly the nudeotide sequences set forth in SEQ ID NOS: 1 and 3, and nucleotide sequence encoding the amino acid sequences set forth in SEQ ID NOS: 2, and 4, and fragments and variants thereof. A plant produced by this method comprises increased AHAS activity or increased herbicide-resistant ALAS activity, when compared to an untransformed plant.

The present disclosure describes a method for producing a herbicide-resistant plant comprising transforming a plant cell with a polynucleotide construct comprising a nucleotide sequence operably linked to a promoter that drives expression in a plant cell and regenerating a transformed plant from said transformed plant cell. The nucleotide sequences is selected from those nucleotide sequences that encode the herbicide-resistant AHASL proteins of the disclosure, particularly the nudeodde sequence set forth in SEQ ID NO: 1, the nucleotide sequence encoding the amino acid sequence set forth in (SEQ ID NO: 2. and fragments and variants thereof. A herbicide-resistant plant produced by this method comprises enhanced resistance to at least one herbicides, particularly an imidazolinone harbiride, when compared to an untransformed plant.

The present disclosure describes a method for enhancing herbicide-tolerance is a herbicide-tolerant plant The methods finds use in enhancing the resistance, of a plant that already is resistant to a level of a herbicide that would kill or significantly injure a wild-type plant. Such a babicide-tolermt plant can be a herbicide-tolerant plant that has been genetically engineered for herbicide-tolerance or a herbicide-tolerant plant that was developed by means that do not involve recombinant DNA such as, for example, the S4897, GM40, and CM1606 sunflower plants of the present: disclosure. The method comprises transforming a herbicide-tolerant plant with a polynucleotide construct comprising a nucleotide sequence operably linked to a promoter that drives expression in a plant cell and regenerating a transformed plant from the transformed plant cell. The nucleotide sequence is selected from those nucleotide sequences that encode the herbicide-resistant AHASL proteins of the disclosure particularly a nucleotide sequence comprising the nucleotide sequence set forth in SEQ ID NO: 1, nucleotide sequences encoding an amino acid sequence comprising the amino acid sequence set forth in SEQ ID NO: 2, and fragments and variants thereof.

The present disclosure describes transformation vectors comprising a selectable marker gene of the discloeare. The selectable marker gene comprises a promoter that drives expression in a host cell operably linked to a polynucleotide comprising a Nucleotide sequence that encodes an herbicide-resistant AHASL protein of the disclosure. The tranafarmation vector can additionally comprise a gene of interest to be expressed in the host cell and can also, if desired, include a chloroplast-targeting sequence that is operably linked to the polynucleotide of the invention.

The present: disclosure further describes methods for using the transformation vectors of the disclosure to select for cells transformed with the gene of interest. Such methods involve the transformation of a host cell with the transformation vector, exposing the cell to a level of an imidazolinone herbicide that would kill or inhibit the growth of a non-transformed host cell, and identifying the transformed host cell by its ability to grow in the presence of the herbicide. The host cell may be a plant cell and the selectable marker gene comprises a promoter that drives expression in a plant cell.

The present: disclosure describes a method for controlling weeds in the vicinity of the herbicide-resistant plants of the disclosure, including the herbicide-resistant sunflower plants described above and plants transformed with the herbicide-resistant AHASL polynucleotides of the disclosure. Such transformed plants comprise in their genomes at least one expression cassette comprising a promoter that drives gene expression in a plant cell, wherein the promoter is operably linked to an AHASL polynucleotide of the disclosure. The method comprises applying an effective amount of an herbicide to the weeds and to the herbicide-resistant plant, wherein the herbicide-resistant plant has increased resistance to at least one herbicide, particularly an imidazolinone, herbicide, when compared to a wild-type or untransformed plant.

The plants of the present disclosure can be transgenic or non-transgenic. An example of a non-transgenic sunflower plant having increased resistance to imidazolinone and/or sulfonylurea herbicides includes S4897, GM40, or GM1606 sunflower plants and sunflower plants having ATCC Patent Deposit No. PTA-6716 or PTA-7646; or mutant, recombinant, or a genetically engineered derivative of and S4897, GM40, or GM1606, the sunflower plant having ATCC Patent Deposit No. PTA-6716 or PTA-7606, or two of more of S4897, GM40, M1606, the sunflower plant having ATCC Patent Deposit No. Pita-6716, and the sunflower plant having ATCC Patent Deposit No. PTA-7606; or of any progeny of S4897, GM40, or GM1606, the sunflower plant having ATCC Patent Deposit No. PTA-6716 or PTA-7606, or two of more of S4897, GM40, GM1606, the sunflower plant having ATCC Patent Deposit No. PTA-6716, and the sunflower plant having ATCC Patent Deposit No. PTA-7606; or a plant that is a progeny of any of these plants; or a plant that comprises the herbicide resistance characteristics of S4897, GM40, GM1606, the sunflower plant having ATCC Patent Deposit No. PTA-6716, and/or the sunflower plant having ATCC Patent Deposit No. PTA-7606.

The present disclosure also includes plants, plant organs, plant tissues, plant cells, seeds, and non-human host cells that are transformed with the at least one polynucleotide, expression cassette, or transformation vector of the disclosure. Such transformed plants, plant organs, plant tissues, plant cells, seeds, and non-human host cells have enhanced tolerance or resistance to at least one herbicide, at levels of the herbicide that kill or inhibit the growth of an untransformed plant, plant tissue, plant cell, or non-human host cell, respectively. The transformed plants, plant tissues, plant cells, and seeds of the disclosure may be *Arabidopsis thaliana,* sunflower, and other crop plants.

The present disclosure further describes isolated polypeptides comprising imidazolinone-resistant and wild-type sunflower AHASL proteins. The isolated polypeptides comprise the amino acid sequences set forth in SEQ ID NOS: 2 and 4, the amino acid sequences encoded by nucleotide sequences set forth in SEQ TOD NOS: 1 and 3, and fragments and variants of said amino acid sequences that encode proteins comprising AHAS activity.

### BRIEF DESCRIPTION THE DRAWINGS

Figure 1 is a nucleotide sequence alignment of the nucleotide sequences of the herbicide-resistant sunflower *AHASL1* gene (SEQ ID NO:1), the wild-type sunflower *AHASL1* gene (SEQ ID NO: 3), GenBank Accession No. U16280 (SEQ ID NO: 5), GenBank Accession No. AY541451 (SEQ ID NO: 11) and GenBank Accession No. AY124092 (SEQ ID NO: 13). The site of the mutation in (SEQ ID NO: 1) is indicated by an asterisk. The mutation is a G-to-A transition at nucleotide position 21 of SBQ ID NO: 1.

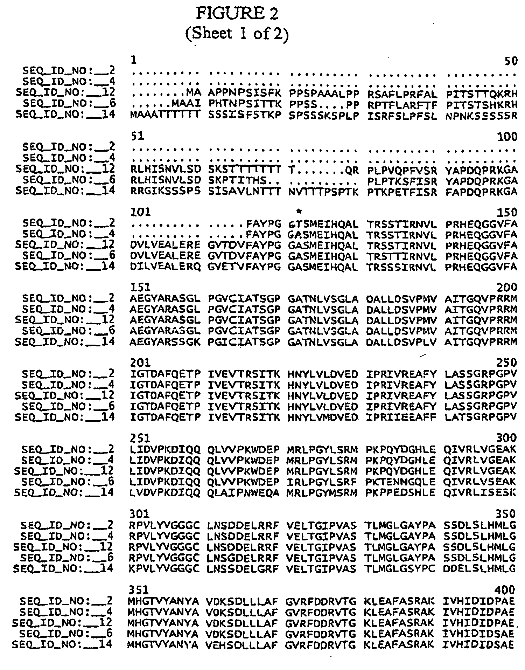

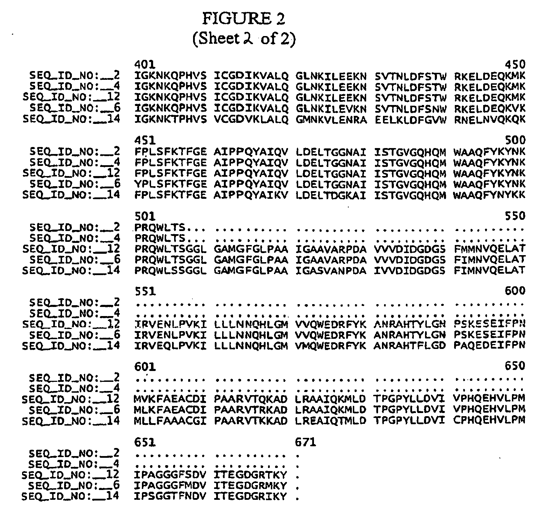

Figure 2 is an amino acid sequence alignment of the herbicide-resistant sunflower AHASL1 protein (SBQ ID NO: 2), the wild-type sunflower AHASL1 protein (SEQ ID NO: 4), GenBank Accession No. U16280 (SBQ ID NO: 6), GenBank Accession No. AY541451 (SEQ ID NO: 12), and GenBank Accession No. AY124092 (SEQ ID NO:14) The asterisk indicates the site of the single amino acid substitution (Ala-to-Thr) found in the herbicide-resistant sunflower AHASL1 protein. This site of the substitution corresponds to amino acid position 7 in the partial-length AHASL1 amino acid sequence set forth in SBQ ID NO: 2. The equivalent position of this substitution in the full-length sunflower AHASL1 amino acid sequence set forth in SEQ ID NO: 12 is 107.

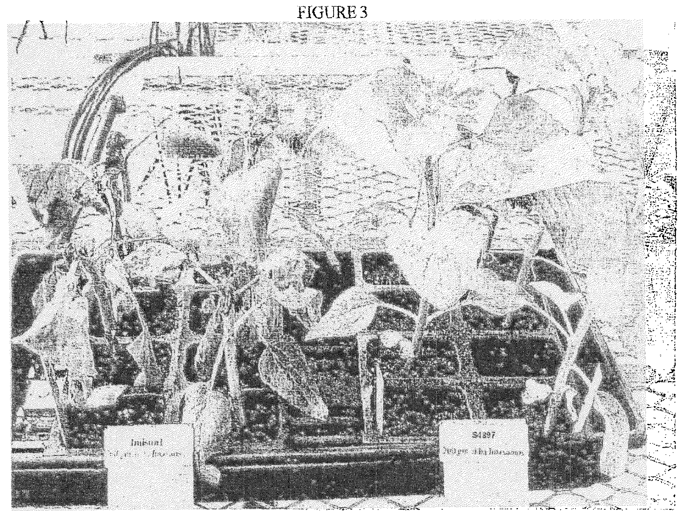

Figure 3 is a photographic illustration demonstrating the increased herbicide tolerance of S4897 sunflower plants (right side) plants as compared to the herbicide-tolerant IMISUN-1 sunflower plants (Al-Khatib and Miller (2000) Crop Sci. 40:869-870) in a greenhouse study. The S4897 and IMISUN-1 sunflower plants were spray-treated with imazamox at rate of 200 g ai/ha. Control, wild-type sunflower plants did not survive following the spray treatment with imazamox, at a rate of either 100 or 200 g ai/ha (not shown). The photograph was taken a few days after the plants were spray treated.

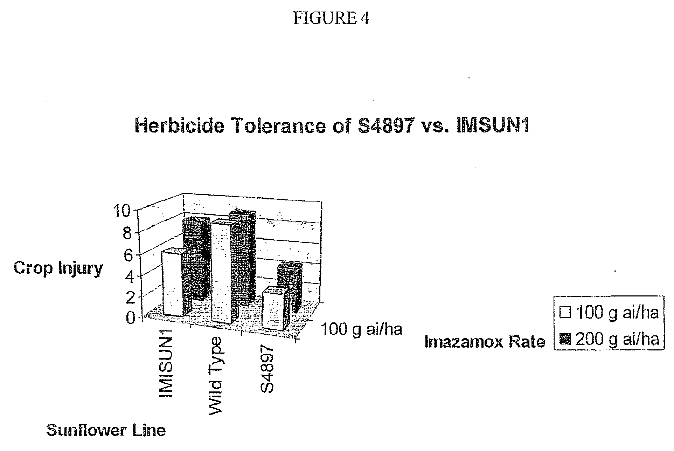

Figure 4 is graphical illustration of herbicide injury in a greenhouse test following the spray treatment of IMISUN-1, wild-type, and S4897 sunflower plants with imazamox, at either 100 (light columns) or 200 g ai/ha (dark columns). This figure demonstrates that the S4897 herbicide-resistance Sunflower plants have a significantly greater resistance or tolerance to two rates of imazamox, when compared either the herbicide-resistant IMISUN-1 plants and wild-type plants. Herbicide injury was evaluated 17 days after the imazamox application. The IMISUN-1 plants are known to possess an alanine-to-valine substitution at amino acid 190 (Kolkman et al. (2004) Theor. Appl. Genet. 109: 1147-1159).
Figure 5 is a photographic illustration demonstrating the increased herbicide tolerance of S4897 sunflower plants (right side) as compared to the herbicide-tolerant IMISUN-1 sunflower plants in the greenhouse trial described in Example 4.
Figure 6 is a photographic illustration demonstrating the increased herbicide tolerance of S4897 sunflower plants as compared to Clearfield® Variety A sunflower plants in the greenhouse trial described in Example 5.
Figure 7 is graphical illustration of a comparison of the inhibition of AHAS by Raptor for a non-Clearfield variety, a Clearfield variety and S4897 sunflower plants as described in Example 5.
Figure 8 is graphical illustration of a comparison of the inhibition of AHAS by Glean for a non-Clearfield variety, a Clearfield variety and S4897 sunflower plants as described in Example 5.
Figure 9 is a graphical illustration of the effect of foliar application of imazapir on plant height 14 days after treatment for two mutants of sunflower. Mean height (% of untreated plots) are represented by squares and error bars represent the standard deviation of the means.
Figure 10 is a graphical illustration of the effect of foliar application of imazapir on Phytotoxicity Index (PI) 14 days after treatment for two mutants of sunflower. Mean PI are represented by squares and error bars represent the standard deviation of the means.
Figure 11 is a graphical illustration of the effect of foliar application of imazapir on biomass accumulation 14 days after treatment for two mutants of sunflower. Mean dry biomass (% of untreated plots) are represented by squares and error bars represent the standard deviation of the means.
Figure 12 is a graphical illustration of the effect of foliar application of imazapir on root biomass 14 days after treatment for two mutants of sunflower. Mean root dry mass (% of untreated plots) are represented by squares and error bars represent the standard deviation of the means.

### SEQUENCE LISTING

The nucleotide and amino acid sequences listed in the accompanying sequence listing are shown using standard letter abbreviations for nucleotide bases, and three-letter code for amino acids. The nucleotide sequences follow the standard convention of beginning at the 5' end of the sequence and proceeding forward (*i*.*e*., from left to right in each line) to the 3' end. Only one strand of each nucleic acid sequence is shown, but the complementary strand is understood to be included by any reference to the displayed strand. The amino acid sequences follow the standard convention of beginning at the amino terminus of the sequence and proceeding forward (*i*.*e*., from left to right in each line) to the carboxy terminus.
SEQ ID NO: 1 sets forth the partial-length nucleotide sequence encoding a herbicide-resistant AHASL1 protein from the sunflower line S4897.
SEQ ID NO: 2 sets forth the partial-length amino acid sequence of the herbicide-resistant AHASL1 protein encoded by the nucleotide sequence set forth in SEQ ID NO: 1.
SEQ ID NO: 3 sets forth the partial-length nucleotide sequence encoding the wild-type AHASL1 protein from sunflower line BTK47.
SEQ ID NO: 4 sets forth the partial-length amino acid sequence of the wild-type AHASL1 protein encoded by the nucleotide sequence set forth SEQ ID NO: 3.
SEQ ID NO: 5 is the nucleotide sequence of GenBank Accession No. U16280.
SEQ ID NO: 6 is the amino acid sequence encoded by the nucleotide sequence of GenBank Accession No. U16280.
SEQ ID NO: 7 sets forth the nucleotide sequence of the HA1U409 primer that is described in Example 2.
SEQ ID NO: 8 sets forth the nucleotide sequence of the HAIL 1379 primer that is described in Example 2.
SEQ ID NO: 9 sets forth the nucleotide sequence of the HA1U1313 primer that is described in Example 2.
SEQ ID NO: 10 sets forth the nucleotide sequence of the HA1L2131 primer that is described in Example 2.
SEQ ID NO: 11 is the nucleotide sequence of GenBank Accession No. AY541451.
SEQ ID NO: 12 is the amino acid sequence encoded by the nucleotide sequence of GenBank Accession No. AY541451.
SEQ ID NO: 13 is the nucleotide sequence of GenBank Accession No. AY124092.
SEQ ID NO: 14 is the amino acid sequence encoded by the nucleotide sequence of GenBank Accession No. AY124092.

### DETAILED DESCRIPTION

The present disclosure relates to sunflower plants having increased resistance to herbicides when compared to a wild-type sunflower plant. Herbicide-resistant sunflower plants were produced as described hereinbelow by exposing wild-type (with respect to herbicide resistance) sunflower plants to a mutagen, allowing the plants to mature and reproduce, and selecting progeny plants that displayed enhanced resistance to an imidazolinone herbicide, relative to the resistance of a wild-type sunflower plant The disclosure describes the herbicide-resistant sunflower lines that are referred to herein as S4897, GM40, and GM1606.

From the S4897 herbicide-resistant sunflower plants and BTK47 wild-type sunflower plants, the coding region of an acetohydroxyacid synthase large subunit gene (designated as AHASL1) was isolated by polymerase chain reaction (PCR) amplification and sequenced. By comparing the polynucleotide sequences of the herbicide-resistant and wild-type sunflower plants, it was discovered that the coding region of the AHASL1 polynucleotide sequence from the herbicide-resistant sunflower plant differed from the AHASL1 polynucleotide sequence of the wild type plant by a single nucleotide, a G-to-A transition at nucleotide 21 (SEQ ID NO: 1). This G-to-A transition in the AHASL1 polynucleotide sequence results in an alanine-to-threonine substitution at amino acid 7 (SEQ ID NO: 2) in a conserved region of the predicted amino acid sequence of the herbicide-resistant sunflower AHASL1 protein (SEQ ID NO: 2), relative to the equivalent amino acid position of the wild-type AHASL1 protein from sunflower line BTK47 (*i*.*e*., amino acid 7 of SEQ ID NO: 4).

Because the nucleotide sequence set forth in SEQ ID NO: 1 does not correspond to a full-length coding region of an AHASL protein, the amino acid sequence encoded thereby that is set forth in SEQ ID NO: 2 is also less than full-length. To facilitate comparison with other sunflower AHASL amino acid sequences, the amino acid positions of sunflower AHASL proteins referred to herein, unless otherwise indicated or apparent for the context in which such positions appear, correspond to the amino acid positions of the full-length amino acid sequence of the sunflower AHASL1 protein encoded by the nucleotide sequence having GenBank Accession No. AY541451 (SEQ ID NO: 12). Accordingly, the alanine-to-threonine substitution at amino acid position 7 of SEQ ID NO: 2 corresponds to amino acid position 107 in the amino acid sequence of SEQ ID NO: 12.

Thus, the present disclosure includes an amino acid substitution that can be used to produce herbicide-resistant sunflower AHASL proteins, the polynucleotides encoding such proteins, and herbicide-resistant plants, plant tissues, plant cells, and seeds. Because the alanine that is found at amino acid position 107 in wild-type, full-length sunflower AHASL 1 proteins or equivalent position is within a region of amino acids that is conserved across plant species, it is expected that the substitution of threonine, for this same conserved alanine in other AHASL proteins from sunflower (e.g., AHASL2 and AHASL3) will also confer herbicide tolerance. Thus, a polynucleotide encoding a sunflower AHASL protein can be mutated by any method known in the art such as, for example, site-directed mutagenesis to produce a sunflower polynucleotide that encodes an AHASL protein with a threonine at position 107 or equivalent position. Polynucleotide sequences and the amino acid sequences encoded thereby corresponding to the sunflower *AHASL1*, *AHASL2,* and *AHASL3* genes are set forth in GenBank Accession Nos. AY541451 (SEQ ID NOS: 11 and 12), AY541452, AY541453, AY541454, AY541455, AY541456, AY541457, and AY541458.

Accordingly, such polynucleotides and the herbicide-resistant AHASL proteins encoded thereby find use in the production of herbicide-resistant plants, plant cells, plant tissues, and seeds by the methods disclosed herein.

The present disclosure additionally encompasses isolated sunflower *AHASL2* and *AHASL3* polynucleotides that encode herbicide-resistant AHASL2 and AHASL3 proteins, respectively.

In such herbicide-resistant AHASL2 and AHASL3 proteins, the amino acid at position 107 or equivalent position is threonine.

The disclosure further relates to isolated polynucleotide molecules comprising Nucleotide sequences that encode acetohydraxyacid synthase large subunit (AHASL) proteins and to such AHASL proteins. The disclosure discloses the isolation and nucleotide sequence of a polynucleotide encoding a herbicide-resistant sunflower AHASL1 protein from an herbicide-resistant sunflower plant that was produced by chemical mutagenesis of wild-type sunflower plants. The herbicide-resistant sunflower AHASL1 proteins of the disclosure possess a alanine-to-threonine substitution at position 107 or equivalent position in their respective amino acid sequences, when compared to the corresponding wild-type amino acid sequence. The disclosure further discloses the isolation and nucleotide sequence of a polynucleotide molecule encoding a wild-type sunflower AHASL1 protein.

The present disclosure describes isolated polynucleotide molecules that encode AHASL proteins from sunflower (*Helianthus annuus* L.). Specifically, the disclosure includes isolated polynucleotide molecules comprising: the nucleotide sequences set forth in SEQ ID NOS: 1 and 3, nucleotide sequences encoding AHASL proteins comprising the amino acid sequences set forth in SEQ ID NOS: 2 and 4, and fragments and variants of such nucleotide sequences that encode functional AHASL proteins.

The isolated herbicide-resistant AHASL polynucleotide molecules of the disclosure comprise nucleotide sequences that encode herbicide-resistant AHASL proteins. Such polynucleotide molecules can be used in polynucleotide constructs for the transformation of plants, particularly crop plants, to enhance the resistance of the plants to herbicides, particularly herbicides that are known to inhibit AHAS activity, more particularly imidazolinone herbicides. Such polynucleotide constructs can be used in expression cassettes, expression vectors, transformation vectors, plasmids and the like. The transgenic plants obtained following transformation with such polynucleotide constructs show increased resistance to AHAS-inhibiting herbicides such as, for example, imidazolinone and sulfonylurea herbicides.

Compositions of the disclosure include nucleotide sequences that encode AHASL proteins. In particular, the present disclosure discloses for isolated polynucleotide molecules comprising nucleotide sequences encoding the amino acid sequences shown in SEQ ID NOS: 2 and 4, and fragments and variants thereof that encode polypeptides comprising AHAS activity. Further provided are polypeptides having an amino acid sequence encoded by a polynucleotide molecule described herein, for example those set forth in SEQ ID NOS: 1 and 3, and fragments and variants thereof that encode polypeptides comprising AHAS activity.

The disclosure encompasses isolated or substantially purified nucleic acid or protein compositions. An "isolated" or "purified" polynucleotide molecule or protein, or biologically active portion thereof, is substantially or essentially free from components that normally accompany or interact with the polynucleotide molecule or protein as found in its naturally occurring environment Thus, an isolated or purified polynucleotide molecule or protein is substantially free of other cellular material, or culture medium when produced by recombinant techniques, or substantially free of chemical precursors or other chemicals when chemically synthesized. An "isolated" nucleic acid may be free of sequences (preferably protein encoding sequences) that naturally flank the nucleic acid (i.e., sequences located at the 5' and 3' ends of the nucleic acid) in the genomic DNA of the organism from which the nucleic acid is derived. For example,
the isolated polynucleotide molecule can contain less than about 5 kb, 4 kb, 3 kb, 2 kb, 1 kb, 0.5 kb, or 0.1 kb of nucleotide sequences that naturally flank the polynucleotide molecule in genomic DNA of the cell from which the nucleic acid is derived. A protein that is substantially free of cellular material includes preparations of protein having less than about 30%, 20%, 10%, 5%, or 1% (by dry weight) of contaminating protein. When the protein of the disclosure or biologically active portion thereof is recombinantly produced, preferably culture medium represents less than about 30%, 20%, 10%, 5%, or 1% (by dry weight) or chemical precursors or non-protein-of-interest chemicals.

The present: disclosure describes isolated polypeptides comprising AHASL proteins. The isolated polypeptides comprise an amino acid sequence selected from the group consisting of the amino acid sequences set forth in SEQ ID NOS: 2 and 4, the amino acid sequences encoded by nucleotide sequences set forth in SEQ ID NOS: 1 and 3, and functional fragments and variants of said amino acid sequences that encode an AHASL polypeptide comprising AHAS activity. By "functional fragments and variants" is intended fragments and variants of the exemplified polypeptides that comprise AHAS activity.

The methods disclosed herein may involve the use of herbicide-tolerant or herbicide-resistant plants. By an "herbicide-tolerant" or "herbicide-resistant" plant, it is intended that a plant that is tolerant or resistant to at least one herbicide at a level that would normally kill, or inhibit the growth of, a normal or wild-type plant. In

The herbicide-tolerant plants of the disclosure may comprise a herbicide-tolerant or herbicide-resistant AHASL protein. By "herbicide-tolerant AHASL protein" or "herbicide-resistant AHASL protein", it is intended that such an AHASL protein displays higher AHAS activity, relative to the AHAS activity of a wild-type AHASL protein, when in the presence of at least one herbicide that is known to interfere with AHAS activity and at a concentration or level of the herbicide that is to known to inhibit the AHAS activity of the wild-type AHASL protein. Furthermore, the AHAS activity of such a herbicide-tolerant or herbicide-resistant AHASL protein may be referred to herein as "herbicide-tolerant" or "herbicide-resistant" AHAS activity.

Herein, the terms "herbicide-tolerant" and "herbicide-resistant" are used interchangeable and are intended to have an equivalent meaning and an equivalent scope. Similarly, the terms "herbicide-tolerance" and "herbicide-resistance" are used interchangeable and are intended to have an equivalent meaning and an equivalent scope. Likewise, the terms "imidazolinone-resistant" and "imidazolinone-resistance" are used interchangeable and are intended to be of an equivalent meaning and an equivalent scope as the terms "imidazolinone-tolerant" and "imidazolinone-tolerance", respectively.

The disclosure encompasses herbicide-resistant AHASL polynucleotides and herbicide-resistant AHASL proteins. By "herbicide-resistant AHASL polynucleotide" is intended polynucleotide that encodes a protein comprising herbicide-resistant AHAS activity. By "herbicide-resistant AHASL protein" is intended a protein or polypeptide that comprises herbicide-resistant AHAS activity.

Further, it is recognized that a herbicide-tolerant or herbicide-resistant AHASL protein can be introduced into a plant by transforming a plant or ancestor thereof with a nucleotide sequence encoding a herbicide-tolerant or herbicide-resistant AHASL protein. Such herbicide-tolerant or herbicide-resistant AHASL proteins are encoded by the herbicide-tolerant or herbicide-resistant *AHASL* polynucleotides. Alternatively, a herbicide-tolerant or herbicide-resistant AHASL protein may occur in a plant as a result of a naturally occurring or induced mutation in an endogenous *AHASL* gene in the genome of a plant or progenitor thereof.

The present disclosure describes plants, plant tissues, plant cells, and host cells with increased resistance or tolerance to at least one herbicide, particularly an imidazolinone or sulfonylurea herbicide. The amount or concentration of the herbicide may be an "effective amount" or "effective concentration." By "effective amount" and "effective concentration" is intended an amount and concentration, respectively, that is sufficient to kill or inhibit the growth of a similar, wild-type, plant, plant tissue, plant cell, or host cell, but that said amount does not kill or inhibit as severely the growth of the herbicide-resistant plants, plant tissues, plant cells, and host cells of the present disclosure. Typically, the effective amount of a herbicide is an amount that is routinely used in agricultural production systems to kill weeds of interest. Such an amount is known to those of ordinary skill in the art.

By "similar, wild-type, plant, plant tissue, plant cell or host cell" is intended a plant, plant tissue, plant cell, or host cell, respectively, that lacks the herbicide-resistance characteristics and/or particular polynucleotide of the disclosure that are disclosed herein. The use of the term "wild-type" is not, therefore, intended to imply that a plant, plant tissue, plant cell, or other host cell lacks recombinant DNA in its genome, and/or does not possess herbicide-resistant characteristics that are different from those disclosed herein.

As used herein unless clearly indicated otherwise, the term "plant" intended to mean a plant at any developmental stage, as well as any part or parts of a plant that may be attached to or separate from a whole intact plant Such parts of a plant include, but are not limited to, organs, tissues, and cells of a plant. Examples of particular plant parts include a stem, a leaf, a root, an inflorescence, a flower, a floret, a fruit, a pedicle, a peduncle, a stamen, an anther, a stigma, a style, an ovary, a petal, a sepal, a carpel, a root tip, a root cap, a root hair, a leaf hair, a seed hair, a pollen grain, a microspore, a cotyledon, a hypocotyl, an epicotyl, xylem, phloem, parenchyma, endosperm, a companion cell, a guard cell, and any other known organs, tissues, and cells of a plant. Furthermore, it is recognized that a seed is a plant.

The plants of the present disclosure include both non-transgenic plants and transgenic plants. By "non-transgenic plant" is intended to mean a plant lacking recombinant DNA in its genome. By "transgenic plant" is intended to mean a plant comprising recombinant DNA in its genome. Such a transgenic plant can be produced by introducing recombinant DNA into the genome of the plant. When such recombinant DNA is incorporated into the genome of the transgenic plant, progeny of the plant can also comprise the recombinant DNA. A progeny plant that comprises at least a portion of the recombinant DNA of at least one progenitor transgenic plant is also a transgenic plant.

The present disclosure describes the herbicide-resistant sunflower line that is referred to herein as S4897 and progeny and derivatives thereof that comprise the herbicide-resistance characteristics of S4897. A Deposit of seeds of the GM40 sunflower which is derived from sunflower line S4897 and comprises the herbicide-resistance characteristics of S4897 was made with the Patent Depository of the American Type Culture Collection (ATCC), Mansassas, VA 20110 USA on May 17, 2005 and assigned. ATCC Patent Deposit Number PTA-6716. The deposit of sunflower line GM40 was made for a term of at least 30 years and at least 5 years after the most recent request for the furnishing of a sample of the deposit is received by the ATCC. Additionally, Applicants have satisfied all the requirements of 37 C.F.R. §§ 1.801-1.809, including providing an indication of the viability of the sample.

The present disclosure further describes the herbicide-resistant sunflower line that is referred to herein as GM1606. A deposit of seeds of the sunflower M1606 was made with the Patent Depository of the American Type Culture Collection (ATCC), Mansassas, VA 20110 USA on May 19, 2006 and assigned ATCC Patent Deposit Number PTA-7606. The deposit of sunflower GM1606 was made for a term of at least 30 years and at least 5 years after the most recent request for the furnishing of a sample of the deposit is received by the ATCC. Additionally, Applicants have satisfied all the requirements of 37 C.F.R. §§ 1.801-1.809, including providing an indication of the viability of the sample.

The present disclosure describes herbicide-resistant sunflower plants that were produced by mutation breeding. Wild-type sunflower plants were mutagenized by exposing the plants to a mutagen, particularly a chemical mutagen, more particularly ethyl methanesulfonate (EMS). However, the present disclosure is not limited to herbicide-resistant sunflower plants that are produced by a mutagenesis method involving the chemical mutagen EMS. Any mutagenesis, method known in the art may be used to produce herbicide-resistant sunflower plants disclosed herein. Such mutagenesis, methods can involve, for example, the use of any one or more of the following mutagens: radiation, such as X-rays, Gamma rays (e.g., cobalt 60 or cesium 137), neutrons, (e.g., product of nuclear fission by uranium 235 in an atomic reactor), Beta radiation (e.g., emitted from radioisotopes such as phosphorus 32 or carbon 14), and ultraviolet radiation (preferably from 2500 to 2900 nm), and chemical mutagens such as base analogues (e.g., 5-bromo-uracil), related compounds (e.g., 8-ethoxy caffeine), antibiotics (e.g., streptonigrin), alkylating agents (e.g., sulfur mustards, nitrogen mustards, epoxides, ethylenamines, sulfates, sulfonates, sulfones, lactones), azide, hydroxylamine, nitrous acid, or acridines. Herbicide-resistant plants can also be produced by using tissue culture methods to select for plant cells comprising herbicide-resistance mutations and then regenerating herbicide-resistant plants therefrom. See, for example, U.S. Patent Nos. 5,773,702 and 5,859,348.

Further details of mutation breeding can be found in "Principals of Cultivar Development" Fehr, 1993 Macmillan Publishing Company .

Analysis of the *AHASL1* gene of the sunflower plant of the S4897 and GM1606 lines revealed that the mutation that results in the substitution of a threonine for the alanine that is found at amino acid position 7 in the wild-type AHASL1 amino acid sequence for SEQ ID NO: 4. Amino acid position 7 in SEQ ID NOS: 2 and 4 corresponds to amino acid position 107 in the full-length amino acid sequence of a sunflower AHASL1 protein set forth in SEQ ID NO: 12. Thus,
substituting threonine for the alanine at amino acid position 107, or equivalent position, in an AHASL protein can cause a sunflower plant to have enhanced resistance to a herbicide, particularly an imidazolinone and/or sulfonylurea herbicide. As disclosed in Example 6 below, the alanine at amino acid position 107 occurs within a conserved region of AHASL proteins. Similarly, amino acid substitutions in other conserved regions of AHASL proteins have been disclosed that are known to confer herbicide resistance on a plant that comprises such an AHASL protein. Accordingly, the herbicide-resistant sunflower plants of the disclosure include,
those sunflower plants which comprise in their genomes at least one copy of an AHASL polynucleotide that encodes a herbicide-resistant AHASL protein that comprises a threonine at amino acid position 107 or equivalent position.

The sunflower plants of the disclosure further include plants that comprise, relative to the wild-type AHASL protein, a threonine at amino acid position 107 or equivalent position, and one or more additional amino acid substitutions in the AHASL protein relative to the wild-type AHASL protein, wherein such a sunflower plant has increased resistance to at least one herbicide when compared to a wild-type sunflower plant. Such sunflower plants comprise AHASL proteins that comprise a threonine at amino acid position 107 or equivalent position and at least one member selected from the group consisting of:
glutamine, or serine at amino acid position 182 or equivalent position; an isoleucine or an amino acid other than threonine at amino acid position 188 or equivalent position; an aspartate or valine at amino acid position 190 or equivalent position; a leucine at amino acid position 559 or equivalent position; and an asparagine, threonine, phenylalanine, or valine at amino acid position 638 or equivalent position.

The present disclosure describes AHASL proteins with amino acid substitutions at particular amino acid positions within conserved regions of the sunflower AHASL proteins disclosed herein. Furthermore, those of ordinary skill will recognize that such amino acid positions can vary depending on whether amino acids are added or removed from, for example, the N-terminal end of an amino acid sequence. Thus, the disclosure encompasses the amino acid substitutions at the recited position or equivalent position (e.g., "amino acid position 107 or equivalent position"). By "equivalent position" is intended to mean a position that is within the same conserved region as the exemplified amino acid position. Such conserved regions are know in the art (see Table 4 below) or can be determined by multiple sequence alignments as disclosed herein or by methods known in the art.

In addition, the present disclosure describes AHASL polypeptides comprising amino acid substitutions that are known to confer resistance on a plant to at least one herbicide, particularly an imidazolinone, herbicide and/or a sulfonylurea herbicide. Such AHASL polypeptides include, for example, those that comprise a threonine at amino acid position 107 and at least one member selected from the group consisting of:
glutamine, or serine at amino acid position 182 or equivalent position; an isoleucine or another amino acid other than threonine at amino acid position 188 or equivalent position; an aspartate or valine at amino acid position 190 or equivalent position; an aspartate or valine at amino acid position 190 or equivalent position; a leucine at amino acid position 559 or equivalent position; and an asparagine, threonine, phenylalanine, or valine at amino acid position 638 or equivalent position. The disclosure further describes isolated polynucleotides encoding such AHASL polypeptides, as well as expression cassettes, transformation vectors, transformed host cells, transformed plants, and methods comprising such polynucleotides.

The present disclosure describes methods for enhancing the tolerance or resistance of a plant, plant tissue, plant cell, or other host cell to at least one herbicide that interferes with the activity of the AHAS enzyme. Such a herbicide may be an imidazolinone herbicide, a sulfonylurea herbicide, a triazolopyrimidine herbicide, a pyrimidinyloxybenzoate herbicide, a sulfonylamino-carbonyltriazolinone herbicide, or mixture thereof. Such a herbicide may be an imidazolinone herbicide, a sulfonylurea herbicide, or mixture thereof. The imidazolinone herbicides include, but are not limited to, PURSUIT® (imazethapyr), CADRE® (imazapic), RAPTOR® (imazamox), SCEPTER® (imazaquin), ASSERT® (imazethabenz), ARSENAL® (imazapyr), a derivative of any of the aforementioned herbicides, and a mixture of two or more of the aforementioned herbicides, for examples, imazapyr/imazamox (ODYSSEY®).

More specifically, the imidazolinone herbicide can be selected from, but is not limited to, 2-(4-isopropyl-4-methyl-5-oxo-2-imidazolin-2-yl)-nicotinic acid, 2-(4-isopropyl-4-methyl-5-oxo-2-imidazolin-2-yl)quinoline-3-carboxylic acid, 5-ethyl-2-(4-isopropyl-4-methyl-5-oxo-2-imidazolin-2-yl)-nicotinic acid, 2-(4-isopropyl-4-methyl-5-oxo-2-imidazolin-2-yl)-5-(methoxymethyl)-nicotinic acid, 2-(4-isopropyl-4-methyl-5-oxo-2-imidazolin-2-yl)-5-methylnicotinic acid, and a mixture of methyl 6-(4-isopropyl-4-methyl-5-oxo-2-imidazonlin-2-yl)-m-toluate and methyl 2-(4-isopropyl-4-methyl-5-oxo-2-imidazolin-2-yl)-p-toluate. The use of 5-ethyl-2-(4-isopropyl-4-methyl-5-oxo-2-imidazolin-2-yl)-nicotinic acid and 2-(4-isopropyl-4-methyl-5-oxo-2-imidazolin-2-yl)-5-methoxymethylnicotinic acid is preferred. The use of 2-(4-isopropyl-4-methyl-5-oxo-2-imidazolin-2-yl)-5-methoxymethylnicotinic acid is particularly preferred.

For the present disclosure, the sulfonylurea herbicides include, but are not limited to, chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfuron, flazasulfuron, imazosulfuron, pyrazosulfuron ethyl, halosulfuron, azimsulfuron. cyclosulfuron, ethoxysulfuron, flazasulfuron, flupyrsulfuron methyl, foramsulfuron, iodosulfuron, oxasulfuron, mesosulfuron, prosulfuron, sulfosulfuron, trifloxysulfuron, tritosulfluron, a derivative of any of the aforementioned herbicides, and a mixture of two or more of the aforementioned herbicides. The triazolopyrimidine herbicides include, but are not limited to, cloransulam, diclosulam, florasulam, flumetsulam, metosulam, and penoxsulam. The pyrimidinyloxybenzoate herbicides include, but are not limited to, bispyribac, pyrithiobac, pyriminobac, pyribenzoxim and pyriftalid. The sulfonylaminocarbonyltriazolinone herbicides include, but are not limited to, flucarbazone and propoxycarbazone.

It is recognized that pyrimidinyloxybenzoate herbicides are closely related to the pyrimidinylthiobenzoate herbicides and are generalized under the heading of the latter name by the Weed Science Society of America. Accordingly, the herbicides of the present disclosure further include pyrimidinylthiobenzoate herbicides, including, but not limited to, the pyrimidinyloxybenzoate herbicides described above.

The present disclosure describes methods for enhancing AHAS activity in a plant comprising transforming a plant with a polynucleotide construct comprising a promoter operably linked to an AHASL1 nucleotide sequence of the disclosure. The methods involve introducing a polynucleotide construct of the disclosure into at least one plant cell and regenerating a transformed plant therefrom. The methods involve the use of a promoter that is capable of driving gene expression in a plant cell. Such a promoter may be a constitutive promoter or a tissue-preferred promoter. The methods find use in enhancing or increasing the resistance of a plant to at least one herbicide that interferes with the catalytic activity of the AHAS enzyme, particularly an imidazolinone herbicide.

The present disclosure describes expression cassettes for expressing the polynucleotides of the disclosure in plants, plant tissues, plant cells, and other host cells. The expression cassettes comprise a promoter expressible in the plant, plant tissue, plant cell, or other host cells of interest operably linked to a polynucleotide of the disclosure that comprises a nucleotide sequence encoding either a full-length (*i.e.* including the chloroplast transit peptide) or mature AHASL1 protein (*i.e.* without the chloroplast transit peptide). If expression is desired in the plastids or chloroplasts of plants or plant cells, the expression cassette may also comprise an operably linked chloroplast-targeting sequence that encodes a chloroplast transit peptide.

The expression cassettes of the disclosure find use in a method for enhancing the herbicide tolerance of a plant or a host cell. The method involves transforming the plant or host cell with an expression cassette of the disclosure, wherein the expression cassette comprises a promoter that is expressible in the plant or host cell of interest and the promoter is operably linked to a polynucleotide of the disclosure that comprises a nucleotide sequence encoding an imidazolinone-resistant AHASL protein of the disclosure.

The use of the term "polynucleotide constructs" herein is not intended to limit the present invention to polynucleotide constructs comprising DNA. Those of ordinary skill in the art will recognize that polynucleotide constructs, particularly polynucleotides and oligonucleotides, comprised of ribonucleotides and combinations of ribonucleotides and deoxyribonucleotides may also be employed in the methods disclosed herein. Thus, the polynucleotide constructs of the present disclosure encompass all polynucleotide constructs that can be employed in the methods of the present disclosure for transforming plants including but not limited to, those comprised of deoxyribonucleotides, ribonucleotides, and combinations thereof. Such deoxyribonucleotides and ribonucleotides include both naturally occurring molecules and synthetic analogues. The polynucleotide constructs of the disclosure also encompass all forms of polynucleotide constructs including, but not limited to, single-stranded forms, double-stranded forms, hairpins, stem-and-loop structures, and the like. Furthermore, it is understood by those of ordinary skill in the art that each nucleotide sequences disclosed herein also encompasses the complement of that exemplified nucleotide sequence.

Furthermore, it is recognized that the methods of the disclosure may employ a polynucleotide construct that is capable of directing, in a transformed plant, the expression of at least one protein, or at least one RNA, such as, for example, an antisense RNA that is complementary to at least a portion of an mRNA. Typically such a polynucleotide construct is comprised of a coding sequence for a protein or an RNA operably linked to 5' and 3' transcriptional regulatory regions. Alternatively, it is also recognized that the methods of the disclosure may employ a polynucleotide construct that is not capable of directing, in a transformed plant, the expression of a protein or an RNA.

Further, it is recognized that, for expression of a polynucleotide of the disclosure in a host cell of interest, the polynucleotide is typically operably linked to a promoter that is capable of driving gene expression in the host cell of interest. The methods of the disclosure for expressing the polynucleotides in host cells do not depend on particular promoter. The methods encompass the use of any promoter that is known in the art and that is capable of driving gene expression in the host cell of interest.

The present disclosure encompasses AHASL polynucleotide molecules and fragments and variants thereof. Polynucleotide molecules that are fragments of these nucleotide sequences are also encompassed by the present disclosure. By "fragments" is intended a portion of the nucleotide sequence encoding an AHASL protein of the disclosure. A fragment of an AHASL nucleotide sequence of the disclosure may encode a biologically active portion of an AHASL protein, or it may be a fragment that can be used as a hybridization probe or PCR primer using methods disclosed below. A biologically active portion of an AHASL protein can be prepared by isolating a portion of one of the AHASL nucleotide sequences of the disclosure, expressing the encoded portion of the AHASL protein (e.g., by recombinant expression *in vitro*), and assessing the activity of the encoded portion of the AHASL1 protein. Polynucleotide molecules that are fragments of an AHASL nucleotide sequence comprise at least about 15, 20, 50, 75, 100, 200, 300, 350, 400, 450, 500, 550, 600, 650, 700, 750, 800, 850, 900, 950, 1000, 1050, or 1100 nucleotides, or up to the number of nucleotides present in a full-length nucleotide sequence disclosed herein (for example, 1178 nucleotides for both SEQ ID NOS: 1 and 3) depending upon the intended use.

A fragment of an AHASL nucleotide sequence that encodes a biologically active portion of an AHASL protein of the disclosure will encode at least about 15, 25, 30, 50, 75, 100, 125, 150, 175, 200, 250, 300, or 350 contiguous amino acids, or up to the total number of amino acids present in a full-lengtb AHASL1 protein of the disclosure (for example, 392 amino acids for both SEQ ID NOS: 2 and 4). Fragments of an AHASL1 nucleotide sequence that are useful as hybridization, probes for PCR primers generally need not encode a biologically active portion of an AHASL1 protein.

Polynucleotide molecules that are variants of the nucleotide sequences disclosed herein are also encompassed by the present disclosure. "variants" of the AHASL nucleotide sequences of the disclosure include those sequences that encode the AHASL proteins disclosed herein but that differ conservatively because of the degeneracy of the genetic code. These naturally occurring allelic variants can be identified with the use of well-known molecular biology techniques, such as polymerase chain reaction (PCR) and hybridization techniques as outlined below. Variant nucleotide sequences also include synthetically derived nucleotide sequences that have been generated, for example, by using site-directed mutagenesis but which still encode the AHASL1 protein disclosed in the present disclosure as discussed below. Generally, nucleotide sequence variants of the disclosure will have at least about 70%, 75%, 80%, 85% 90%, 91%. 92%, 93%, 94%, 95%, 96%, 97%, 98%, or 99% identity to a particular nucleotide sequence disclosed herein. A variant AHASL nucleotide sequence will encode an AHASL protein, respectively, that has an amino acid sequence having at least 95%, identity to the amino acid sequence of an AHASL protein disclosed herein.

In addition, the skilled artisan will further appreciate that changes can be introduced by mutation into the nucleotide sequences of the disclosure thereby leading to changes in the amino acid sequence of the encoded AHASL proteins without altering the biological activity of the AHASL proteins. Thus, an isolated polynucleotide molecule encoding an AHASL protein having a sequence that differs from that of SEQ ID NOS: 1 or 3, respectively, can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis, and PCR-mediated mutagenesis. Such variant nucleotide sequences are also encompassed by the present disclosure.

For example, conservative amino acid substitutions may be made at one or more predicted, optionally nonessential amino acid residues. A "nonessential" amino acid residue is a residue that can be altered from the wild-type sequence of an AHASL protein (*e.g*., the sequence of (SEQ ID NOS: 2 and 4, respectively) without altering the biological activity, whereas an "essential" amino acid residue is required for biological activity. A conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (*e.g.,* aspartic acid, glutamic acid), uncharged polar side chains (*e.g*., glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.,* threonine, valine, isoleucine) and aromatic side chains (*e.g*., tyrosine, phenylalanine, tryptophan, histidine). Such substitutions would not be made for conserved amino acid residues, or for amino acid residues residing within a conserved motif.

The proteins of the disclosure may be altered in various ways including amino acid substitutions, deletions, truncations, and insertions. Methods for such manipulations are generally known in the art. For example, amino acid sequence variants of the AHASL proteins can be prepared by mutations in the DNA. Methods for mutagenesis and nucleotide sequence alterations are well known in the art. See, for example, Kunkel (1985) Proc. Natl. Acad Sci. USA. 82:488-492; Kunkel at al. (1987) Methods in Ezymol. 154:367-382; U.S. Patent No. 4,873,192; Walker and Gaastra, ads. (1983) Techniques in Molecular Biology (MacMillan. Publishing Company, New York) and the references cited therein. Guidance as to appropriate amino acid substitutions that do not affect biological activity of the protein of interest may be found in the model of Dayhoff et al. (1978) Atlas of Protein Sequence and Structure (Natl. Biomed. Res. Found., Washington, D.C.).

Conservative substitutions, such as exchanging one amino acid with another having similar properties, may be desirable.

Alternatively, variant AHASL nucleotide sequences can be made by introducing mutations randomly along all or part of an AHASL coding sequence, such as by saturation mutagenesis, and the resultant mutants can be screened for AHAS activity to identify mutants that retain AHAS activity, including herbicide-resistant AHAS activity. Following mutagenesis, the encoded protein can be expressed recombinantly, and the activity of the protein can be determined using standard assay techniques.

Thus, the nucleotide sequences of the disclosure include the sequences disclosed herein as well as fragments and variants thereof The *AHASL* nucleotide sequences of the disclosure, and fragments and variants thereof, can be used as probes and/or primers to identify and/or clone *AHASL* homologues in other plants. Such probes can be used to detect transcripts or genomic sequences encoding the same or identical proteins.

In this manner, methods such as PCR, hybridization, and the like can be used to identify such sequences having substantial identity to the sequences of the disclosure. *See,* for example, Sambrook et al. (1989) Molecular Cloning: Laboratory Manual (2d ed., Cold Spring Harbor Laboratory Press, Plainview, NY) and Innis, el al. (1990) PCR Protocols: A Guide to Methods and Applications (Academic Press, NY). *AHASL* nucleotide sequences isolated based on their sequence identity to the AHASL 1 nucleotide sequences set forth herein or to fragments and variants thereof are encompassed by the present disclosure.

In a hybridization method, all or part of a known *AHASL* nucleotide sequence can be used to screen cDNA or genomic libraries. Methods for construction of such cDNA and genomic libraries are generally known in the art and are disclosed in Sambrook et al. (1989) Molecular Cloning: A Laboratory Manual (2d ed., Cold Spring Harbor Laboratory Press, Plainview, NY). The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known *AHASL* nucleotide sequence disclosed herein. Degenerate primers designed on the basis of conserved nucleotides or amino acid residues in a known *AHASL* nucleotide sequence or encoded amino acid sequence can additionally be used. The probe typically comprises a region of nucleotide sequence that hybridizes under stringent conditions to at least about 12, preferably about 25, more preferably about 50, 75, 100, 125, 150, 175, 200, 250, 300, 350, 400, 500, 600, 700, 800, 900, 1000, 1100, or 1178 consecutive nucleotides of an *AHASL* Nucleotide sequence of the disclosure or a fragment or variant thereof. Preparation of probes for hybridization is generally known in the art and is disclosed in Sambrook et al. (1989) Molecular Cloning: A Laboratory Manual (2d ed., Cold Spring Harbor Laboratory Press, Plainview, New York).

For example, the entire *AHASL* sequence disclosed herein, or one or more portions thereof may be used as a probe capable of specifically hybridizing to corresponding *AHASL* sequences and messenger RNAs. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Plainview, New York).

Hybridization of such sequences may be carried out under stringent conditions. By "stringent conditions" or "Stringent hybridization conditions" is intended conditions under which a probe will hybridize to its target sequence to a detectably greater degree than to other sequences (e.g., at least 2-fold over background). Stringent conditions are sequence-dependent and will be different in different circumstances.

Typically, stringent conditions will be those in which the salt concentration is less than about 1.5 M Na ion, typically about 0.01 to 1.0 M Na. ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash is 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1% to about 1% SDS. The duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1% of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately Stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et al., eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Plainview, New York).

It is recognized that the polynucleotide molecules and proteins of the disclosure encompass polynucleotide molecules and proteins comprising a Nucleotide or an amino acid sequence that is sufficiently identical to the nucleotide sequence of SEQ ID NOS: 1 and/or 3, or to the amino acid sequence of SEQ ID NOS: 2 and/or 4. The term "sufficiently identical" is used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain and/or common functional activity. For example, amino acid or nucleotide sequences that contain a common structural domain having at least about 45%, 55%, or 65% identity, 75% identity, 85%, 95%, or 98% identity are defined herein as sufficiently identical.

To determine the percent identity of two amino acid sequences or of two nucleic acids, the sequences are aligned for optimal comparison purposes. The percent identity between the two sequences is a function of the number of identical positions shared by the sequences (*i.e.*, percent identity = number of identical positions/total number of positions (e.g., overlapping positions) x 100). The two sequences may be the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A preferred, nonlimiting example of a mathematical algorithm utilized for the comparison of two sequences is the algorithm of Karlin and Altschul (1990) Proc. Natl. Acad. Sci. USA 87:2264, modified as in Karlin and Altschul (1993) Proc. Natl. Acad. Sci. USA 90:5873-5877. Such an algorithm is incorporated into the NBLAST and XBLAST programs of Altschul et al. (1990) J. Mo/. Blol. 215:403. BLAST nucleotide searchers can. be performed with the NBLAST program, score = 100, wordlength = 12, to obtain nucleotide sequences homologous to the polynucleotide molecules of the disclosure. BLAST protein searches can be performed with the XBLAST program, score = 50, wordlength = 3, to obtain amino acid sequences homologous to protein molecules of the disclosure. To obtain gapped alignments for comparison purposes, Gapped BLAST can be utilized as described in Altschul et al. (1997) Nucleic Acids Res. 25:3389. Alternatively, PSI-Blast can be used to perform an iterated search that detects distant relationships between molecules. See Altschul *et al.* (1997) *supra.* When utilising BLAST, Gapped BLAST, and PSI-Blast programs, the default parameters of the respective programs (e.g., XBLAST and BLAST) can be used. See http://www.ncbi.nim.nih.gov. Another preferred, non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG sequence alignment software package. When utilizing the ALIGN program for comparing amino acid sequences, a PAM120 weight residue table, a gap length penalty of 12, and a gap penalty of 4 can be used. Alignment may also be performed manually by inspection.

Unless otherwise stated, sequence identity/similarly value provided herein refer to the value obtained using the full-length sequences of the disclosure and using multiple alignment by mean of the algorithm Clustal W (Nucleic Acid Research, 22(22):4673-4680, 1994) using the program AlignX included in the software package Vector NTI Suite Version 7 (InforMax, Inc., Bethesda, MD, USA) using the default parameters; or any equivalent program thereof. By "equivalent program" is intended any sequence comparison program that, for any two sequences in question, generates an alignment having identical nucleotide or amino acid residue matches and an identical percent sequence identity when compared to the corresponding alignment generated by AlignX in the software package Vector NTI Suite Version 7.

The *AHASL* nucleotide sequences of the disclosure include both the naturally occurring sequences as well as mutant forms, particularly mutant forms that encode AHASL proteins comprising herbicide-resistant AHAS activity. Likewise, the proteins of the disclosure encompass both naturally occurring proteins as well as variations and modified forms thereof. Such variants will continue to possess the desired AHAS activity. Obviously, the mutations that will be made in the DNA encoding the variant must not place the sequence out of reading frame and preferably will not create complementary regions that could produce secondary mRNA stricture. See, EP Patent Application Publication No. 75,444.

The deletions, insertions, and substitutions of the proteins sequences encompassed herein are not expected to produce radical changes in the characteristics of the protein. However, when it is difficult to predict the exact effect of the substitution, deletion, or insertion in advance of doing so, one skilled in the art will appreciate that the effect will be evaluated by routine screening assays. That is, the activity can be evaluated by AHAS activity assays. *See,* for example. Singh et al. (1988) Anal. Biochem. 171:173-179.

Variant nucleotide sequences and proteins also encompass sequences and proteins derived from a mutagenic and recombinogenic procedure such as DNA shuffling. With such a procedure, one or more different *AHASL* coding sequences can be manipulated to create a new AHAS protein possessing the desired properties. In this manner, libraries of recombinant polynucleotides are generated from a population of related sequence polynucleotides comprising sequence legions that have substantial sequence identity and can be homologously recombined *in vitro* or *in vivo.* For example, using this approach, sequence motifs encoding a domain of interest may be shuffled between the *AHASL* gene of the disclosure and other known *AHASL* genes to obtain a new gene coding for a protein with an improved property of interest, such as an increased Kₘ in the case of an enzymes. Strategies for such DNA shuffling are known in the art. See, for example, Stemmer (1994) Proc. Nalt. Acad. Sci. USA 91:10747-10751; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad Sci. USA 94:4504-4509; Crameri et al. (1998) Nature 391:288-291; and U.S. Patent Nos. 5,605,793 and 5,837,458.

The nucleotide sequences of the disclosure can be used to isolate corresponding sequence from other organism, particularly other plants, more particularly other dicots, In this manner, methods such as PCR, hybridization, and the like can be used to identity such sequences based on their sequence homology to the sequences set forth herein. Sequences isolated based on their sequence identity to the entire *AHASL* polynucleotide sequences set forth herein or to fragments thereof are encompassed by the present disclosure. Thus, isolated polynucleotide sequences that encode for an AHASL protein and which hybridize under stringent conditions to the sequence disclosed herein, or to fragments thereof are encompassed by the present disclosure.

In a PCR approach, oligonucleotide primers can be designed for use in PCR reactions to amplify corresponding DNA sequences from cDNA or genomic DNA extracted from any plant of interest. Methods for designing PCR primers and PCR cloning are generally known in the art and are disclosed in Sambrook et al. (1989) Molecular Cloning: A Laboratory Manual (2d ed., Cold Spring Harbor Laboratory Press, Plainview, New York). See also Innis et al., eds. (1990) PCR Pratocols: A Guide to Methods and Applications (Academic Press, New York); Innis and Gelfand, ads. (1995) PCR Strategies (Academic Press, New York); and Innis and Gelfand, eds. (1999) PCR Methods Manual (Academic Press, New York). Known methods of PGR include, but are not limited to, methods using paired primers, nested primers, single specific primers, degenerate primers, gene-specific primers, vector-specific primers, partially-mismatched primers, and the like.

The *AHASL* polynucleotide sequences of the disclosure are provided in expression cassettes for expression in the plant of interest. The cassette will include 5' and 3' regulatory sequences operably linked to an *AHASL* polynucleotide sequence of the disclosure. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

Such an expression cassette is provided with a plurality of restriction sites for insertion of the *AHASL* polynucleotide sequence to be under the transcriptional regulation of the regulatory regions. The expression cassette may additionally contain selectable marker genes.

The expression cassette will include in the 5'-3' direction of transcription, a transcriptional and translational initiation region (i.e., a promoter), an *AHASL* polynucleotide sequences of the disclosure, and a transcriptional and translanonal termination region (i.e., termination region) functional in plants. The promoter may be native or analogous, or foreign or heterologous, to the plant host and/or to the *AHASL* polynucleotide sequence of the disclosure. Additionally, the promoter may be the natural sequence or alternatively a synthetic sequence. Where the promoter is "foreign" or "heterologous" to the plant host, it is intended that the promoter is not found in the native plant into which the promoter is introduced. Where the promoter is "foreign" or "heterologous" to the *AHASL* polynucleotide sequence of the disclosure, it is intended that the promoter is not the native or naturally occurring promoter for the operably linked *AHASL* polynucleotide sequence of the disclosure. As used herein, a chimeric gene comprises a coding sequence operably linked to a transcription initiation region that is heterologous to the coding sequence.

While it may be desirable to express the *AHASL* polynucleoti des of the: disclosure using heterologous promoters, the native promoter sequences may be used. Such constructs would change expression levels of the AHASL protein in the plant or plant cell. Thus, the phenotype of the plant or plant cell is altered.

The termination region may be native with the transcriptional initiation region, may be native with the operably linked *AHASL* sequence of interest, may be native with the plant host, or may be derived from another source (i.e., foreign or heterologous to the promoter, the *AHASL* polynucleotide sequence of interest, the plant host, or any combination thereof), Convenient termination regions are available from the Ti-plasmid of *A. tumefaciens,* such as the octopine synthase and nopaline synthase termination regions. See also Guerineau et al. (1991) Mo/. Gen. Genet. 262:141-144; Proudfoot (1991) Cell 64:671-674; Sanfacon et al. (1991) Genes Dev. 5: 141-149; Mogen et al, (1990) Plant Cell 14:1261-1272; Munroe et al (1990) Gene 91:151-158; Ballas et al. (1989) Nucleic Acids Res. 17:7891-7903; and Joshi et al. (1987) Nucleic Acid Res. 15:9627-9639.

Where appropriate, the gene(s) may be optimized for increased expression in the transformed plant. That is, the genes can be synthesized using plant-preferred codons for improved expression. See, for example, Campbell and Growri (1990) Plant Physiol. 92:1-11 for a discussion of host-preferred codon usage. Methods are available in the art for synthesizing plant-preferred genes. See, for example, U.S. Patent Nos. 5,380,831, and 5,436,391, and Murray et al. (1989) Nucleic .Acids Res. 17:477-498.

Additional sequence modifications are known to enhance gene expression in a cellular host. These include elimination of sequences encoding spurious polyadenylation signals, exon-intron splice site signals, transposon-like repeats, and other such well-characterized sequences that may be deleterious to gene expression. The G-C content of the sequence may be adjusted to levels average for a given cellular host, as calculated by reference to known genes expressed in the host cell. When possible, the sequence is modified to avoid predicted hairpin secondary mRNA structures.

Nucleotide sequences for enhancing gone expression can also be used in the plant expression vectors. These include the introns of the maize AdbI, intronl gene (Callis et al. Genes and Development 1:1183-1200,1987), and leader sequences, (W-sequence) from the Tobacco Mosaic virus (TMV), Maize Chlorotic Mottle Virus and Alfalfa Mosaic Virus (Gallie et al. Nucleic Acid Res. 15:8693-8711, 1987 and Skuzeski et al. Plant Mol. Biol. 15:65-79,1990). The first intron from the shrunken-1 locus of maize, has been shown to increase expression of genes in chimeric gene constructs. U.S. Pat Nos. 5,424,412 and 5,593,874 disclose the use of specific introns in gene expression constructs, and Gallie et al. (Plant Physiol. 106:929-939,1994) also have shown that introns are useful for regulating gene expression an a tissue specific basis. To further enhance or to optimize AHAS large subunit gene expression, the plant expression vectors of the disclosure may also contain DNA sequences containing matrix attachment regions (MARs). Plant cells transformed with such modified expression systems, then, may exhibit overexpression or constitutive expression of a nucleotide sequence of the disclosure.

The expression cassettes may additionally contain 5' leader sequences in the expression cassette construct. Such leader sequences can act to enhance translation. Translation leaders are known in the art and include: picornavirus leaders, for Example, EMCV leader (Encephalomyocarditis 5' noncoding region) (Elroy-Stein et al. (1989) Proc. Natl. Acad Sci. USA 86:6126-6130); potyvirus leaders, for example, TEV leader (Tobacco Etch Virus) (Gallie et al. (1995) Gene 165(2):233-238), MDMV leader (Maize Dwarf Mosaic Virus) *(*Virology 154:9-20), and human immunoglobulin heavy-chain binding protein (BiP) (Macejak et al, (1991) Nature 353:90-94); untranslated leader from the coat protein mRNA of alfalfa mosaic virus (AMV RNA 4) (Jobling et al. (1987) Nature 325:622-625); tobacco mosaic virus leader (TMV) (Gallie et al. (1989) in Molecular Biology of RNA, ed. Cech (Liss, New York), pp. 237-256); and maize chlorotic mottle virus leader (MCMV) (Lommel et al. (1991) Vitrology 81:382-385). See also, Della-Cioppa. et al. (1987) Plant Physiol 84:965-968. Other methods known to enhance translation can also be utilized, for example, introns, and the like.

In preparing the expression cassette, the various DNA fragments may be manipulated, so as to provide for the DNA sequences in the proper orientation and, as appropriate, in the proper reading frame. Toward this end, adapters or linkers may be employed to join the DNA fragments or other manipulations may be involved to provide for convenient restriction sites, removal of superfluous DNA, removal of restriction sites, or the like. For this purpose, *in vitro* mutagenesis, primer repair, restriction, annealing, resubstitutions, e.g., transitions and transversions, may be involved,

A number of promoters can be used in the practice of the disclosure. The promoters can be selected based on the desired outcome. The nucleic acids can be combined with constitutive, tissue-preferred, or other promoters for expression in plants.

Such constitutive promoters include, for example, the core promoter of the Rsyn7 promoter and other constitutive promoters disclosed in WO 99/43838 and U.S. Patent No. 6,072,050; the core CaMV 35S promoter (Odell et al. (1985) Nature 313:810-812); rice actin (MeElroy et al. (1990) Plant Cell 2: 163-171)*;* ubiquitin (Christensen et al. (1989) Plant Mol. Biol. 12:619-632 and Christensen et al. (1992) Plant Mol. Biol. 18:675-689); pEMU (Last et al. (1991) Theor. Appl. Genet. 81:581-588); MAS (Velten et al. (1984) EMBO J. 3:2723-2730); ALS promoter (U.S. Patent No. 5,659,026), and the like. Other constitutive promoters include, for example, U.S. Patent Nos. 5,608,149; 5,608,144; 5,604,121; 5,569,597; 5,466,785; 5,399,680; 5,268,463; 5,608,142; and 6,177,611.

Tissue-preferred promoters can be utilized to target enhanced AHASL 1 expression within a particular plant tissue. Such tissue-preferred promoters include, but are not limited to, leaf-preferred promoters, root-preferred promoters, seed-preferred promoters, and stem-preferred promoters. Tissue-preferred promoters include Yamamoto et al. (1997) Plant J. 12(2):255-265; Kawamata et al. (1997) Plant Cell Physiol. 38(7):792-803; Hansen et al. (1997) Mol. Gen Genet. 254(3):337-343; Russell et al. (1997) Transgenic Res. 6(2):157-168; Rinehart et al. (1996) Plant Physiol. 112(3):1331-1341; Van Camp et al. (1996) Plant Physiol. 112(2):525-535; Canevascini et al. (1996) Plant Physiol. 112(2):513-524; Yamamoto et at. (1994) Plant Cell Physiol. 35(5):773-778; Lam (1994) Results Probl. Cell Differ. 20:181.196; Orozco et al. (1993) Plant Mol Biol. 23(6):1129-1138; Matsuoka et al. (1993) Proc Natl. Acad. Sci. USA 90(20):9586-9590; and Guevara-Garcia et al. (1993) Plant J. 4(3):495-505. Such promoters can be modified, if necessary, for weak expression

The nucleic acids of interest may be targeted to the chloroplast for expression. In this manner, where the nucleic acid of interest is not directly inserted into the chloroplast, the expression cassette will additionally contain a chloroplast-targeting sequence comprising a nucleotide sequence that encodes a chloroplast transit peptide to direct the gene product of interest to the chloroplasts. Such transit peptides are known in the art. With respect to chloroplast-targeting sequences, "operably linked" means that the nucleic acid sequence encoding a transit peptide (*i.e.,* the chloroplast-targeting sequence) is linked to the *AHASL* polynucleotide of the disclosure such that the two sequences are contiguous and in the same reading frame. See, for example, Von Heijne et al. (1991) Plant Mol Biol. Rep. 9:104-126; Clark et al. (1989) J. Biol. Chem. 264:17544-17550; Della-Cioppa et al. (1987) Plant Plysiol. 84:965-968; Romer et al. (1993) Biochem. Biophys. Res. Commun. 196:1414-1421; and Shah et al. (1986) Science 233:478-481. While the AHASL proteins of the disclosure include a native chloroplast transit peptide, any chloroplast transit peptide known in the art can be fused to the amino acid sequence of a marure AHASL protein of the disclosure by operably linking a choloroplast-targeting sequence to the 5'-end of a nucleotide sequence encoding a mature AHASL protein of the disclosure.

Chloroplast targeting sequences are known in the art and include the chloroplast small subunit of ribulose-1,5-bisphosphate carboxylase (Rubisco) (de Castro Silva Filho et al. (1996) Plant Mol. Biol. 30:769-780; Schnell et al. (1991) J. Biol. Chem. 266(5):3335-3342); 5-(enolpyruvyl)shikimate-3-phosphate synthate (EPSPS) (Archer et al. (1990) J. Bioenerg. Biomemb. 22(6):789-810); tryptophan synthase (Zhao et al. (1995) J. Biol. Chem. 270(11):6081-6087); plastocyanin (Lawrence et al. (1997) J. Biol. Chem. 272(33):20357-20363); chorismate synthase (Schmidt et al. (1993) J. Biol. Chem. 268(36):27447-27457); and the light harvesting chlorophyll a/b binding protein (LHBP) (Lamppa et al. (1988) J. Biol. Chem. 263:14996-14999). See also Von Heijne et al. (1991) Plant Mol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

Methods for transformation of chloroplasts are known in the art. See, for example, Svab et. al. (1990) Proc. Natl. Acad. Sci. USA 87:8526-8530; Svab and Maliga (1993) Proc. Natl. Acad Sci. USA 90:913-917; Svab and Maliga (1993) EMBO J. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

The nucleic acids of interest to be targeted to the chloroplast may be optimized for expression in the chloroplast to account for differences in codon usage between the plant nucleus and this organelle. In this manner, the nucleic acids of interest may be synthesized using ohloroplast-preferred codons, See, for example, U.S. Patent No. 5,380,831.

As disclosed herein, the AHASL nucleotide sequences of the disclosure find use in enhancing the herbicide tolerance of plants that comprise in their genomes a gene encoding a herbicide-tolerant AHASL protein. Such a gene may be an endogenous gene or a transgenic Additionally, the nucleic acid sequences of the present disclosure can be stacked with any combination of polynucleotide sequences of interest in order to create plants with a desired phenotype. For example, the polynucleotides of the present disclosure may be stacked with any other polynucleotides encoding polypeptides having pesticidal and/or insecticidal activity, such as, for example, the *Bacillus thuringiensis* toxin proteins (described in U.S. Patent Nos. 5,366,892; 5,747,450; 5,737,514; 5,723,756; 5,593,881; and Geiser et al. (1986) Gene 48:109). The combinations generated can also include multiple copies of any one of the polynucleotides of interest.

It is recognized that with these nucleotide sequences, antisense constructions, complementary to at least a portion of the messenger RNA (mRNA) for the AHASL polynucleotide sequences can be constructed. Antisense nucleotides are constructed to hybridize with the corresponding mRNA. Modifications of the antisense sequences may be made as long as the sequences hybridize to and interfere with expression of the corresponding mRNA. In this manner, antisense constructions having 70%, preferably 80%, more preferably 85% sequence identity to the corresponding antisensed sequences may be used. Furthermore, portions of the antisense nucleotides may be used to disrupt the expression of the target gene. Generally, sequences of at least 50 nucleotides, 100 nucleotides, 200 nucleotides, or greater may be used.

The nucleotide sequences of the present disclosure may also be used in the sense orientation to suppress the expression of endogenous genes in plants. Methods for suppressing gene expression in plants using nucleotide sequences in the sense orientation are known in the art. The methods generally involve transforming plants with a DNA construct comprising a promoter that drives expression in a plant operably linked to at least a portion of a nucleotide sequence that corresponds to the transcript of the endogenous gene. Typically, such a nucleotide sequence has substantial sequence identity to the sequence of the transcript of the endogenous gene, e.g. greater than about 65% sequence identity, greater than about 85% sequence identity, or greater than about 95% sequence identity. See, U.S. Patent Nos. 5,283,184 and 5,034,323.

While the herbicide-resistant AHASL1 polynucleotides of the disclosure find use as selectable maker genes for plant transformation, the expression cassettes of the disclosure can include another selectable marker gene for the selection of transformed cells. Selectable marker genes, including those of the present disclosure are utilized for the selection of transformed cells or tissues. Marker genes include, but are not limited to, genes encoding antibiotic resistance, such as those encoding neomycin phosphotransferase II (NEO) and hygromycin phosphotransferase (HPT), as well as genes conferring resistance to herbicidal compounds, such as glufosinate ammonium, bromoxynil, imidazolinones, and 2,4-dichlorophenoxyacetate (2,4-D). See generally, Yarranton (1992) Curr. Opin. Biotech. 3:506-511; Christopherson et al. (1992) Proc. Natl. Acad Sci. USA 89:6314-6318; Y ao et al. (1992) Cell 71:63-72;Reznikoff (1992) Mol. Microbiol. 6:2419-2422; Barkley et al. (1980) in The Operon, pp. 177-220; Hu et al. (1987) Cell 48:555-566; Brown et al. (1987) Cell 49:603-612; Figge et al. (1988) Cell 52:713-722; Deuschla et al. (1989) Proc. Natl. Acad. Aci. USA 86:5400-5404; Fuerst et al. (1989) Proc. Natl. Acad. Sci. USA 86:2549-2553; Deuschle et al. (1990) Science 248:480-483; Gossen (1993) Ph.D. Thesis, University of Heidelberg; Reines et al. (1993) Proc. Natl. Acad. Sci. USA 90:1917-1921; Labow et al. (1990) Mol. Cell. Biol. 10:3343-3356; Zambretti et al. (1992) Proc. Natl. Acad. Sci. USA 89:3952-3956; Baim et al. (1991) Proc. Natl. Acad. Sci. USA 88:5072-5076; Wyborski et al. (1991) Nucleic Acids Res. 19:4647-4653; Hillenand-Wissman (1989) Topics Mol. Struc. Biol. 10:143-162; Degenkolb et al. (1991) Antimicrob. Agents Chemother. 35:1591-1595; Kleinschnidt et al. (1988) Biochemistry 27:1094-1104; Bonin (1993) Ph.D. Thesis, University of Heidelberg; Gossen et al. (1992) Proc. Natl. Acad. Sci. USA 89:5547-5551; Oliva et al. (1992) Antimicrob. Agents Chemother. 36:913-919; Hlavka et al. (1985) Harrdbook of Experimental Pharmacology, Vol. 78 (Springer-Verlag, Berlin); Gill et al (1988) Nature 334:721-724.

The above list of selectable marker genes is not meant to be limiting. Any selectable marker gene can be used.

The isolated polynucleotides molecules comprising nucleotide sequence that encode the AHASL proteins of the disclosure can be used in vectors to transform plants so that the plants created have enhanced resistant to herbicides, particularly imidazolinone herbicide. The isolated AHASL polynucleotide molecules of the disclosure can be used in vectors alone or in combination with a nucleotide sequence encoding the small subunit of the AHAS (AHASS) enzyme in conferring herbicide resistance in plants. *See,* U.S. Patent No. 6,348,643 .

The disclosure also relates to a plant expression vector comprising a promoter that drives expression in a plant operably linked to an isolated polynucleotides molecule of the disclosure. The isolated polynucleotide molecule comprises a Nucleotide sequence encoding an AHASL protein, particularly an AHASL protein comprising an amino sequence that is set forth in SEQ ID NO: 2 or 4, or a functional fragment and variant thereof. The plant expression vector of the disclosure does not depend on a particular promoter, only that such a promoter is capable of driving gene expression in a plant cell. Preferred promoters include constitutive promoters and tissue-preferred promoters.

The transformation vectors of the disclosure can be used to produce plants transformed with a gene of interest. The transformation vector will comprise a selectable marker gene of the disclosure and a gene of interest to be introduced and typically expressed in the transformed plant. Such a selectable marker gene comprises a herbicide-resistant AHASL polynucleotide of the disclosure operably linked to a promoter that drives expression in a host cell. For use in plants and plant cells, the transformation vector comprises a selectable marker gene comprising a herbicide-resistant AHASL polynucleotide of the disclosure operably linked to a promoter that drives expression in a plant cell.

The genes of interest of the disclosure vary depending on the desired outcome. For example, various changes in phenotype can be of interest including modifying the fatty acid composition in a plant, altering the amino acid content of a plant, altering a plant's insect and/or pathogen defense mechanisms, and the like. These results can be achieved by providing expression of heterologous products or increased expression of endogenous produots in planta. Alternatively, the results can be achieved by providing for a reduction of expression of one or more endogenous products, particularly enzymes or cofactors in the plant, These changes result in a change in phenotype of the transformed plant.

The genes of interest may include insect resistance genes such as, for example, *Bacillus thuringiensis* toxin protein genes (U.S. Patent Nos. 5,366,892; 5,747,450; 5,736,514; 5,723,756; 5,593,881; and Geiser et al. (1986) Gene 48:109).

The AHASL proteins or polypeptides of the disclosure can be purified from, for example, sunflower plants and can be used in compositions. Also, an isolated polynucleotide molecule encoding an AHAS protein of the disclosure can be used to express an AHASL protein of the disclosure in a microbe such as *E*. *coli* or a yeast The expressed AHASL protein can be purified from extracts of *E. coli* or yeast by any method known to those of ordinary skill in the art.

The disclosure also relates to a method for creating a transgenic plant that is resistant to herbicides, comprising transforming a plant with a plant expression vector comprising a promoter that drives expression in a plant operably linked to an isolated polynucleotide molecule of the disclosure. The isolated polynucleotide molecule comprises a nucleotide sequence encoding an AHASL protein of the disclosure, particularly an AHASL protein comprising: an amino sequence that is set forth in SEQ ID NO: 2, an amino acid sequence encoded by SEQ ID NO: 1, or a functional fragment and variant of said amino acid sequences.

The disclosure also relates to the non-transgenic sunflower plants, transgenic plants produced by the methods of the disclosure, and progeny and other descendants of such non-transgenic and transgenic plants, which plants exhibit enhanced or increased resistance to herbicides that interfere with the AHAS enzyme, particularly imidazolinone and sulfonylurea herbicides.

The AHASL polynucleotides of the disclosure, particularly those encoding herbicide-resistant AHASL proteins, find use in methods for enhancing the resistance of herbicide-tolerant plants. The herbicide-tolerant plants may comprise a herbicide-tolerant or herbicide-resistant AHASL protein. The herbicide-tolerant plants include both plants transformed with a herbicide-tolerant AHASL nucleotide sequences and plants that comprise in their genomes an endogenous gene that encodes a herbicide-tolerant AHASL protein. Nucleotide sequences encoding herbicide-tolerant AHASL proteins and herbicide-tolerant plants comprising an endogenous gene that encodes a herbicide-tolerant AHASL protein include the polynucleotides and plants of the present disclosure and those that are known in the art. *See*, for example, U.S. Patent Nos. 5,013,659, 5,731, 180, 5,767,361, 5,545, 822, 5,736,629, 5,773,703, 5,773,704, 5,952,553 and 6,274, 796.

Such methods for enhancing the resistance of herbicide-tolerant plants comprise transforming a herbicide-tolerant plant with at least one polynucleotide construction comprising a promoter that drives expression in a plant cell that is operably linked to a herbicide-resistant AHASL polynucleotide of the disclosure, particularly the polynucleotide encoding a herbicide-resistant AHASL protein set forth in SEQ ID NO: 1, polynucleotides enooding the amino acid sequence set forth in SEQ ID NO: 2, and fragments and variants said polynucleotides that encode polypeptides comprising herbicide-resistant AHAS activity.

Numerous plant transformation vectors and methods for transforming plants are available. *See,* for example, An., G. et al. (1986) Plant Pysiol, 81:301-305; Fry, J., et al. (1987) Plant Cell Rep. 6:321-325; Block, M. (1988) Theor, Appl Genet.76:767-774; Hinchee, et al. (1990) Stadler. Genet, Symp.203212.203-212; Cousins, et al. (1991) Aust. J. Plant Physiol. 18:481-494; Chee, P. P. and Slightom, J. L. (1992) Gene. 118:255-260; Christou, et al. (1992) Trends. Biotechnol, 10:239-246; D'Halluin, et al, (1992) Bio/Technol. 10:309-314; Dhir, et al. (1992) Plant Physiol, 99:81-88; Casas et al. (1993) Proc. Nat. Acad Sci. USA 90:11212-11216; Christou, P. (1993) In Vitro Cell. Dev.Biol.-Plant; 29P:119-124; Davies, et al. (1993) Plant Cell Rep. 12:180-183; Dong, J. A. and Mchughen, A (1993) Plant Sci. 91:139-148; Franklin. C.I. and Trieu, T. N. (1993) Plant. Physiol. 102:167; Golovkin, et al. (1993) Plant Sci. 90:41-52; Guo Chin Sci, Ball. 38:2072-2078; Asano, et al. (1994) Plant Cell Rep. 13; Ayeres N. M and Park, W. D. (1994) Crit, Rev. Plant. Sci. 13:219-239; Baroelo, et al. (1994) Plant. J, 5: 583-592; Becker, et al. (1994) Plant. J. 5:299-307; Borkowska et al. (1994) Acta Physiol Plant. 16:225-230; Christou, P. (1994) Agro. Food. Ind. Hi Tech. 5: 17-27; Eapen et al. (1994) Plant Cell Rep. 13:582-586; Harrman, et al. (1994) Bio-Technology 12: 919923; Ritaìa, et al, (1994) Plant. Mol. Biol. 24:317-325; and Wan, Y. C. and Lemaux, P. G. (1994) Plant Physiol. 104:3748.

The methods of the disclosure involve introducing a polynucleotide construct into a plant. By "introducing" is intended presenting to the plant the polynucleotide construct in such a manner that the construct gains access to the interior of a cell of the plant The methods of the disclosure do not depend on a particular method for introducing a polynucleotide construct to a plant, only that the polynucleotide construct gains access to the interior of at least one cell of the plant. Methods for introducing polynucleotide constructs into plants are known in the art including, but not limited to, stable transformation methods, transient transformation methods, and virus-mediated methods.

By "stable transformation" is intended that the polynucleotide construct introduced into a plant integrates into the genome of the plant and is capable of being inherited by progeny thereof. By "transient transformation" is intended that a polynucleotide construct introduced into a plant does not integrate into the genome of the plant.

For the transformation of plants and plant cells, the nucleotide sequances of the disclosure are inserted using standard techniques into any vector known in the art that is suitable for expression of the nucleotide sequences in a plant or plant cell. The selection of the vector depends on the preferred transformation technique and the targer plant species to be transformed. An AHASL1 nucleotide sequence may be operably linked to a plant promoter that is known for high-level expression in a plant cell, and this construct is then introduced into a plant that is susceptible to an imidazolinone herbicide and a transformed plant is regenerated. The transformed plant is tolerant to exposure to a level of an imidazolinone, herbicide that would kill or significantly injure an untransformed plant. This method can be applied to any plant species; however, it is most beneficial when applied to crop plants.

Methodologies for constructing plant expression cassettes and introducing foreign nucleic acids into plants are generally known in the art and have been previously described. For example, foreign DNA can be introduced into plants, using tumor inducing (Ti) plasmid vectors. Other methods utilized for foreign DNA delivery involve the use of PEG mediated protoplast transformation, electroporation, microinjection whiskers, and biolistics or microprojectile bombardment for direct DNA uptake. Such methods are known in the art. (U.S. Pat No. 5,405,765 to Vasil et al.*;* Bilang et al. (1991) Gene 100: 247-250; Scheid et al., (1991) Mol. Gen. Genet., 228: 104-112; Guerche et al., (1987) Plant Science 52: 111-116; Neuhause et al., (1987) Theor. Appl Genet 75: 30-36; Klein et al., (1987) Nature 327: 70-73; Howell et al., (1980) Science 208:1265; Horsch et al., (1985) Science 227:1229-1231; DeBlock et al., (1989) Plant Physiology 91: 694-701; Methods for Plant Molecular Biology (Weissbach and Weissbach, eds.) Academic Press, Inc. (1988) and Methods in Plant Molecular Biology (Schuler and Zielinski, eds.) Academic Press, Inc. (1989). The method of transformation depends upon the plant cell to be transformed, stability of vectors used, expression level of gene products and other parameters.

Other suitable methods of introducing nucleotide sequences into plant cells and subsequent insertion into the plant genome include microinjection as Crossway et al. (1986) Biotechniques 4:320-334, electroporation as described by Riggs et al. (1986) Proc. Natl. Acad. Sci. USA 83:5602-5606, *Agrobacterium-*mediated transformation as described by Townsend et al., U.S. Patent No. 5,563,055, Zhao *et al.,* U.S. PatentNo. 5,981,840, direct gene transfer as described by Paszkowski et al. (1984) EMBO J. 3:2717-2722, and ballistic particle acceleration as described in, for example, Sanford et al., U.S. Patent No. 4,945,050; Tomes et al., U.S. Patent No. 5,879,918; Tomes et al., U.S. Patent No. 5,886,244; Bidney et al., U.S. Patent No. 5,932,782; Tomes et al. (1995) "Direct DNA Transfer into Intact Plant Cells via Microprojectile Bombardment," in Plant Cell, Tissue, and Origan Culture: Fundamental Methods, ed. Gamborg and Phillips (Springer-Verlag, Berlin); McCabe et al. (1988) Biotechnology 6:923-926); and Lecl transformation (WO 00/28058). *Also see,* Weissinger et al. (1988) Ann. Rev. Genet. 22:421-477; Sanford et al. (1987) Particulate Science and Technology 5:27-37 (onion); Christou et al. (1988) Plant Physiol. 87:671-674 (soybean); McCabe et al. (1988) Bio Technology 6:923-926 (soybean); Finer and McMullen (1991) In Vitro Cell Dev. Biol, 27P:175-182 (soybean); Singh et al. (1998) Theor. Appl Genet. 96:319-324 (soybean); Datta et al. (1990) Biotechnology 8:736-740 (rice); Klein et al. (1988) Proc. Natl. Acad. Sci. USA 85:4305-4309 (maize); Klein et al, (1988) Biotechnology 6:559-563 (maize); Tomes, U.S. Patent No. 5,240,855; Buising et al., U.S. Patent Nos. 5,322,783 and 5,324,646; Tomes et al. (1995) "Direct DNA Transfer into Intact Plant Cells via Microprojectile Bombardment," in Plant Cell, Tissue, and Origan Culture: Fundamental Methods, ed. Gamborg (Springer-Verlag, Berlin) (maize); Klein et al. (1988) Plant Physiol 91:440-444 (maize); Fromm et al. (1990) Biotechnology 8:833-839 (maize). Hooykaas-Van Slogterenet al. (1984) Nature (London) 311:763-764; Bowen et al., U.S. Patent No. 5,736,369 (cereals); Bytebier et al. (1987) Proc. Natl. Acad Sci. USA 84:5345-5349 (Liliaceae); De Wet et al. (1985) in The Experimental Manipulation of Ovule Tissues, ed. Chapman et al. (Longman, New York), pp. 197-209 (pollen); Kaeppler et al. (1990) Plant Cell Reports 9:415-418 and Kaeppler et al. (1992) Theor. Appl. Genet. 84:560-566 (whisker-mediated transformation); D'Haliuin et al. (1992) Plant Cell 4:1495-1505 (electroporation); Li et al. (1993) Plant Cell Reports 12:250-255 and Christou and Ford (1995) Annals of Botany 75:407-413 (rice); Osjoda et al. (1996) Nature Biotechnology 14:745-750 (maize *via Agrobacterium tumefaciens*)*.*

The polynucleotides of the disclosure may be introduced into plants by contacting plants with a virus or viral nucleic acids. Generally, such methods involve incorporating a polynucleotide construct of the disclosure within a viral DNA or RNA molecule. It is recognized that the an AHASL protein of the disclosure may be initially synthesized as part of a viral polyprotein, which later may be processed by proteolysis *in vivo* or *in vitro* to produce the desired recombinant protein. Further, it is recognized that promoters of the disclosure also encompass promoters utilized for transcription by viral RNA polymarases. Methods for introducing polynucleotide constructs into plants and expressing a protein encoded therein, involving viral DNA or RNA molecules are know in the art. See, for example, U.S. Patent Nos. 5,889,191, 5,889,190, 5,866,785, 5,589,367 and 5,316,93.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristics identified. Two or more generations may be grown to ensure that expression of the desired phenotypic characteristic is stably maintained and inherited and then seeds harvested to ensure expression of the desired phenotypic characteristic has been achieved. In this manner, the present disclosure describes transformed seed (also referred to as "transgenic seed") having a polynucleotide construct of the disclosure, for example, an expression cassette of the disclosure, stably incorporated into their genome.

The present disclosure relates to transformation of any plant species, including, but not limited to, monocots and dicots. Examples of plant species of interest include, but are not limited to, corn or maize (*Zea mays*), *Brassica* sp. (e.g., *B. napus, B. rapa, B. juncea*), particularly those *Brassica* species useful as sources of seed oil, alfalfa (*Medicago sativa*)*,* rice (*Oryza sativa*), rye (*Secale cereale*), sorghum (*Sorghum bicolor*, *Sorghum vulgare*), millet (e.g., pearl millet (*Pennisetum glaucum*), proso millet (*Panicum miliaceum*), foxtail millet (*Setaria italica*), finger millet (*Eleusine coracana*)), sunflower (*Helianthus annuus*), safflower (*Carthamus tinctorius*), wheat (*Triticum aestivum, T. Turgidum ssp. durum*), soybean. (*Glycine max*), tobacco (*Nicotiana tabacum*), potato (*Solanum tuberosum*), peanuts (*Arachis hypogaea*), cotton (*Gossypium barbadense, Gossypium hirsutum*), sweet potato (*Ipomoea batatus*), cassava (*Manihot esculenta*), coffee (*Coffee* spp.), coconut (*Cocos micifera*), pineapple (*Ananas comosus*), citrus trees (*Citrus* spp.), cocoa (*Theobroma cacao*), tea (*Camellia sinensis*), banana (*Musi*a spp.), avocado (*Persea americana*), fig (*Ficus casica*), guava (*Psidium guajava*), mango (*Mangifera indica*), olive (*Olea. europaea*), papaya (*Curica papaya*), cashew (*Amacardium occidentale*), macadamia (*Macadamia integrifolia*), almond (*Prumus amygdalus*), sugar beets (*Beta vulgaris*), sugarcane (*Saccharum* spp.), oats, barley, vegetables, ornamentals, and conifers.

Plants of the present disclosure include crop plants (for example, sunflower, *Brassica sp.,* cotton, sugar beet, soybean, peanut, alfalfa, safflower; tobacco, corn, rice, wheat, rye, barley triticale, sorghum, millet, etc.).

The herbicide-resistant plants of the disclosure find use in methods for controlling weeds. Thus, the present disclosure further describes a method for controlling weeds in the vicinity of a herbicide-resistant plant of the disclosure. The method comprises applying an effective amount of a herbicide to the weeds and to the herbicide-resistant plant, wherein the plant has increased resistance to at least one herbicide, particularly an imidazolinone or sulfonylurea herbicide, when compared to a wild-type plant. In such a method for controlling weeds, the herbicide-resistant plants of the disclosure may be crop plants, including, but not limited to, sunflower, alfalfa, *Brassica sp.,* soybean, cotton, safflower, peanut, tobacco, tomato, potato, wheat, rice, maize, sorghum, barley, rye, millet, and sorghum.

By providing plants having increased resistance to herbicides, particularly imidazolinone and sulfonylurea herbicides, a wide variety of formulations can be employed for protecting plants from weeds, so as to enhance plant growth and reduce competition for nutrients. A herbicide can be used by itself for pre-emergence, post-emergence, pre-planting and at planting control of weeds in areas surrounding the plants described herein or an imidazolinone herbicide formulation can be used that contains other additives. The herbicide can also be used as a seed treatment. Additives found in an imidazolinone or sulfonylurea herbicide formulation include other herbicides, detergents, adjuvants, spreading agents, sticking agents, stabilizing agents, or the like. The herbicide formulation can be a wet or dry preparation and can include, but is not limited to, flowable powders, emulsifiable concentrates and liquid concentrates. The herbicide and herbicide formulations can be applied in accordance with conventional methods, for example, by spraying, irrigation, dusting, or the like.

The present disclosure describes non-transgenic and transgenic seeds with increased tolerance to at least one herbicide, particularly an AHAS-inhibiting herbicide, more particularly imidazolinone and sulfonylurea herbicides. Such seeds include, for example, non-transgenic sunflower seeds comprising the herbicide-tolerance characteristics of the sunflower plant S4897, the sunflower plant GM40, the sunflower plant GM1606, the sunflower plant with ATCC Patent Deposit Number PTA-6716, or the sunflower plant with ATCC Patent Deposit Number PTA-7606, and transgenic seeds comprising a polynucleotide molecule of the disclosure that encodes a herbicide-resistant AHASL protein.

The present disclosure describes methods for producing a herbicide-resistant plant, particularly a herbicide-resistant sunflower plant, through conventional plant breeding involving sexual reproduction. The methods comprise crossing a first plant that is resistant to a herbicide to a second plant that is not resistant to the herbicide. The first plant can be any of the herbicide resistant plants of the present disclosure including, for examples, transgenic plants comprising at least one of the polynucleotide molecules of the present disclosure that encode a herbicide resistant AHASL protein and non-transgenic sunflower plants that comprise the herbicide-tolerance characteristics of the sunflower plant S4897, the sunflower plant GM40, the Sunflower plant GM1606, the sunflower plant with ATCC Parent Deposit Number PTA-6716, or the Sunflower plant with ATCC Patent Deposit Number PTA-7606. The second plant can be any plant that is capable of producing viable progeny plants (i.e., seeds) when crossed with the first plant Typically, but not necessarily, the first and second plants are of the same species. The methods of the disclosure can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant The methods of the disclosure can additionally involve selecting plants that comprise the herbicide tolerance characteristics of the first plant.

The present disclosure further describes methods for increasing the herbicide-resistance of a plant, particularly a herbicide-resistant sunflpower plant, through conventional plant breeding involving sexual reproduction. The methods comprise crossing a first plant that is resistant to a herbicide to a second plant that may or may not be resistant to the herbicide or may be resistant to different herbicide or herbicides than the first plant The first plant can be any of me herbicide resistant plants of the present disclosure including, for examples, transgenic plants comprising at least one of the polynucleotide molecules of the present disclosure that encode a herbicide-resistant AHASL protein and non-transgenic sunflower plants that comprise the herbicides-tolerance characteristics of the sunflower plant S4897, the sunflower plant GM40, the sunflower plant GM1606, the sunflower plant with ATCC Patent Deposit Number PTA-6716, or the sunflower plant with ATCC Patent Deposit Number PTA-7606. The second plant can be any plant that is capable of producing viable progeny plants (i.e., seeds) when crossed with the first plant Typically, but not necessarily, the first and second plants are of the same species. The progeny plants produced by this method of the present disclosure have increased resistance to a herbicide when compared to either the first or second plant or both. When the first and second plants are resistant to different herbicides, the progeny plants will have the combined herbicide tolerance characteristics of the first and second plants. The methods of the disclosure can further involve one or more generations of backcrossing the progeny plants of the first cross to a plant of the same line or genotype as either the first or second plant. Alternatively, the progeny of the first cross or any subsequent cross can be crossed to a third plant that is of a different line or genotype than either the first or second plant. The methods of the disclosure can additionally involve selecting plants that comprise the herbicide tolerance characteristics of the first plant, the second plant, or both the first and the second plant.

The plants of the present disclosure can be transgenic or non-tranefrenic. An example of a non-transgenic sunflower plant having increased resistance to imidazolinone is the sunflower plant sunflower plant S4897, the sunflower plant GM40, the sunflower plant GM1606, the sunflower plant wi th ATCC Patent Deposit Number PTA-6716, or the sunflower plant with ATCC Patent Deposit Number Pita-7606, or mutant, recombinant, or a. genetically engineered derivative of the sunflower plant S4897, the sunflower plant GM40, the sunflower plant GM1606, the sunflower plant with ATCC Patent Deposit Number PTA-6716, or the sunflower plant with ATCC Patent Deposit Number PTA-7606; or of any progeny of the sunflower plant S4897, the sunflower plant GM40, the sunflower plant GM1606, the sunflower plant with ATCC Patent Deposit Number PTA-6716, or the sunflower plant with ATCC Patent Deposit Number PTA-7606; or a plant that is a progeny of any of these plants; or a plant that comprises the herbicide tolerance characteristics of the sunflower plant S4897, the sunflower plant GM40, the sunflower plant GM1606, the sunflower plant with ATCC Patent Deposit Number PTA-6716, or the sunflower plant with ATCC Patent Deposit Number PTA-7606.

The present disclosure also describes plants, plant organs, plant tissues, plant cells, seeds, and non-human host cells that are transformed with the at least one polynucleotide molecule, expression cassette, or transformation vector of the disclosure. Such transformed plants, plant organs, plant tissues, plant cells, seeds, and non-human host cells have enhanced tolerance or resistance to at least one herbicide, at levels of the herbicide that kill or inhibit the growth of an untransformed plant, plant tissue, plant cell, or non-human host cell, respectively. The transformed plants, plant tissues, plant cells, and seeds of the disclosure may be *Arabidopsis thaliana* and crop plants.

The present disclosure describes methods that involve the use of at least one AHAS-inhibiting herbicide selected from the group consisting of imidazolinone herbicides, sulfonylurea herbicides, triazolopyrimidine herbicides, pyrimidinyloxybenzoate herbicides, sulfonylamino-carbonyltriazolinone herbicides, and mixtures thereof. In these methods, the AHAS-inhibiting herbicide can be applied by any method known in the art including, but not limited to, seed treatment, soil treatment, and foliar treatment.

Prior to application, the AHAS-inhibiting herbicide can be converted into the customary formulations, for example solution, emulsions, suspensions, dusts, powders, pastes and granules. The use form depends on the particular intended purpose; in each case, it should ensure a fine and even distribution of the compound according to the disclosure.

The formulations are prepared in a known manner (see e.g. for review US 3,060,084, EP-A 707 445 (for liquid concentrates), Browning, "Agglomeration", Chemical Engineering, Dec. 4, 1957, 147-48, Perry's Chemical Engineer's Handbook, 4th Ed., McGraw-Hill. New York 1963, pages 8-57 and et seq. WO 91/13546, US 4,172,714, US 4,144,050, US 3,920,442, US 5,180,587, US 5,232,701, US 5,208,030, GB 2,095,558, US 3,299,566, Kiingman, Weed Control as a Science, John Wiley and Sons, Inc., New York, 1961, Hance et al., Weed Control Handbook, 8th Ed., Blackwell Scientific Publications, Oxford, 1989 and Mollet, H., Grubemann, A., Formulation technology. Wiley VCH Verlag GmbH, Weinheim (Germany), 2001, 2. D. A. Knowles, Chemistry and Technology of Agrocbemical Formulations, Kluwer Academic Publishers, Dordrecht, 1998 (ISBN 0-7514-0443-8), for example by extending the active compound with auxiliaries suitable for the formulation of agrochemicals, such as solvents and/or carriers, if desired emulsifier, surfactants and dispersants, preservatives, antifoaming agents, anti-freezing agents, for seed treatment formulation also optionally colorants and/or binders and/or gelling agents.

Example of suitable solvents are water, aromatic solvents (for example Solvesso products, xylene), paraffins (for example mineral oil fractions), alcohols (for example methanol, butanol, pentanol, benzyl alcohol), ketones (for example cyclobexanone, gamma-butyrolactone), pyrrolidones (NMP. NOP), acetates (glycol diacetate), glycols, fatty acid dimethylamides, fatty acids and fatty acid esters. In principle, solvent mixtures may also be used.

Examples of suitable carriers are ground natural minerals (for example kaolins, clays, talc, chalk) and ground synthetic minerals (for example highly disperse silica, silicates).

Suitable emulsifiers are nonionic and anionic emulsifiers (for example polyoxyethylene fatty alcohol ethers, alkylsulfonates and arylsulfonates).

Examples of dispersants are lignin-sulfite waste liquors and methylcellulose,

Suitable surfactants used are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenol ether, ethoxylated isooctylphenol, octylphenol, nonylphenol, alkylphenol polyglycol ethers, tributylphenyl polyglycol ether, tristearylphenyl polyglycol ether, alkylaryl polyether alcohols, alcohol and fatty alcohol ethylene oxide condensates, ethoxylated castor oil, polyoxyethylene alkyl ethers, ethoxylated polyoxypropylene, lauryl alcohol polyglycol ether acetal, sorbitol esters, lignosulfite waste liquors and methylcellulose.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, highly polar solvents, for example dimethyl sulfoxide, N-methylpyrrolidone or water.

Also anti-freezing agents such as glycerin, ethylene glycol, propylene glycol and bactericides such as can be added to the formulation.

Suitable antifoaming agents are for example antifoaming agents based on silicon or magnesium stearate.

Suitable preservatives are for example Dichlorophen und enzylalkoholhemiformal.

Seed Treatment formulations may additionally comprise binders and optionally colorants.

Binders can be added to improve the adhesion of the active materials on the seeds after treatment Suitable binders are block copolymers EO/PO surfactants but also polyvinylalcoholsl, polyvinylpyrrolidones, polyacrylates, polymethacrylates, polybutenes, polyisobutylenes, polystyrene, polyethyleneamines, polyethyleneamides, polyethyleneimines (Lupasol®, Polymin®), polyethers, polyurethans, polyvinylacetate, tylose and copolymers derived from these polymers.

Optionally, also colorants can be included in the formulation. Suitable colorants or dyes for seed treatment formulations are Rhodamin B, C.I. Pigment Red 112, C.I. Solvent Red 1, pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

An example of a suitable gelling agent is carrageen (Sauagel^{®}).

Powders, materials for spreading, and dustable products can be prepared by mixing or concomitantly grinding the active substances with a solid carrier.

Granules, for example coated granules, impregnated granules and homogeneous granules, can be prepared by binding the active compounds to solid carriers. Examples of solid carriers are mineral earths such as silica gels, silicate, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the AHAS-inhibiting herbicide. In this case, the AHAS-inhibiting herbicides are employed in a purity of from 90% to 100% by weight, preferably 95% to 100% by weight (according to NMR spectrum). For seed treatment purposes, respective formulations can be diluted 2-10 fold leading to concentrations in the ready to use preparations of 0.01 to 60% by weight active compound by weight, preferably 0.1 to 40% by weight.

The AHAS-inhibiting herbicide can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; they are intended to ensure in each case the finest possible distribution of the AHAS-inhibiting herbicide according to the disclosure.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. However, it is also possible to prepare concentrates composed of active substance, wetter, tackifer, dispersant or emulsifier and, if appropriate, solvent or oil, and such concentrates are suitable for dilution with water.

The active compound concentrations in the ready-to-use preparations can be varied within relatively wide ranges. In general, they are from 0.0001 to 10%, e.g. from 0.01 to 1% per weight.

The AHAS-inhibiting herbicide may also be used successfully in the ultra-low-volume process (ULV), it being possible to apply formulations comprising over 95% by weight of active compound, or even to apply the active compound without additives.

The following are examples of formulations:
1. Products for dilution with water for foliar applications. For seed treatment purposes, such products may be applied to the seed diluted or undiluted.
   A) Water-soluble concentrates (SL, LS)
      Ten parts by weight of the AHAS-inhibiting herbicide are dissolved in 90 parts by weight of water or a water-soluble solvent. As an alternative, wetters or other auxiliaries are added. The AHAS-inhibiting herbicide dissolves upon dilution with water, whereby a formulation with 10 % (w/w) of AHAS-inhibiting herbicide is obtained.
   B) Dispersible concentrates (DC)
      Twenty parts by weight of the AHAS-inhibiting herbicide are dissolved in 70 parts by weight of cyclohexanone with addition of 10 parts by weight of a dispersant, for example polyvinylpyrrolidone. Dilution with water gives a dispersion, whereby a formulation with 20% (w/w) of AHAS-inhibiting herbicide is obtained.
   C) Emulsifiable concentrates (EC)
      Fifteen parts by weight of the AHAS-inhibiting herbicide are dissolved in 7 parts by weight of xylene with addition of calcium dodecylbenzenesulfonate and castor oil ethoxylate (in each case 5 parts by weight). Dilution with water gives an emulsion, whereby a formulation with 15% (w/w) of AHAS-inhibiting herbicide is obtained.
   D) Emulsions (EW, EO, ES)
      Twenty-five parts by weight of the AHAS-inhibiting herbicide are dissolved in 35 parts by weight of xylene with addition of calcium dodecylbenzenesulfonate and castor oil ethoxylate (in each case 5 parts by weight). This mixture is introduced into 30 parts by weight of water by means of an emulsifier machine (e.g. Ultraturrax) and made into a homogeneous emulsion. Dilution with water gives an emulsion, whereby a formulation with 25% (w/w) of AHAS-inhibiting herbicide is obtained.
   E) Suspensions (SC, OD, FS)
      In an agitated ball mill, 20 parts by weight of the AHAS-inhibiting herbicide are comminuted with addition of 10 parts by weight of dispersants, wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained.
   F) Water-dispersible granules and water-soluble granules (WG, SG) Fifty parts by weight of the AHAS-inhibiting herbicide are ground finely with addition of 50 parts by weight of dispersants and wetters and made as water-dispersible or water-soluble granules by means of technical appliances (for example extrusion, spray tower, fluidized bed). Dilution with water gives a stable dispersion or solution of the AHAS-inhibiting herbicide, whereby a formulation with 50% (w/w) of AHAS-inhibiting herbicide is obtained.
   G) Water-dispersible powders and water-soluble powders (WP, SP, SS, WS)
      Seventy-five parts by weight of the AHAS-inhibiting herbicide are ground in a rotor-stator mill with addition of 25 parts by weight of dispersants, wetters and silica gel. Dilution with water gives a stable dispersion or solution of the AHAS-inhibiting herbicide, whereby a formulation with 75% (w/w) of AHAS-inhibiting herbicide is obtained.
   I) Gel-Formulation (GF)
      In an agitated ball mill, 20 parts by weight of the AHAS-inhibiting herbicide are comminuted with addition of 10 parts by weight of dispersants, 1 part by weight of a gelling agent wetters and 70 parts by weight of water or of an organic solvent to give a fine AHAS-inhibiting herbicide suspension. Dilution with water gives a stable suspension of the AHAS-inhibiting herbicide, whereby a formulation with 20% (w/w) of AHAS-inhibiting herbicide is obtained. This gel formulation is suitable for us as a seed treatment.
2. Products to be applied undiluted for foliar applications. For seed treatment purposes, such products may be applied to the seed diluted.
   A) Dustable powders (DP, DS)
      Five parts by weight of the AHAS-inhibiting herbicide are ground finely and mixed intimately with 95 parts by weight of finely divided kaolin. This gives a dustable product having 5% (w/w) of AHAS-inhibiting herbicide.
   B) Granules (GR, FG, GG, MG)
      One-half part by weight of the AHAS-inhibiting herbicide is ground finely and associated with 95.5 parts by weight of carriers, whereby a formulation with 0.5% (w/w) of AHAS-inhibiting herbicide is obtained. Current methods are extrusion, spray-drying or the fluidized bed. This gives granules to be applied undiluted for foliar use.

Conventional seed treatment formulations include for example flowable concentrates FS, solutions LS, powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds.

A FS formulation is used for seed treatment. Typacally, a FS formulation may comprise 1-800 g/l of active ingredient, 1-200 g/l Surfactant, 0 to 200 g/l antifreezing agent, 0 to 400 g/l of binder, 0 to 200 g/l of a pigment and up to 1 liter of a solvent, e.g. water.

The present disclosure non-transgenic and transgenic seeds of the herbicide-resistant plants of the present disclosure. Such seeds inolude, for example, non-transgenic sunflower seeds comprising the herbicide-tolerance characteristics of the plant with NCIMB Accession Number NCIMB 41262, and transgenic seeds comprising a polynucleotide molecule of the disclosure that encodes an IMI protein.

For seed treatment, seeds of the herbicide resistant plants according of the present disclosure are treated with herbicides, e.g. herbicides selected from the group consisting of AHAS-inbibiting herbicides such as amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, clorantsulam, diclosulam, florasulam, flumetsulam, matosulam, penoxsulam, bispyribac, pyriminobac, propoxycarbazone, flucarbazone, pyribenzoxim, pyriftalid, pyrithiobac, and mixtures thereof, or with a formulation comprising a AHAS-inhibiting herbicide,

The term seed treatment comprises all suitable seed treatment techniques known in the art, such as seed dressing, seed coating, seed dusting, seed soaking, and seed pelleting.

Also disclosed herein
is a method of treating soil by the application, in particular into the seed drill: either of a granular formulation containing the AHAS-inhibiting herbicide as a composition/formulation (e.g, a granular formulation, with optionally one or more solid or liquid, agriculturally acceptable carriers and/or optionally with one or more agriculturally acceptable surfactants. This method is advantageously employed, for example, in seedbeds of cereals, maize, cotton, and sunflower.

The present disclosure also comprises seeds coated with or containing with a seed treatment formulation comprising at least one AHAS-inhibiting herbicide selected from the group consisting of amidosulfuron, azimsulfuron, bensulfuron, chlorimuron, chlorsulfuron, cinosulfuron, cyclosulfamuron, ethametsulfuron, ethoxysulfuron, flazasulfuron, flupyrsulfuron, foramsulfuron, halosulfuron, imazosulfuron, iodosulfuron, mesosulfuron, metsulfuron, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, imazamethabenz, imazamox, imazapic, imazapyr, imazaquin, imazethapyr, cloransulam, diclosulam, florasulam, flumetsulam, metosulam, penoxsulam, bimpyribac, pyriminobac, propoxycarbazone, flucarbazone, pyribenzoxim, pyriftalid and pyrithiobac.

The term seed embraces seeds and plant propagules of all kinds including but not limited to true seeds, seed pieces, suckers, corms, bulbs, fruit, tubers, grains, cuttings, cut shoots and the like and means in a preferred embodiment true seeds.

The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

The seed treatment application with the AHAS-inhibiting herbicide or with a formulation comprising the AHAS-inhibiting herbicide is carried out by spraying or dusting the seeds before sowing of the plants and before emergence of the plants.

In the treatment of seeds, the corresponding formulations are applied by treating the seeds with an effective amount of the AHAS-inhibiting herbicide or a formulation comprising the AHAS-inhibiting herbicide. Herein, the application rates are generally from 0.10 g to 10 kg of the a.i. (or of the mixture of a.i. or of the formulation) per 100 kg of seed, preferably from 1 g to 5 kg per 100 kg of seed, in particular from 1 g to 2.5 kg per 100 kg of seed. For specific crops such as lettuce the rate can be higher.

The present disclosure describes a method for combating undesired vegetation or controlling weeds comprising contacting the seeds of the resistant plants according to the present disclosure before sowing and/or after pregermination with an AHAS-inhibiting herbicide. The method can further comprise slowing the seeds, for example, in soil in a field or in a potting medium in greenhouse. The method finds particular use in combating undesired vegetation or controlling weeds in the immediate vicinity of the seed.

The control of undesired vegetation is understood as meaning the killing of weeds and/or otherwise retarding or inhibiting the normal growth of the weeds. Weeds, in the broadest sense, are understood as meaning all those plants which grow in locations where they are undesired.

The weeds of the present disclosure include for example, dicotyledonous and monocotyledonous weeds. Dicotyledonous weeds include, but are not limited to, weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Ciraium, Carduus, Sonchus, Solanum, Rorippa, Rotala, Lindernia. Lamium, Veronica, Abutilon, Emex, Datura, Viola, Galeopsis, Papaver, Centaurea, Trifolium, Ranunculus, and Taraxacum. Monocotylodonous weeds include, but are not limited to, weeds of of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristyelis, Sagittaria, Eleocharis, Scirpus, Paspalum, Ischaemum, Sphenoclea, Dactyloctenium, Agrostis, Alopecurus, and Apera.

In addition, the weeds of the present disclosure can include, for example, crop plants that are growing in an undesired location. For example, a volunteer maize plant that is in a field that predominantly comprises soybean plants can be considered a weed, if the maize plant is undesired in the field of soybean plants.

The articles "a" and "an" are used herein to refer to one or more than one (i.e., to at least one) of the grammatical object of the article. By way of example, "an element," means one or more elements.

As used herein, the word "comprising," or variations such as "comprises" or "comprising," will be understood to imply the inclusion of a stated element, integer or step, or group of elements, integers or steps, but not the exclusion of any other element, integer or step, or group of elements, integers or steps.

### EXAMPLE 1: Mutagenesis of Helianthus annuus Line BTK47 and Selection of Imidazolinone-Resistant Plants

BTK47, a *Helianthus annuus* L. breeding line, was chemically mutated as follows. Sixty thousand seeds of the BTK47 line were treated with a solution of ethyl methanesulfonate (EMS) for 15 hours. The treated seeds were then sown under field conditions on December 16, 2002 at Nidera Experimental Station in Venado Tuerto, Santa Fe, Argentina. Approximately 30,000 M₁ plants flowored and were covered with bags in order to self-pollinate each plant. Bach plant was harvested and threshed by hand. On May 10, 2003 at Nidera Experimental Station in Formosa, 20 M₂ seeds from each capitulum were sown under greenhouse conditions. Approximately 590,000 plants were sprayed at V2-V4 stage with imazapyr at a rate of 80 g ai/ha. Eight plants survived the herbicide treatment and were self-pollinated and harvested. M₃ seeds from each of these eight plants were evaluated for their resistance to imazapyr. Out of these eight M₃ families, one family (S4897) segregated for herbicide resistance at a ratio of 1 resistant (i.e., conferring tolerance to commercial rates of imidazolinone herbicides): 2 intermediate (i.e., partially resistant): 1 susceptible. Fully fertile resistant plants were self pollinated and harvested in order to propagate the line S4897.

Leaf tissue from the M3 S4897 plants from both the resistant and intermediate classes were used a source ofDNA for analysis of the sequence of the AHASL1 gene as described in Example 2.

### EXAMPLE 2: PCR Amplification and Sequencing of Sunflower Polynucleotides Encoding Imidazolinone-Resistant and Wild-Type AHASL1 Proteins

The *AHASL1* gene was PCR amplified from DNA isolated from M₃ S4897 and BTK47 (wild-type) sunflower plants as two overlapping fragments. PCR amplification was accomplished with Hotstart Taq DNA polymerase and associated reagents (Qiagen Inc, Valencia, California, USA; Cat. No. 203205) using standard methods. The PCR primers for the two fragments are set forth in Table 1 and in the Sequence Listing. HA1U409 (SEQ ID NO: 7) is the forward primer for the first fragment and corresponds to base pair 409 of GenBank Accession No. AY124092. HA1L1379 (SEQ ID NO: 8) is the reverse primer for the first fragments and corresponds to base pair 1379 of GenBank Accession No. AY124092. HA1U1313 (SEQ ID NO: 9) is the forward primer for the second fragment and corresponds to base pair 1313 of GenBank Accession No. AY124092. HA1L2131 (SEQ ID NO: 10) is the reverse primer for the second fragment and corresponds to base pair 2131 of GenBank Accession No. AY124092. The primer pair HA1U409-HA1L1379 produced a 970 base pair fragment. The primer pair HA1U1313-HA1L2131 produced an 818 base pair fragment.

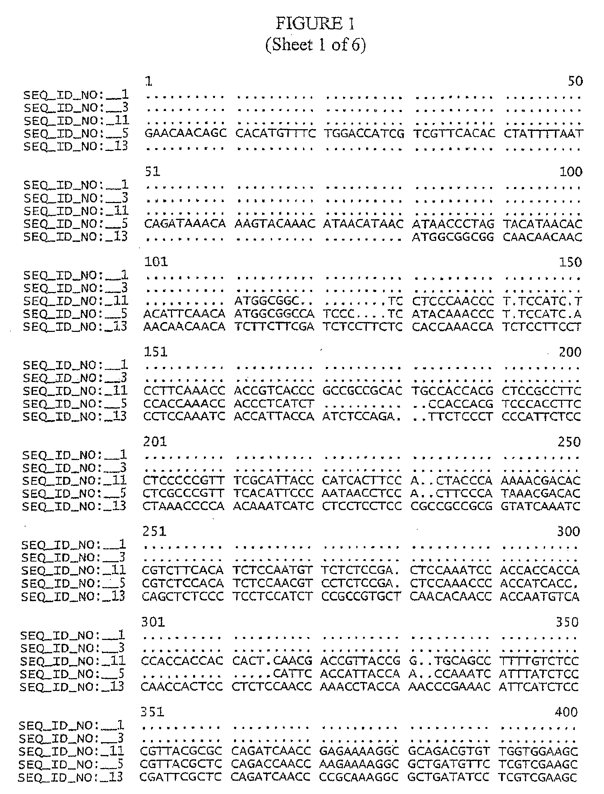

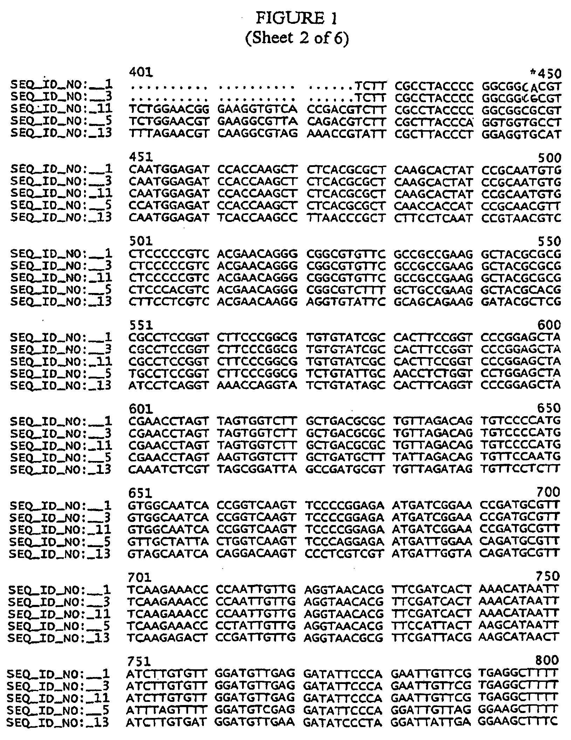

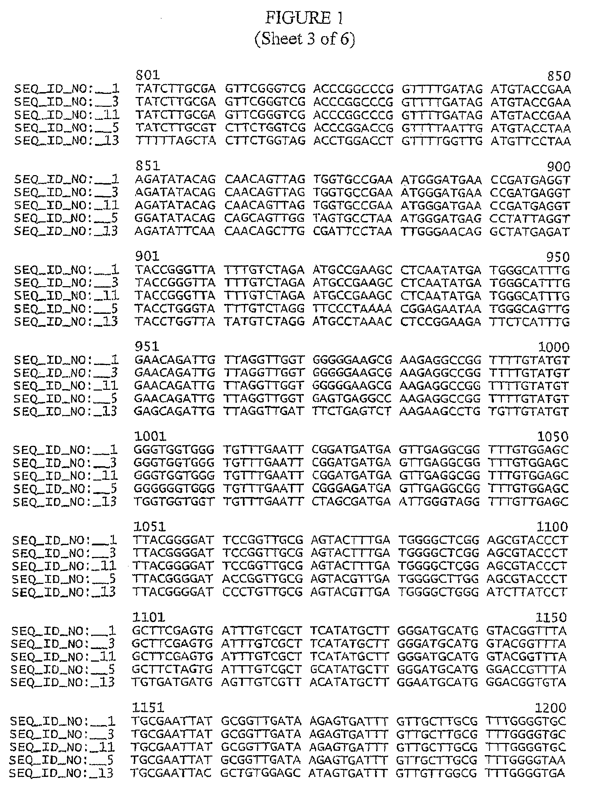

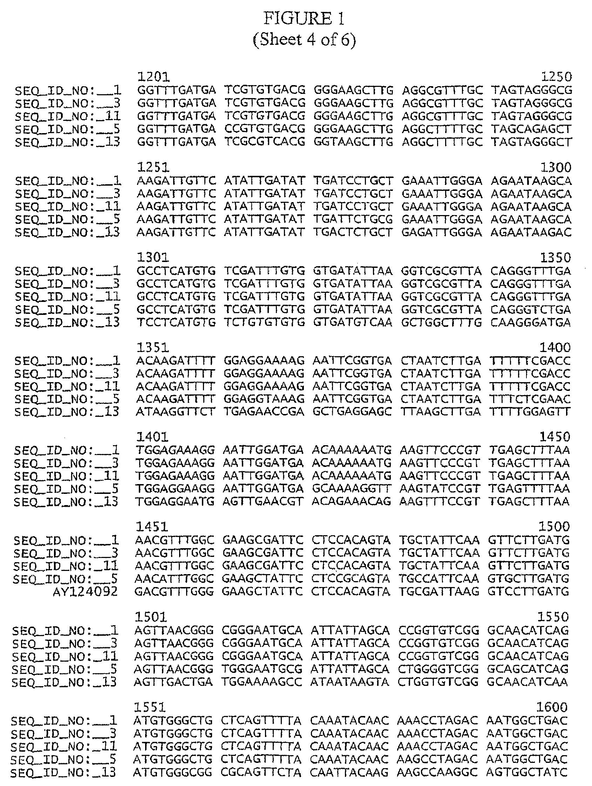

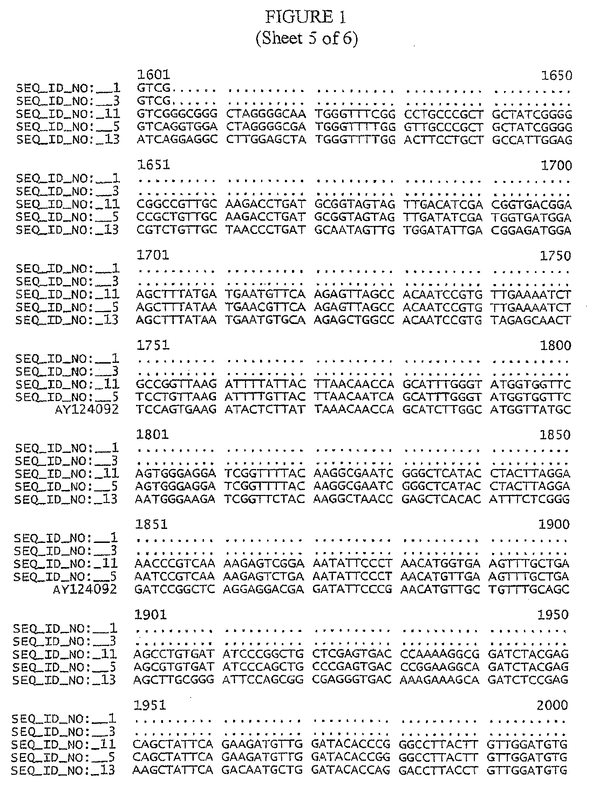

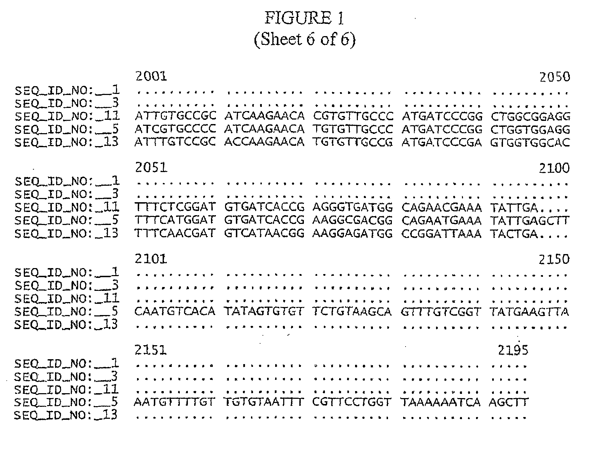

The resulting PCR products were sequenced to produce the AHASL1 sequences for S4897 and BTK47. An alignment of these nucleotide sequences and the nucleotide sequence of the *Xanthium sp.* ALS gene (GenBank Accession No. U16280; SEQ ID NO: 5) is provided in Figure 1. The alignment revealed that the *AHASL1* gene from S4897 had a single mutation relative to the AHASL1 of BTK47. The site of the mutation is indicated by an asterisk in Figure 1. This mutation is a G-to-A transition that corresponds to Nucleotide 21 of SEQ ID NO: 1.

An alignment of the predicted amino acid sequences of the *AHASL1* nucleotide sequences of S4897, BTK47, and *Xanthium sp.* is provided in Figure 2. Relative to the AHASL1 amino add sequence of BTK47, the AHASL1 amino acid sequence of S4897 has an alanine-to-threonine substitution at amino acid position 7 (SEQ ID NO: 2). This amino acid position in SEQ ID NO: 2 corresponds to amino acid position 107 in the full-length amino acid sequence encoded by the sunflower AHASL1 nucleotide sequence of GenBank Accession No. AY541451 (SEQ ID NO:11) and amino acid position 122 in the full-length amino acid sequence encoded by the *Arabidopsis thaliana* AHASL nucleotide sequence of GenBank Accession No. X51514.

**Table 1. PCR Primers for Amplifying the Coding Region of the Sunflower AHASL1 Gene**

| **Primer Name** | **Primer Sequence** |
|---|---|
| HA1U409 | CAGACGTGTTGGTGGAAGC (SEQ ID NO: 7) |
| HA1L1379 | CTGTAACGCGACCTTAATATC (SEQ ID NO: 8) |
| HA1U1313 | TGCTGAAATTGGGAAGAATAAG (SEQ ID NO: 9) |
| HA1L2131 | TTTCGTTCTGCCATCACCC (SEQ ID NO: 10) |

### EXAMPLE 3: Production of a Sunflower Line that is Homozygous for the Herbicide Resistance Characteristics of S4897

A sunflower line that is homozygous for the herbicide-resistance trait was produced from S4897 by selfing and screening resistance to imazapyr. This sunflower line and plants thereof were designated as GM40. After confirming the homogeneity of the progeny for herbicide resistance, plants from the GM40 sunflower line were transplanted under greenhouse conditions and self-pollinated for seed production.

### EXAMPLE 4: Resistance of S4897 to Imazapic

In field trials in Argentina, S4897 plants were tested for resistance to imazapic. The resistance to imazapic was clearly superior to the IMISUN-1 (Ala₁₉₀-to-Val mutation; equivalent position in Arabidopsis thaliana is 205; see Table 4 below). This trial had single row plots and was treated on a windy day, so the actual herbicide dose reaching the plants was likely less than was applied. Nevertheless, S4897 displayed a superior tolerance to imazapic than the Ala₁₉₀-to-Val, substitution we present no data from this field trial.

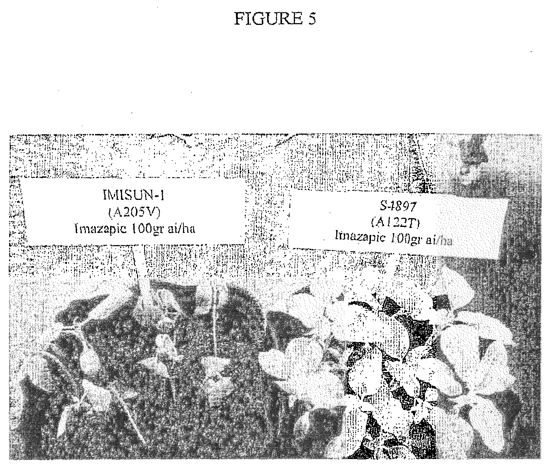

Plants of S4897 and IMISUN-1 were also subjected to imazapic treatment under greenhouse conditions. The photograph in Figure 5 shows this comparison when the plants were treated with 100 g ai/ha of imazapic.

### EXAMPLE 5: Summary of Tolerance and AHAS Activity for Sunflower Line S4897 and Other Clearfield® Varieties of Herbicide-Resistant Sunflower

Methods: Sunflower lines S4897, Clearfield® sunflower varieties A, B, C and a conventional non-Clearfield variety were sprayed with three rates of imazamox (Raptor™), 100, 200 and 300 gm ai/ha plus 0.5% Sun It II and two rates of imazapyr (Arsenal™) 160 and 360 gm ai/ha plus 0.5% Sun It II when the plants were at the two to three leaf stage. Ratings were taken at 14 days after application (DAT) for injury. Injury was rated on a scale from 0 to 9 where 0 = no injury to 9 = dead plant Twelve plants were sprayed per herbicide/rate treatment. Statistical analysis was conducted with STATGRAPHICS Plus 5.0 by using ANOVA and LSD procedures.

AHAS activity analysis was also conducted by selecting actively growing young leaves from plants that were not sprayed with herbicide at approximately four weeks after planting.

Results: After plants were sprayed, it was realized that a height difference between S4897 and other sunflower varieties would have impacted the actual herbicide dose that was delivered. The spray boom height had been calibrated against S4897 and since the other varieties were taller and closer to the boom they would have received a greater dose of herbicide. Rates were recalibrated for the other varieties to determine the approximate dose they would have received. Assessments were made as presented in Table 2 since it was not possible to make a direct rate comparison due to the effect of the height For example injury scores were compare for S4897 treated imazamox rate 100 gm ai/ha to the other varieties treated at 75 gm ai/ha. The treatment dose of 300 gm ai/ha for line S4897 was equivalent to the treatment of 200 gm ai/ha for the other varieties, which approximately was 300 gm ai/ha.

**Table 2. Adjustment due/herbicide dose received**

| | Actual dose received (gm ai/ha) | | Comparison made (gm ai/ha) | |
|---|---|---|---|---|
| Herbicide | S4897 | Other varieties | S4897 | Other varieties |
| Imazamox | 50 | 75 | | |
| | 100 | 150 | 100 | 75 |
| | 200 | 300 | 200 | 150 |
| | 300 | 450 | 300 | 300 |
| Imazapyr | 160 | 240 | | |
| | 360 | 540 | 360 | 240 |

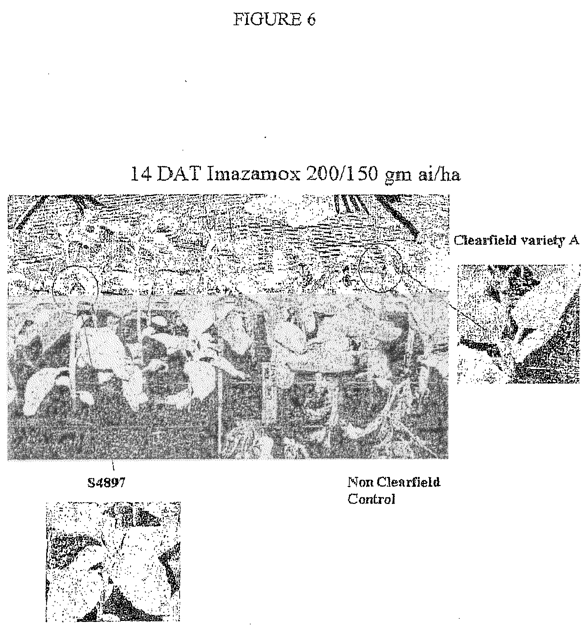

Line S4897 had significantly less injury across all herbicide treatments (Table 3). In fact very little injury was observed at the highest rate of either imazamox or imazapyr. The other varieties showed increasing injury across herbicide rate as would be expected. Figure 6 shows a comparison of the injury at the 200/150 gm ai/ha for line S4897, Clearfield® variety A, and the non-Clearfield control. The growing tip of S4897 showed little to no injury whereas Clearfield® variety A was significantly in juried and had stopped growing.

**Table 3. Injury data at 14 DAT for three IMISUN1 lines and S4897 sprayed with imazamox or imazapyr.**

| | | Imazamox (gm ai/ha) | | Imazapyr (gm ai/ha) |
|---|---|---|---|---|
| Lines | 100/75 | 200/150 | 300 | 360/240 |
| S4897 | 0.5 a | 0.8 a | 0.8 a | 0.8 a |
| Clearfield-Sunflower variety A | 4.3 c | 4.6 c | 6.5 d | 5.0 c |
| Clearfield-Sunflower variety B | 1.9 b | 4.8 c | 5.3 c | 4.3 b |
| Clearfield-Sunflower variety C | 1.8 b | 2.9 b | 4.6 b | 4.9 c |
| Conventional non-Clearfield | 7.2 d | 8.2 d | 8.7 e | 8.2 d |
| | LSD = 0.7 | LSD = 0.7 | LSD = 0.6 | LSD = 0.7 |

| | | | | |
|---|---|---|---|---|
| Statistical analysis was conducted with STATORAPHICS Plus 5.0 Each value is the mean of approximate 12 observation | | | | |

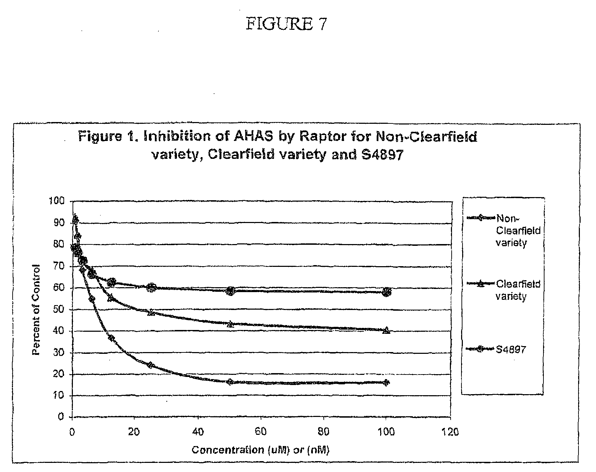

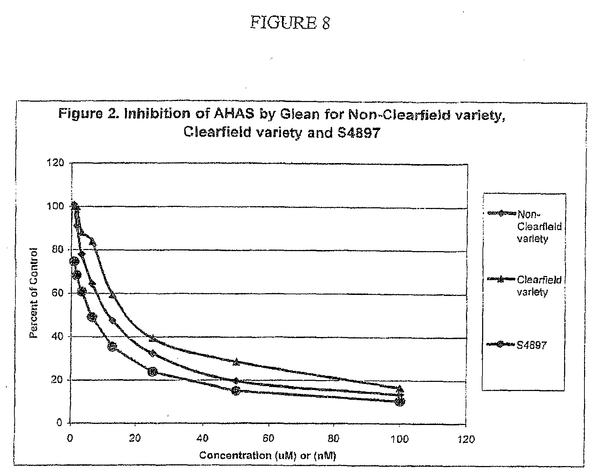

AHAS activity results also showed less inhibition at the higher concentration of imazamox as compared to a Clearfield® sunflower and conventional non Clearfield variety (Figure 7). Inhibition by Glean™ was similar across the three sunflower varieties (Figure 8). Feedback was not presented since the parental background of line S4897 was not available. Since both mutations are in the same *AHASL1* locus and all tested varieties were homozygous, there is a qualitative difference in the tolerance conferred by the amino acid substitution in the AHASL1 protein of S4897. Thus, the same amount of AHAS enzyme with this new substitution (i.e., Ala₁₀₇-to-Thr) is capable of catalyzing the formation of more product in the presence of herbicide than does AHAS with the Ala₁₉₀-to-Val substitution. This indicates that AHAS the Ala₁₀₇-to-Thr substitution has superior tolerance to imidazolinone, herbicides than AHAS with the Ala₁₉₀-to-Val substitution.

### EXAMPLE 6: Herbicide-Resistant Sunflower AHASL Proteins

Disclosed herein are both the nucleotide and amino acid sequences for wild-type and herbicide-resistant sunflower AHASL polypeptides. Plants comprising herbicide-resistant AHASL polypeptides have been previously identified, and a number of conserved regions of AHASL polypeptides that are the sites of amino acid substitutions that confer herbicide resistance have been described. See, Devine and Eberlein (1997) "Physiological, biochemical and molecular aspects of herbicide resistance based on altered target sites". In: Herbicide Activity: Toxicology, Biochemistry and Molecular Biology, Roe et al. (eds.), pp. 159-185, IOS Press, Amsterdam; and Devine and Shukla, (2000) Crop Protection 19:881-889.

Using the AHASL sequences of the invention as defined in the claims and methods known to those of ordinary skill in art, one can produce additional polynucleotides encoding herbicide-resistant AHASL polypeptides having one, two, three, or more amino acid substitutions at the identified sites in these conserved regions. Table 4 provides the conserved regions of AHASL proteins, the amino acid substitutions known to confer herbicide resistance within these conserved regions, and the corresponding amino acids in the sunflower AHASL1 protein set forth in SEQ ID NO: 4.

**Table 4. Mutations in conserved regions of AHASL1 polypeptides known to confer herbicide-resistance and their equivalent position in sunflower AHASL1**

| Conserved region¹ | Mutation² | Reference | Amino acid position in sunflower |
|---|---|---|---|
| VFAYPGG**A**SMEIHQALTRS³ | Ala₁₂₂ to Thr | Bernasconi *et al*.⁶ | Ala₁₀₇ |
| | | Wright & Penner¹⁴ | |
| AITGQV**P**RRMIGT⁴ | Pro₁₉₇ to Ala | Boutsalis *et al*.⁷ | Pro₁₈₂ |
| | Pro₁₉₇ to Thr | Guttieri *et al*.⁸ | |
| | Pro₁₉₇ to His | Guttieri *et al*.⁹ | |
| | Pro₁₉₇ to Leu | Guttieri *et al*.⁸ | |
| | | Kolkman *et al*.¹⁵ | |
| | Pro₁₉₇ to Arg | Guttieri *et al*.⁸ | |
| | Pro₁₉₇ to Ile | Boutsalis *et al*.⁷ | |
| | Pro₁₉₇ to Gln | Guttieri *et al*.⁸ | |
| | Pro₁₉₇ to Ser | Guttieri *et al*.⁸ | |
| **A**FQETP⁴ | Ala₂₀₅ to Asp | Hartnett *et al*_{.}¹⁰ | Ala₁₉₀ |
| | | Simpson¹¹ | |
| | Ala₂₀₅ to Val | Kolkman *et al*.¹⁵ | |
| | | White *et al*.¹⁶ | |
| Q**W**ED⁴ | Trp₅₇₄ to Leu | Boutsalis *et al*.⁷ | Trp₅₅₉ |
| IP**S**GG⁵ | Ser₆₅₃ to Asn | Devine & Eberlein¹³ | Ala₆₃₈ |
| | Ser₆₅₃ to Thr | Chang & Duggleby¹⁷ | |
| | Ser₆₅₃ to Phe | | |

| | | | |
|---|---|---|---|
| ¹Conserved regions from Devine and Eberlein (1997) "physiological biochemical and molecular aspects of herbicide resistance based on altered target sites". In: Herbicide Activity: Toxicology, Biochemistry and Molecular Biology, Roe et al. (eds.), pp. 159-185, IOS Press, Amsterdam and Devine and Shukla, (2000) Crop Protection 19:881-889. ²Amino acid numbering corresponds to the amino acid sequence of the *Arabidopsis thaliana* AHASL1 polypeptide. ³The sunflower AHASL1 amino acid sequences set forth in SEQ ID NOS:2 and 4 are not full length and begin with the amino acid sequences FAYPGG**T**SMEIHQALTRS and FAYPGG**A**SMEIHQALTRS, respectively. ⁴The sunflower AHASL amino acid sequences set forth in SEQ ID NOS: 2 and 4 possess this conserved region. ⁵The region of the sunflower AHASL1 (GenBank Accession No. AY541451) corresponding to this conserved region has the sequence IP**A**GG. ⁶Bernasconi et al. (1995) J. Biol. Chem. 270(29):17381-17385. ⁷Boutsalis et al. (1999) Pestic. Sci. 55:507-516. ⁸Guttieri et al. (1995) Weed Sci. 43:143-178. ⁹Guttieri et al. (1992) Weed Sci. 40:670-678. ¹⁰Hartnett et al. (1990) "Herbicide-resistant plants carrying mutated acetolactate synthase genes," In: Managing Resistance to Agrochemicals: Fundamental Research to Practical Strategies, Green et al. (eds.), American Chemical Soc. Symp., Series No. 421, Washington, DC, USA ¹¹Simpson (1998) Down to Earth 53(1):26-35. ¹²Bruniard (2001) Inheritance of imidazolinone resistance, characterization of cross-resistance pattern, and identification of molecular markers in sunflower (Helianthus annuus L.). Ph.D. Thesis, North Dakota State University, Fargo, ND, USA, pp 1-78. ¹³Devine and Eberlein (1997) "Physiological, biochemical and molecular aspects of herbicide resistance based on altered target sites". In: Herbicide Activity: Toxicology, Biochemistry and Molecular Biology, Roe et al. (eds.), pp. 159-185, IOS Press, Amsterdam ¹⁴Wright and Penner (1998) Theor. Appl. Genet. 96:612-620. ¹⁵Kolkman et al. (2004) Theor. Appl. Genet. 109: 1147-1159. ¹⁶White et al. (2003) Weed Sci. 51:845-853. ¹⁷Chang and Duggleby (1998) Biochem J. 333:765-777. | | | |

### EXAMPLE 7: Production of a GM1606 Sunflower Line

A second herbicide-resistant Sunflower line was produced by the mutagenesis of sunflower seeds that are wild-type with respect to herbicide resistant by a method essentially as described above in Example 1. The new line and plants of that line are referred to herein as GM1606. The GM1606 Sunflower line comprises the same mutation in the AHASL1 gene as in the S4897 sunflower line. This mutation in GM1606 is a G-to-A transition that corresponds to nucleotide 21 of SEQ ID NO: 1. Such a mutation gives rise an alanine-to-threonine substitution at amino acid position 7 (SEQ ID NO: 2). This amino acid position in SEQ ID NO: 2 corresponds to amino acid position 107 in the full-length amino acid sequence encoded by the sunflower AHASL1 nucleotide sequence of GenBank Accession No. AY541451 (SEQ ID NO:11) and amino acid position 122 in the full-length amino acid sequence encoded by the *Arabidopsis thaliana* AHASL nucleotide sequence of GenBank Accession No. X51514.

### EXAMPLE 8: Response of Mutant Events A122T and A205V to Imazapir

A greenhouse study was conducted to quantify and contrast the imazapir sensivity of the mutants A122T and A205V in different genetic backgrounds at the whole plant level in sunflower. Seeds of the different Sunflower lines that were used in this study were obtained under field conditions. The lines used in the study are listed in Table 5.

**Table 5. Sunflower Materials**

| **Hybrid/Line Code** | **Type of Material** | **Mutation Event** |
|---|---|---|
| TH1 | Hybrid | A205V |
| TH9 | Hybrid | A205V |
| TH10 | Restorer Line | A205V |
| GIM 5-7 | Manteiner Line | A205V |
| IB920 | Manteiner Line | A205V |
| IR79 | Restorer Line | A205V |
| TH6 | Hybrid | A122T |
| TH11 | Hybrid | A122T |
| TH12 | Restorer Line | (A122T |
| GM40 | Manteiner Line | A122T |
| GM1606 | ManteinerLine | A122T |
| GIM 5-6 | Restorer Line | A122T |
| TH13 | Manteiner Line | Wild Type |

### Methods

Seeds were sown in Petri dishes and, after germination, plantlets were transplanted to pots of 10 cm of diameter in a potting media consisting of equal parts of vermiculite, soil and sand. Plants were grown in a greenhouse under natural light conditions supplemented with 400 W sodium halide lamps to provide a 16 hrs daylength. Day/night temperatures were 25 and 20 °C, respectively. At the V2 stage 10 plants of each genotype were randomly assigned to each treatment consisting of seven imazapir doses (0, 40, 80, 160, 240, 320, 400 and 480 g ai/ha), and a zero-time biomass determination. Experiment was arranged as a randomized block design with a full factorial (sunflower line x treatment) arrangement of treatments and 10 replications.

On the day of herbicide application ten plants of each genotype were cut at the cotyledonal node and dried at 60°C for 48 hrs zero-time for dried weight determination. The rest of the plants were maintained for 10 days after imazapir treatment (DAT) and their height and root and above ground dry biomass were recorded. Height was determined as the distance between the cotyledonal node and the apex of each plant. For root biomass determination, each plant was taken from the pot and the potting media was washed out from the roots. Above ground biomass data from each line were converted to biomass accumulation after application by subtracting the appropriate average zero-time biomass from each sample. Dry biomass data were converted to percentages of the untreated control plants within each line to allow direct comparisons between groups.

### Results

### 1. Height

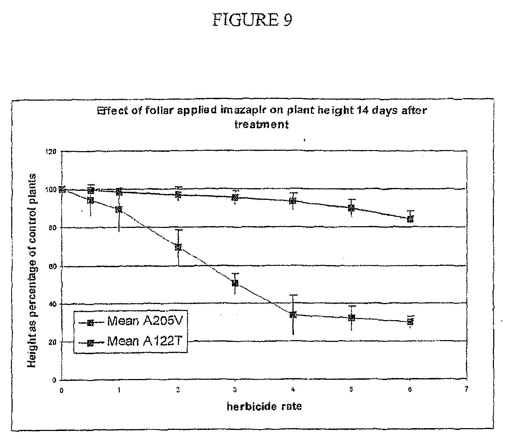

Height of the sunflower lines carrying the A205V mutation did not differ from the untreated controls when a rate of 0.5X or 1X of imazapir was applied. From 2X to 6X, these lines showed a significant reduction in height which reached 68.9% +/- 3.1 of the untreated controls (Table 6 and Figure 9). In contrast, sunflower lines carrying the A122T mutation showed a lesser height reduction (from 0.6 to 15.8% of the untreated controls for 0.5X and 6X rate of imazapir, respectively). Both groups of lines showed a significative difference between them for their response to an increase in herbicide rate from 2X to 6X (Table 6 and Figure 9).

### 2. Phytotoxicity Index

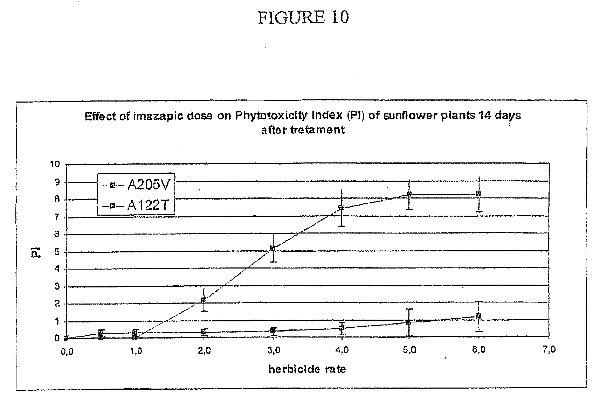

Both mutants showed great differences in their response to the increase in herbicide rate from 0.5X to 6X (Figure 10). Sunflower lines carrying the A122T mutation showed a slightly reduction in leaf size and lighter green color than the control plants as the herbicide rate increase (Table 7). In contrast, plants carrying the A205V mutation did not show any injury at 0.5X or 1X of herbicide rate, but the level of injury (yellowish, leaf deformation and leaf necrosis) increased quickly from 2X to 6X (Table 7). Both mutants differed between them significantly for the phytotoxicity index from 2X to 6X (Table 7).

### 3. Above ground Dry Weight Biomass

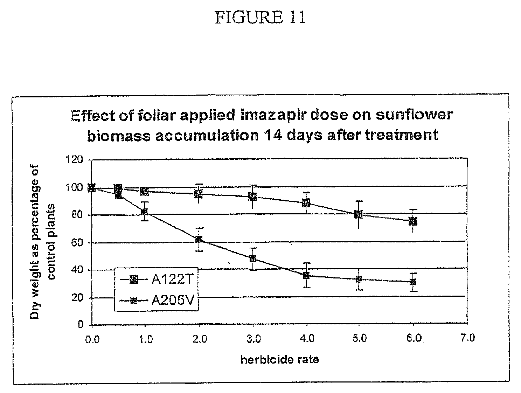

Dose response curves for dry weight of mutants A122T and A205V are shown in Figure 11. Biomass weight of event A122T was reduced with respect to control plants at 4X, 5X and 6X rates, and this reduction reached 25% for the higher dose. Meanwhile, dry weight of event A205V was reduced with respect to the control plants from 0.5X (40 gai./ha) to 6X. Both mutants showed significant differences between them with respect to this variable from 0.5X to 6X (Table 8). The same trends were obtained for dry matter accumulation (not shown) but without the confounding effects of the initial differences among genotypes for their zero-time dry weight.

### 4. Root Biomass

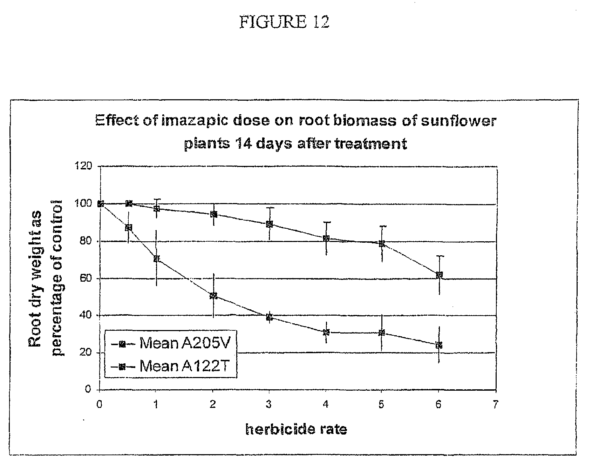

As the doses of imazapir increased, root dry biomass of both mutants were reduced with respect to control plants, but the rate of reduction was very different between A122T and A205V (Figure 12). In fact, A205V showed a significant reduction in dry weight root biomass from 12.8% at 0.5X (40 g.a.i/ha) to 75.6% at 6X (Table 9). In contrast, A122T carriers showed a significative decrease in root weight biomass from 3X to 6X, and at the higher dose the reduction reached 38.3% (Table 9). Both mutants showed significant differences between them in their root dry weight response to herbicides rates from 0.5X to 6X (Table 9 and Figure 12).

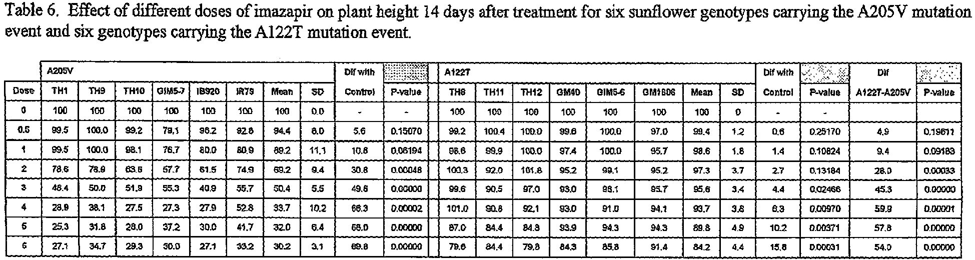

**Table 7- Effect of different doses of imazapir on Phytotoxicity Index 14 days after treatment for three sunflower genotypes carrying the A205V mutation event and three genotypes carrying the A122T mutation event.**

| | A205V | | | | | | | A122T | | | | | | Dif A205V-A122T | |
|---|---|---|---|---|---|---|---|---|---|---|---|---|---|---|---|
| Dose | TH1 | TH9 | TH10 | Mean | SD | P-value | | TH6 | TH11 | TH12 | Mean | SD | P-value | | P-value |
| 0 | 0 | 0 | 0 | 0 | 0 | | | 0 | 0 | 0 | 0 | 0 | | | |
| 0.5 | 0 | 0 | 0 | 0 | 0 | ns | | 0.5 | 0.4 | 0.0 | 0.3 | 0.3 | 0.19171 | -0.29 | 0.19171 |
| 1 | 0 | 0 | 0 | 0 | 0 | ns | | 0.5 | 0.4 | 0.0 | 0.3 | 0.3 | 0.19461 | -0.29 | 0.19461 |
| 2 | 1.8 | 1.6 | 3.1 | 2.2 | 0.8 | 0.04648 | | 0.5 | 0.4 | 0.0 | 0.3 | 0.3 | 0.18981 | 1.87 | 0.0530 |
| a | 6.4 | 5.1 | 3.9 | 5.1 | 1.2 | 0.01793 | | 0.5 | 0.5 | 0.0 | 0.3 | 0.3 | 0.18350 | 4.81 | 0.02622 |
| 4 | 8.0 | 8.4 | 5.9 | 7.4 | 1.3 | 0.01084 | | 0.5 | 1.0 | 0.0 | 0.5 | 0.5 | 0.22540 | 6.92 | 0.00657 |
| 5 | 8.9 | 8.9 | 6.9 | 8.2 | 1.1 | 0.00601 | | 0.5 | 2.0 | 0.0 | 0.8 | 1.0 | 0.29986 | 7.38 | 0.00111 |
| 6 | 9.0 | 8.9 | 6.7 | 8.2 | 1.3 | 0.00799 | | 0.5 | 2.5 | 0.5 | 1.2 | 1.2 | 0.22222 | 7.03 | 0.00219 |

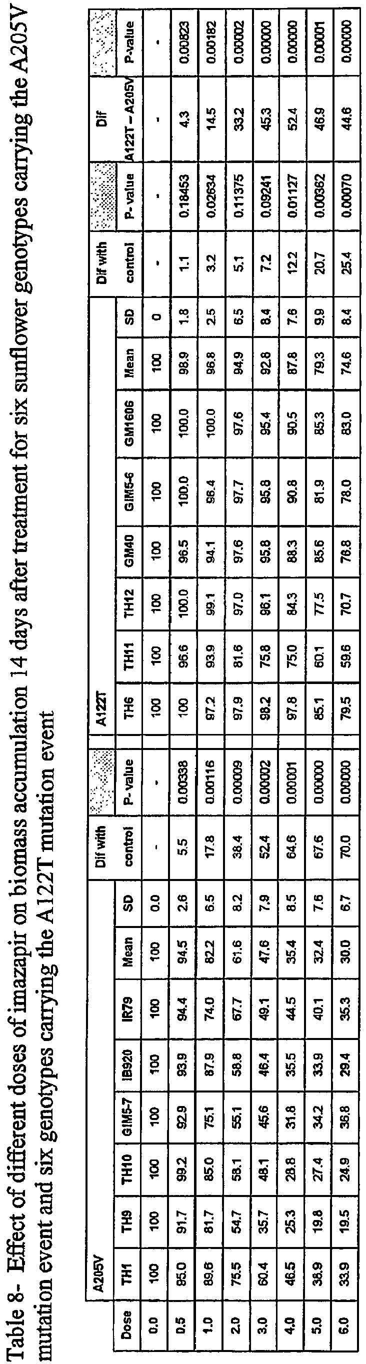

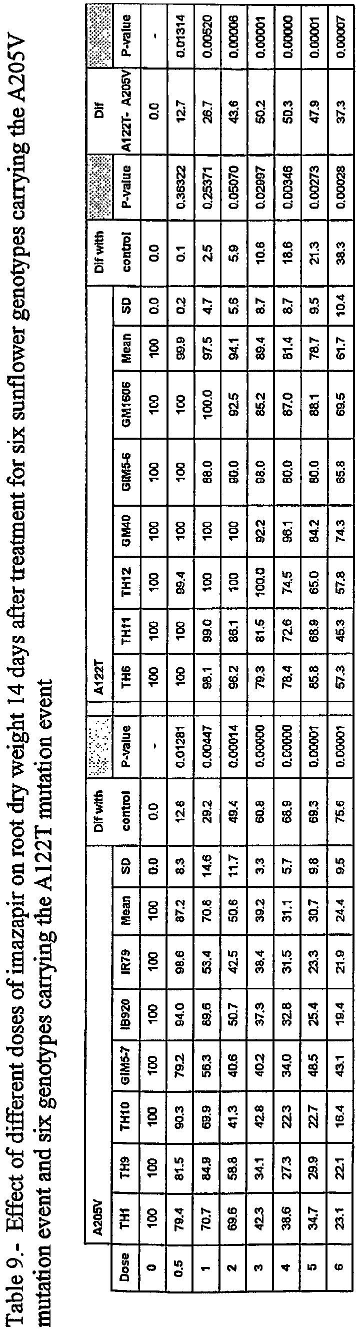

## Claims

1. A sunflower acetohydroxyacid synthase large subunit (*AHASL*) polynucleotide, wherein said *AHASL* polynucleotide encodes an imidazolinone herbicide-tolerant AHASL1 protein comprising a substitution from alanine to threonine at amino acid position 7 of SEQ ID NO:2 or, equivalently, at position 107 of SEQ ID NO:12; wherein said AHASL1 protein comprises a polypeptide that is at least 95% identical to SEQ ID NO:12; and wherein said AHASL1 protein confers upon a plant increased tolerance to an imidazolinone herbicide as compared to a wild-type variety of said plant.

2. The *AHASL* polynucleotide of claim 1, wherein said AHASL1 protein comprises:
a) the amino acid sequence set forth in SEQ ID NO:2 with a substitution from alanine to threonine at amino acid position 7 of SEQ ID NO:2 or, equivalently, at position 107 of SEQ ID NO:12, which optionally contains at least one member selected from the group consisting of:
(i) a substitution from proline to glutamine or serine at amino acid position 182 or an equivalent position relative to the amino acid sequence set forth in SEQ ID NO:12;
(ii) a substitution from alanine to aspartate or valine at amino acid position 190 or an equivalent position relative to the amino acid sequence set forth in SEQ ID NO:12;
(iii) a substitution from tryptophan to leucine at amino acid position 559 or an equivalent position relative to the amino acid sequence set forth in SEQ ID NO:12; and
(iv) a substitution from alanine to any one of asparagine, threonine, phenylalanine, or valine at amino acid position 638 or an equivalent position relative to the amino acid sequence set forth in SEQ ID NO: 12.

3. An expression cassette comprising a promoter operably linked to the polynucleotide of claim 1 or 2.

4. A method for producing a plant that is tolerant to an imidazolinone herbicide, the method comprising
(i) transforming a plant cell with a polynucleotide construct comprising the polynucleotide of claim 1 or 2 operably linked to a promoter that drives expression in a plant cell; and
(ii) regenerating the plant from said transformed plant cell.

5. A plant comprising at least one copy of a polynucleotide as defined in claims 1 or 2

6. A method for selecting for a transformed plant cell, the method comprising:
(i) providing a plant cell comprising a plant transformation vector comprising a promoter and an operably linked polynucleotide according to claim 1 or 2;
(ii) exposing said transformed plant cell to an imidazolinone herbicide at a concentration that inhibits the growth of an untransformed plant cell; and
(iii) identifying said transformed plant cell by its ability to grow in the presence of said herbicide.

7. A method for growing a plant according to claim 5 while controlling weeds growing in the vicinity of said plant, said method comprising
(i) growing in a field a plant according to claim 5; and
(ii) contacting said plant and weeds in the field with an effective amount of an AHAS-inhibiting herbicide formulation comprising an imidazolinone herbicide, a sulfonylurea herbicide, a triazolopyrimidine herbicide, a pyrimidinyloxybenzoate herbicide, a sulfonylamino-carbonyltriazolinone herbicide, or mixture thereof to which the plant is tolerant and which would inhibit the growth of a wild-type variety of the plant, wherein the herbicide formulation does not inhibit the growth of said plant as severely as it would inhibit the growth of the wild-type variety of the plant.

8. The method according to any one of claims 4, 6, and 7, wherein said imidazolinone herbicide comprises one or more of imazethapyr, imazapic, imazamox, imazaquin, imazethabenz, and imazapyr, or a mixture of any of the foregoing.

9. The method of claim 7, wherein said sulfonylurea herbicide comprises chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfuron, flazasulfuron, imazosulfuron, pyrazosulfuron ethyl, halosulfuron, or a mixture of any of the foregoing.

10. An organ, tissue or cell of the plant of claim 5.

11. A seed comprising at least one copy of a polynucleotide as defined in claims 1 or 2..

12. The seed of claim 11, wherein said seed is treated with an effective amount of an AHAS-inhibiting herbicide.

13. The seed of claim 12, wherein said AHAS-inhibiting herbicide comprises an imidazolinone herbicide, a sulfonylurea herbicide, a triazolopyrimidine herbicide, a sulfonylamino-carbonyltriazolinone herbicide, or a mixture of any of the foregoing.

14. The seed of claim 13, wherein said AHAS-inhibiting herbicide is an imidazolinone herbicide.

15. A method for treating seeds, the method comprising treating the seed of any one of claims 11-14 with an effective amount of an AHAS-inhibiting herbicide.

16. The method of claim 15, wherein said AHAS-inhibiting herbicide comprises an imidazolinone herbicide, a sulfonylurea herbicide, a triazolopyrimidine herbicide, a sulfonylamino-carbonyltriazolinone herbicide, or a mixture of any of the foregoing.

17. The method of claim 16, wherein said AHAS-inhibiting herbicide is an imidazolinone herbicide.

18. A method for detecting a sunflower *AHASL1*, said method comprising:
(i) obtaining genomic DNA from a plant;
(ii) using said DNA as a template for a polymerase chain reaction (PCR) amplification comprising a forward *AHASL1* primer comprising the sequence set forth as SEQ ID NO:7 and a reverse *AHASL1* primer comprising the sequence set forth as SEQ ID NO:8; and
(iii) sequencing the products of said PCR amplification.

## Patentansprüche

1. Großes Acetohydroxysäuresynthase-Untereinheit (*AHASL*)-Polynukleotid aus der Sonnenblume, wobei dieses *AHASL-*Polynukleotid ein Imidazolinonherbizid-tolerantes AHASL1-Protein kodiert, umfassend eine Substitution von Alanin zu Threonin an der Aminosäureposition 7 der SEQ ID NO: 2 oder entsprechend an Position 107 der SEQ ID NO: 12, wobei dieses AHASL1-Protein ein Polypeptid umfasst, das wenigstens 95% identisch mit SEQ ID NO: 12 ist und wobei dieses AHASL1-Protein einer Pflanze erhöhte Toleranz gegenüber einem Imidazolinonherbizid im Vergleich zu einer Wildtyp-Sorte dieser Pflanze verleiht.

2. *AHASL*-Polynukleotid nach Anspruch 1, wobei dieses AHASL1-Protein umfasst:
a) die Aminosäuresequenz gemäß SEQ ID NO: 2 mit einer Substitution von Alanin zu Threonin an der Aminosäureposition 7 der SEQ ID NO: 2 oder entsprechend an der Position 107 der SEQ ID NO: 12, die gegebenenfalls zumindest einen Vertreter enthält, ausgewählt aus der Gruppe, bestehend aus:
(i) eine Substitution von Prolin zu Glutamin oder Serin an der Aminosäureposition 182 oder einer entsprechenden Position in Bezug auf die Aminosäuresequenz gemäß SEQ ID NO: 12;
(ii) eine Substitution von Alanin zu Aspartat oder Valin an der Aminosäureposition 190 oder einer entsprechenden Position in Bezug auf die Aminosäuresequenz gemäß SEQ ID NO: 12;
(iii) eine Substitution von Tryptophan zu Leucin an der Aminosäureposition 559 oder einer entsprechenden Position in Bezug auf die Aminosäuresequenz gemäß SEQ ID NO: 12; und
(iv) eine Substitution von Alanin zu einem von Asparagin, Threonin, Phenylalanin oder Valin an der Aminosäureposition 638 oder einer entsprechenden Position in Bezug auf die Aminosäuresequenz gemäß SEQ ID NO: 12.

3. Expressionskassette, umfassend einen Promotor, der operabel mit dem Polynukleotid nach Anspruch 1 oder 2 verknüpft ist.

4. Verfahren zur Herstellung einer Pflanze, die gegenüber einem Imidazolinonherbizid tolerant ist, wobei das Verfahren umfasst:
(i) Transformieren einer Pflanzenzelle mit einem Polynukleotidkonstrukt, umfassend das Polynukleotid nach Anspruch 1 oder 2, das operabel mit einem Promotor verknüpft ist, der die Expression in einer Pflanzenzelle vorantreibt; und
(ii) Regenerieren der Pflanze aus dieser transformierten Pflanzenzelle.

5. Pflanze, umfassend zumindest eine Kopie eines Polynukleotids nach Anspruch 1 oder 2.

6. Verfahren zum Selektieren einer transformierten Pflanzenzelle, wobei das Verfahren umfasst:
(i) Bereitstellen einer Pflanzenzelle, umfassend einen Pflanzentransformationsvektor, umfassend einen Promotor und ein operabel verknüpftes Polynukleotid nach Anspruch 1 oder 2;
(ii) Aussetzen dieser transformierten Pflanzenzelle gegenüber einem Imidazolinonherbizid bei einer Konzentration, die das Wachstum der nicht transformierten Pflanzenzelle hemmt; und
(iii) Identifizieren dieser transformierten Pflanzenzelle durch deren Fähigkeit, in der Gegenwart dieses Herbizids zu wachsen.

7. Verfahren zum Wachsen einer Pflanze nach Anspruch 5, während Unkrautwachstum in der Nähe dieser Pflanze kontrolliert wird, wobei dieses Verfahren umfasst:
(i) Wachsen einer Pflanze nach Anspruch 5 in einem Feld; und
(ii) Inkontaktbringen dieser Pflanze und der Unkräuter in diesem Feld mit einer wirksamen Menge einer AHAS-hemmenden Herbizidformulierung, umfassend ein Imidazolinonherbizid, ein Sulfonylharnstoffherbizid, ein Triazolopyrimidinherbizid, ein Pyrimidinyloxybenzoatherbizid, ein Sulfonylaminocarbonyltriazolinonherbizid oder eine Mischung hiervon, gegenüber der die Pflanze tolerant ist und die das Wachstum einer Wildtyp-Sorte der Pflanze hemmen würde, wobei die Herbizidformulierung das Wachstum dieser Pflanze nicht so stark hemmt wie sie das Wachstum der Wildtyp-Sorte der Pflanze hemmen würde.

8. Verfahren nach einem der Ansprüche 4, 6 und 7, wobei dieses Imidazolinonherbizid einen oder mehrere Vertreter von Imazethapyr, Imazapic, Imazamox, Imazaquin, Imazethabenz und Imazapyr oder eine Mischung hiervon umfasst.

9. Verfahren nach Anspruch 7, wobei dieses Sulfonylharnstoffherbizid Chlorsulfuron, Metsufuronmethyl, Sulfometuron-methyl, Chlorimuron-ethyl, Thifensulfuron-methyl, Tribenuron-methyl, Bensulfuronmethyl, Nicosulfuron, Ethametsulfuron-methyl, Rimsulfuron, Triflusulfuron-methyl, Triasulfuron, Primisulfuron-methyl, Cinosulfuron, Amidosulfuron, Flazasulfuron, Imazosulfuron, Pyrazosulforon-ethyl, Halosulfuron oder eine Mischung hiervon umfasst.

10. Organ, Gewebe oder Zelle der Pflanze nach Anspruch 5.

11. Same, umfassend wenigstens eine Kopie eines Polynukleotids nach Anspruch 1 oder 2.

12. Same nach Anspruch 11, wobei dieser Same mit einer wirksamen Menge eines AHAS-hemmenden Herbizids behandelt wird.

13. Same nach Anspruch 12, wobei dieses AHAS-hemmende Herbizid ein Imidazolinonherbizid, ein Sulfonylharnstoffherbizid, ein Triazolopyrimidinherbizid, ein Sulfonylaminocarbonyltriazolinonherbizid oder eine Mischung hiervon umfasst.

14. Same nach Anspruch 13, wobei dieses AHAS-hemmende Herbizid ein Imidazolinonherbizid ist.

15. Verfahren zur Behandlung von Samen, wobei das Verfahren Behandeln des Samens nach einem der Ansprüche 11-14 mit einer wirksamen Menge eines AHAS-hemmenden Herbizids umfasst.

16. Verfahren nach Anspruch 15, wobei dieses AHAS-hemmende Herbizid ein Imidazolinonherbizid, ein Sulfonylharnstoffherbizid, ein Triazolopyrimidinherbizid, ein Sulfonylaminocarbonyltriazolinonherbizid oder eine Mischung hiervon umfasst.

17. Verfahren nach Anspruch 16, wobei dieses AHAS-hemmende Herbizid ein Imidazolinonherbizid ist.

18. Verfahren zum Nachweisen eines *AHASL1* aus der Sonnenblume, wobei das Verfahren umfasst:
(i) Erhalten genomischer DNA aus einer Pflanze;
(ii) Verwenden dieser DNA als ein Templat für eine Polymerasekettenreaktion (PCR)-Amplifikation, umfassend einen Forward-*AHASL1*-Primer, umfassend die Sequenz gemäß SEQ ID NO: 12, und einen Reverse-*AHASL1*-Primer, umfassend die Sequenz gemäß SEQ ID NO: 8, und
(iii) Sequenzieren der Produkte dieser PCR-Amplifikation.

## Revendications

1. Polynucléotide de grande sous-unité d'acétohydroxyacide synthase (*AHASL*) de tournesol, **caractérisé en ce que** ledit polynucléotide *AHASL* code pour une pt AHASL1 tolérante à l'herbicide imidazolinone comprenant une substitution d'alanine en thréonine à la position d'acide aminé 7 de SEQ ID NO: 2 ou, de façon équivalente, à la position 107 de SEQ ID NO: 12 ; où ladite protéine AHASL1 comprend un polypeptide qui est au moins 95 % identique à SEQ ID NO: 12 ; et où ladite protéine AHASL1 confère à une plante une tolérance accrue à un herbicide imidazolinone par rapport à une variété de type sauvage de ladite plante.

2. Polynucléotide *AHASL* de la revendication 1, **caractérisé en ce que** ladite protéine AHASL1 comprend :
a) la séquence d'acides aminés décrite dans SEQ ID NO: 2 avec une substitution d'alanine en thréonine à la position d'acide aminé 7 de SEQ ID NO: 2 ou, de façon équivalente, à la position 107 de SEQ ID NO: 12, qui contient facultativement au moins un membre choisi dans le groupe constitué de :
(i) une substitution de proline en glutamine ou sérine à la position d'acide aminé 182 ou une position équivalente par rapport à la séquence d'acides aminés décrite dans SEQ ID NO: 12 ;
(ii) une substitution d'alanine en aspartate ou valine à la position d'acide aminé 190 ou une position équivalent par rapport à la séquence d'acides aminés décrite dans SEQ ID NO: 12 ;
(iii) une substitution de tryptophane en leucine à la position d'acide aminé 559 ou une position équivalente par rapport à la séquence d'acides aminés décrite dans SEQ ID NO: 12 ; et
(iv) une substitution d'alanine en l'un quelconque d'asparagine, thréonine, phénylalanine ou valine à la position d'acide aminé 638 ou une position équivalente par rapport à la séquence d'acides aminés décrite dans SEQ ID NO: 12.

3. Cassette d'expression comprenant un promoteur fonctionnellement lié au polynucléotide de la revendication 1 ou 2.

4. Procédé de production d'une plante qui est tolérante à un herbicide imidazolinone, le procédé comprenant
(i) la transformation d'une cellule de plante avec une construction de polynucléotide comprenant le polynucléotide de la revendication 1 ou 2 fonctionnellement lié à un promoteur qui contrôle l'expression dans une cellule de plante ; et
(ii) la régénération de la plante à partir de ladite cellule de plante transformée.

5. Plante comprenant au moins une copie d'un polynucléotide tel que défini dans les revendications 1 ou 2.

6. Procédé de sélection d'une cellule de plante transformée, le procédé comprenant :
(i) la fourniture d'une cellule de plante comprenant un vecteur de transformation de plante comprenant un promoteur et un polynucléotide fonctionnellement lié selon la revendication 1 ou 2 ;
(ii) l'exposition de ladite cellule de plante transformée à un herbicide imidazolinone à une concentration qui inhibe la croissance d'une cellule de plante non transformée ; et
(iii) l'identification de ladite cellule de plante transformée par sa capacité à croître en présence dudit herbicide.

7. Procédé de culture d'une plante selon la revendication 5 en contrôlant les mauvaises herbes dans le voisinage de ladite plante, ledit procédé comprenant
(i) la culture dans un champ d'une plante selon la revendication 5 ; et
(ii) la mise en contact de ladite plante et des mauvaises herbes dans le champ avec une quantité efficace d'une formulation d'herbicide inhibiteur d'AHAS comprenant un herbicide imidazolinone, un herbicide sulfonylurée, un herbicide triazolopyrimidine, un herbicide pyrimidinyloxybenzoate, un herbicide sulfonylamino-carbonyltriazolinone, ou un mélange de ceux-ci à laquelle la plante est tolérante et qui inhiberait la croissance d'une variété de type sauvage de la plante, dans lequel la formulation d'herbicide n'inhibe pas la croissance de ladite plante aussi fortement qu'elle inhiberait la croissance de la variété de type sauvage de la plante.

8. Procédé selon l'une quelconque des revendications 4, 6, et 7, dans lequel ledit herbicide imidazolinone comprend l'un ou plusieurs des imazéthapyr, imazapic, imazamox, imazaquine, imazéthabenz, et imazapyr, ou un mélange de l'un quelconque de ceux-ci.

9. Procédé de la revendication 7, dans lequel ledit herbicide sulfonylurée comprend les chlorsulfuron, metsulfuron méthyl, sulfométuron méthyl, chlorimuron éthyl, thifensulfuron méthyl, tribénuron méthyl, bensulfuron méthyl, nicosulfuron, éthametsulfuron méthyl, rimsulfuron, triflusulfuron méthyl, triasulfuron, primisulfuron méthyl, cinosulfuron, amidosulfuron, flazasulfuron, imazosulfuron, pyrazosulfuron éthyl, halosulfuron, ou un mélange de l'un quelconque de ceux-ci.

10. Organe, tissu ou cellule de la plante de la revendication 5.

11. Semence comprenant au moins une copie d'un polynucléotide tel que défini dans les revendications 1 ou 2.

12. Semence de la revendication 11, **caractérisée en ce que** ladite semence est traitée avec une quantité efficace d'un herbicide inhibiteur d'AHAS.

13. Semence de la revendication 12, **caractérisée en ce que** ledit herbicide inhibiteur d'AHAS comprend un herbicide imidazolinone, un herbicide sulfonylurée, un herbicide triazolopyrimidine, un herbicide sulfonylamino-carbonyltriazolinone, ou un mélange de l'un quelconque de ceux-ci.

14. Semence de la revendication 13, **caractérisée en ce que** ledit herbicide inhibiteur d'AHAS est un herbicide imidazolinone.

15. Procédé de traitement de semences, le procédé comprenant le traitement de la semence de l'une quelconque des revendications 11 à 14 avec une quantité efficace d'un herbicide inhibiteur d'AHAS.

16. Procédé de la revendication 15, dans lequel ledit herbicide inhibiteur d'AHAS comprend un herbicide imidazolinone, un herbicide sulfonylurée, un herbicide triazolopyrimidine, un herbicide sulfonylamino-carbonyltriazolinone, ou un mélange de l'un quelconque de ceux-ci.

17. Procédé de la revendication 16, dans lequel ledit herbicide inhibiteur d'AHAS est un herbicide imidazolinone.

18. Procédé de détection d'un *AHASL1* de tournesol, ledit procédé comprenant :
(i) l'obtention dudit ADN génomique à partir d'une plante ;
(ii) l'utilisation dudit ADN en tant que matrice pour une amplification par réaction de polymérase en chaîne (PCR) comprenant une amorce *AHASL1* sens comprenant la séquence décrite dans SEQ ID NO: 7 et une amorce *AHASL1* antisens comprenant la séquence décrite dans SEQ ID NO: 8 ; et
(iii) le séquençage des produits de ladite amplification par PCR.


### SEQUENCE LISTING

<110> BASF SE Nidera S.A.
<120> HERBICIDE-RESISTANT SUNFLOWER PLANTS,
   POLYNUCLEOTIDES ENCODING HERBICIDE-RESISTANT
   ACETOHYDROXYACID SYNTHASE LARGE SUBUNIT PROTEINS, AND
   METHODS OF USE
<130> 038867/310344
<150> US 60/695,952
   <151> 2005-07-01
<160> 14
<170> FastSEQ for Windows Version 4.0
<210> 1
   <211> 1178
   <212> DNA
   <213> Helianthus annus
<220>
   <221> CDS
   <222> (3)...(1178)
<400> 1
   tc ttc gcc tac ccc ggc ggc acg tca atg gag atc cac caa gct ctc     47
      Phe Ala Tyr Pro Gly Gly Thr Ser Met Glu Ile His Gln Ala Leu
       1               5                  10                  15
   acg cgc tca agc act atc cgc aat gtg ctc ccc cgt cac gaa cag ggc    95
   Thr Arg Ser Ser Thr Ile Arg Asn Val Leu Pro Arg His Glu Gln Gly
                   20                  25                  30
   ggc gtg ttc gcc gcc gaa ggc tac gcg cgc gcc tcc ggt ctt ccc ggc   143
   Gly Val Phe Ala Ala Glu Gly Tyr Ala Arg Ala Ser Gly Leu Pro Gly
               35                  40                  45
   gtg tgt atc gcc act tcc ggt ccc gga gct acg aac cta gtt agt ggt   191
   Val Cys Ile Ala Thr Ser Gly Pro Gly Ala Thr Asn Leu Val Ser Gly
           50                  55                  60
   ctt gct gac gcg ctg tta gac agt gtc ccc atg gtg gca atc acc ggt   239
   Leu Ala Asp Ala Leu Leu Asp Ser Val Pro Met Val Ala Ile Thr Gly
       65                  70                  75
   caa gtt ccc cgg aga atg atc gga acc gat gcg ttt caa gaa acc cca   287
   Gln Val Pro Arg Arg Met Ile Gly Thr Asp Ala Phe Gln Glu Thr Pro
   80                  85                  90                  95
   att gtt gag gta aca cgt tcg atc act aaa cat aat tat ctt gtg ttg   335
   Ile Val Glu Val Thr Arg Ser Ile Thr Lys His Asn Tyr Leu Val Leu
                   100                 105                 110
   gat gtt gag gat att ccc aga att gtt cgt gag gct ttt tat ctt gcg   383
   Asp Val Glu Asp Ile Pro Arg Ile Val Arg Glu Ala Phe Tyr Leu Ala
               115                 120                 125
   agt tcg ggt cga ccc ggc ccg gtt ttg ata gat gta ccg aaa gat ata   431
   Ser Ser Gly Arg Pro Gly Pro Val Leu Ile Asp Val Pro Lys Asp Ile
           130                 135                 140
   cag caa cag tta gtg gtg ccg aaa tgg gat gaa ccg atg agg tta ccg    479
   Gln Gln Gln Leu Val Val Pro Lys Trp Asp Glu Pro Met Arg Leu Pro
       145                 150                 155
   ggt tat ttg tct aga atg ccg aag cct caa tat gat ggg cat ttg gaa    527
   Gly Tyr Leu Ser Arg Met Pro Lys Pro Gln Tyr Asp Gly His Leu Glu
   160                 165                 170                 175
   cag att gtt agg ttg gtg ggg gaa gcg aag agg ccg gtt ttg tat gtg    575
   Gln Ile Val Arg Leu Val Gly Glu Ala Lys Arg Pro Val Leu Tyr Val
                   180                 185                 190
   ggt ggt ggg tgt ttg aat tcg gat gat gag ttg agg cgg ttt gtg gag    623
   Gly Gly Gly Cys Leu Asn Ser Asp Asp Glu Leu Arg Arg Phe Val Glu
               195                 200                 205
   ctt acg ggg att ccg gtt gcg agt act ttg atg ggg ctc gga gcg tac    671
   Leu Thr Gly Ile Pro Val Ala Ser Thr Leu Met Gly Leu Gly Ala Tyr
           210                 215                 220
   cct gct tcg agt gat ttg tcg ctt cat atg ctt ggg atg cat ggt acg    719
   Pro Ala Ser Ser Asp Leu Ser Leu His Met Leu Gly Met His Gly Thr
       225                 230                 235
   gtt tat gcg aat tat gcg gtt gat aag agt gat ttg ttg ctt gcg ttt    767
   Val Tyr Ala Asn Tyr Ala Val Asp Lys Ser Asp Leu Leu Leu Ala Phe
   240                 245                 250                 255
   ggg gtg cgg ttt gat gat cgt gtg acg ggg aag ctt gag gcg ttt gct    815
   Gly Val Arg Phe Asp Asp Arg Val Thr Gly Lys Leu Glu Ala Phe Ala
                   260                 265                 270
   agt agg gcg aag att gtt cat att gat att gat cct gct gaa att ggg    863
   Ser Arg Ala Lys Ile Val His Ile Asp Ile Asp Pro Ala Glu Ile Gly
               275                 280                 285
   aag aat aag cag cct cat gtg tcg att tgt ggt gat att aag gtc gcg    911
   Lys Asn Lys Gln Pro His Val Ser Ile Cys Gly Asp Ile Lys Val Ala
           290                 295                 300
   tta cag ggt ttg aac aag att ttg gag gaa aag aat tcg gtg act aat    959
   Leu Gln Gly Leu Asn Lys Ile Leu Glu Glu Lys Asn Ser Val Thr Asn
       305                 310                 315
   ctt gat ttt tcg acc tgg aga aag gaa ttg gat gaa caa aaa atg aag    1007
   Leu Asp Phe Ser Thr Trp Arg Lys Glu Leu Asp Glu Gln Lys Met Lys
   320                 325                 330                 335
   ttc ccg ttg agc ttt aaa acg ttt ggc gaa gcg att cct cca cag tat    1055
   Phe Pro Leu Ser Phe Lys Thr Phe Gly Glu Ala Ile Pro Pro Gln Tyr
                   340                 345                 350
   gct att caa gtt ctt gat gag tta acg ggc ggg aat gca att att agc    1103
   Ala Ile Gln Val Leu Asp Glu Leu Thr Gly Gly Asn Ala Ile Ile Ser
               355                 360                 365
   acc ggt gtc ggg caa cat cag atg tgg gct gct cag ttt tac aaa tac    1151
   Thr Gly Val Gly Gln His Gln Met Trp Ala Ala Gln Phe Tyr Lys Tyr
           370                 375                 380
   aac aaa cct aga caa tgg ctg acg tcg                                1178
   Asn Lys Pro Arg Gln Trp Leu Thr Ser
       385                 390
<210> 2
   <211> 392
   <212> PRT
   <213> Helianthus annus
<400> 2
   Phe Ala Tyr Pro Gly Gly Thr Ser Met Glu Ile His Gln Ala Leu Thr
    1               5                  10                  15
   Arg Ser Ser Thr Ile Arg Asn Val Leu Pro Arg His Glu Gln Gly Gly
               20                  25                  30
   Val Phe Ala Ala Glu Gly Tyr Ala Arg Ala Ser Gly Leu Pro Gly Val
           35                  40                  45
   Cys Ile Ala Thr Ser Gly Pro Gly Ala Thr Asn Leu Val Ser Gly Leu
       50                  55                  60
   Ala Asp Ala Leu Leu Asp Ser Val Pro Met Val Ala Ile Thr Gly Gln
   65                  70                  75                  80
   Val Pro Arg Arg Met Ile Gly Thr Asp Ala Phe Gln Glu Thr Pro Ile
                   85                  90                  95
   Val Glu Val Thr Arg Ser Ile Thr Lys His Asn Tyr Leu Val Leu Asp
               100                 105                 110
   Val Glu Asp Ile Pro Arg Ile Val Arg Glu Ala Phe Tyr Leu Ala Ser
           115                 120                 125
   Ser Gly Arg Pro Gly Pro Val Leu Ile Asp Val Pro Lys Asp Ile Gln
       130                 135                 140
   Gln Gln Leu Val Val Pro Lys Trp Asp Glu Pro Met Arg Leu Pro Gly
   145                 150                 155                 160
   Tyr Leu Ser Arg Met Pro Lys Pro Gln Tyr Asp Gly His Leu Glu Gln
                   165                 170                 175
   Ile Val Arg Leu Val Gly Glu Ala Lys Arg Pro Val Leu Tyr Val Gly
               180                 185                 190
   Gly Gly Cys Leu Asn Ser Asp Asp Glu Leu Arg Arg Phe Val Glu Leu
           195                 200                 205
   Thr Gly Ile Pro Val Ala Ser Thr Leu Met Gly Leu Gly Ala Tyr Pro
       210                 215                 220
   Ala Ser Ser Asp Leu Ser Leu His Met Leu Gly Met His Gly Thr Val
   225                 230                 235                 240
   Tyr Ala Asn Tyr Ala Val Asp Lys Ser Asp Leu Leu Leu Ala Phe Gly
                   245                 250                 255
   Val Arg Phe Asp Asp Arg Val Thr Gly Lys Leu Glu Ala Phe Ala Ser
               260                 265                 270
   Arg Ala Lys Ile Val His Ile Asp Ile Asp Pro Ala Glu Ile Gly Lys
           275                 280                 285
   Asn Lys Gln Pro His Val Ser Ile Cys Gly Asp Ile Lys Val Ala Leu
       290                 295                 300
   Gln Gly Leu Asn Lys Ile Leu Glu Glu Lys Asn Ser Val Thr Asn Leu
   305                 310                 315                 320
   Asp Phe Ser Thr Trp Arg Lys Glu Leu Asp Glu Gln Lys Met Lys Phe
                   325                 330                 335
   Pro Leu Ser Phe Lys Thr Phe Gly Glu Ala Ile Pro Pro Gln Tyr Ala
               340                 345                 350
   Ile Gln Val Leu Asp Glu Leu Thr Gly Gly Asn Ala Ile Ile Ser Thr
           355                 360                 365
   Gly Val Gly Gln His Gln Met Trp Ala Ala Gln Phe Tyr Lys Tyr Asn
       370                 375                 380
   Lys Pro Arg Gln Trp Leu Thr Ser
   385                 390
<210> 3
   <211> 1178
   <212> DNA
   <213> Helianthus annus
<220>
<221> CDS
   <222> (3)...(1178)
<400> 3
   tc ttc gcc tac ccc ggc ggc gcg tca atg gag atc cac caa gct ctc    47
      Phe Ala Tyr Pro Gly Gly Ala Ser Met Glu Ile His Gln Ala Leu
       1               5                  10                  15
   acg cgc tca agc act atc cgc aat gtg ctc ccc cgt cac gaa cag ggc   95
   Thr Arg Ser Ser Thr Ile Arg Asn Val Leu Pro Arg His Glu Gln Gly
                       20                  25                  30
   ggc gtg ttc gcc gcc gaa ggc tac gcg cgc gcc tcc ggt ctt ccc ggc  143
   Gly Val Phe Ala Ala Glu Gly Tyr Ala Arg Ala Ser Gly Leu Pro Gly
                   35                  40                  45
   gtg tgt atc gcc act tcc ggt ccc gga gct acg aac cta gtt agt ggt  191
   Val Cys Ile Ala Thr Ser Gly Pro Gly Ala Thr Asn Leu Val Ser Gly
               50                  55                  60
   ctt gct gac gcg ctg tta gac agt gtc ccc atg gtg gca atc acc ggt  239
   Leu Ala Asp Ala Leu Leu Asp Ser Val Pro Met Val Ala Ile Thr Gly
        65                  70                  75
   caa gtt ccc cgg aga atg atc gga acc gat gcg ttt caa gaa acc cca  287
   Gln Val Pro Arg Arg Met Ile Gly Thr Asp Ala Phe Gln Glu Thr Pro
    80                  85                  90                  95
   att gtt gag gta aca cgt tcg atc act aaa cat aat tat ctt gtg ttg  335
   Ile Val Glu Val Thr Arg Ser Ile Thr Lys His Asn Tyr Leu Val Leu
                       100                 105                 110
   gat gtt gag gat att ccc aga att gtt cgt gag gct ttt tat ctt gcg  383
   Asp Val Glu Asp Ile Pro Arg Ile Val Arg Glu Ala Phe Tyr Leu Ala
                   115                 120                 125
   agt tcg ggt cga ccc ggc ccg gtt ttg ata gat gta ccg aaa gat ata  431
   Ser Ser Gly Arg Pro Gly Pro Val Leu Ile Asp Val Pro Lys Asp Ile
               130                 135                 140
   cag caa cag tta gtg gtg ccg aaa tgg gat gaa ccg atg agg tta ccg  479
   Gln Gln Gln Leu Val Val Pro Lys Trp Asp Glu Pro Met Arg Leu Pro
       145                 150                 155
   ggt tat ttg tct aga atg ccg aag cct caa tat gat ggg cat ttg gaa  527
   Gly Tyr Leu Ser Arg Met Pro Lys Pro Gln Tyr Asp Gly His Leu Glu
   160                 165                 170                 175
   cag att gtt agg ttg gtg ggg gaa gcg aag agg ccg gtt ttg tat gtg  575
   Gln Ile Val Arg Leu Val Gly Glu Ala Lys Arg Pro Val Leu Tyr Val
                       180                 185                 190
   ggt ggt ggg tgt ttg aat tcg gat gat gag ttg agg cgg ttt gtg gag  623
   Gly Gly Gly Cys Leu Asn Ser Asp Asp Glu Leu Arg Arg Phe Val Glu
                   195                 200                 205
   ctt acg ggg att ccg gtt gcg agt act ttg atg ggg ctc gga gcg tac  671
   Leu Thr Gly Ile Pro Val Ala Ser Thr Leu Met Gly Leu Gly Ala Tyr
               210                 215                 220
   cct gct tcg agt gat ttg tcg ctt cat atg ctt ggg atg cat ggt acg  719
   Pro Ala Ser Ser Asp Leu Ser Leu His Met Leu Gly Met His Gly Thr
       225                 230                 235
   gtt tat gcg aat tat gcg gtt gat aag agt gat ttg ttg ctt gcg ttt  767
   Val Tyr Ala Asn Tyr Ala Val Asp Lys Ser Asp Leu Leu Leu Ala Phe
   240                 245                 250                 255
   ggg gtg cgg ttt gat gat cgt gtg acg ggg aag ctt gag gcg ttt gct      815
   Gly Val Arg Phe Asp Asp Arg Val Thr Gly Lys Leu Glu Ala Phe Ala
                   260                 265                 270
   agt agg gcg aag att gtt cat att gat att gat cct gct gaa att ggg      863
   Ser Arg Ala Lys Ile Val His Ile Asp Ile Asp Pro Ala Glu Ile Gly
               275                 280                 285
   aag aat aag cag cct cat gtg tcg att tgt ggt gat att aag gtc gcg      911
   Lys Asn Lys Gln Pro His Val Ser Ile Cys Gly Asp Ile Lys Val Ala
           290                 295                 300
   tta cag ggt ttg aac aag att ttg gag gaa aag aat tcg gtg act aat      959
   Leu Gln Gly Leu Asn Lys Ile Leu Glu Glu Lys Asn Ser Val Thr Asn
       305                 310                 315
   ctt gat ttt tcg acc tgg aga aag gaa ttg gat gaa caa aaa atg aag      1007
   Leu Asp Phe Ser Thr Trp Arg Lys Glu Leu Asp Glu Gln Lys Met Lys
   320                 325                 330                 335
   ttc ccg ttg agc ttt aaa acg ttt ggc gaa gcg att cct cca cag tat      1055
   Phe Pro Leu Ser Phe Lys Thr Phe Gly Glu Ala Ile Pro Pro Gln Tyr
                   340                 345                 350
   gct att caa gtt ctt gat gag tta acg ggc ggg aat gca att att agc      1103
   Ala Ile Gln Val Leu Asp Glu Leu Thr Gly Gly Asn Ala Ile Ile Ser
               355                 360                 365
   acc ggt gtc ggg caa cat cag atg tgg gct gct cag ttt tac aaa tac      1151
   Thr Gly Val Gly Gln His Gln Met Trp Ala Ala Gln Phe Tyr Lys Tyr
           370                 375                 380
   aac aaa cct aga caa tgg ctg acg tcg                                  1178
   Asn Lys Pro Arg Gln Trp Leu Thr Ser
       385                 390
<210> 4
   <211> 392
   <212> PRT
   <213> Helianthus annus
<400> 4
   Phe Ala Tyr Pro Gly Gly Ala Ser Met Glu Ile His Gln Ala Leu Thr
    1               5                  10                  15
   Arg Ser Ser Thr Ile Arg Asn Val Leu Pro Arg His Glu Gln Gly Gly
               20                  25                  30
   Val Phe Ala Ala Glu Gly Tyr Ala Arg Ala Ser Gly Leu Pro Gly Val
           35                  40                  45
   Cys Ile Ala Thr Ser Gly Pro Gly Ala Thr Asn Leu Val Ser Gly Leu
       50                  55                  60
   Ala Asp Ala Leu Leu Asp Ser Val Pro Met Val Ala Ile Thr Gly Gln
   65                  70                  75                  80
   Val Pro Arg Arg Met Ile Gly Thr Asp Ala Phe Gln Glu Thr Pro Ile
                   85                  90                  95
   Val Glu Val Thr Arg Ser Ile Thr Lys His Asn Tyr Leu Val Leu Asp
               100                 105                 110
   Val Glu Asp Ile Pro Arg Ile Val Arg Glu Ala Phe Tyr Leu Ala Ser
           115                 120                 125
   Ser Gly Arg Pro Gly Pro Val Leu Ile Asp Val Pro Lys Asp Ile Gln
       130                 135                 140
   Gln Gln Leu Val Val Pro Lys Trp Asp Glu Pro Met Arg Leu Pro Gly
   145                 150                 155                 160
   Tyr Leu Ser Arg Met Pro Lys Pro Gln Tyr Asp Gly His Leu Glu Gln
                   165                 170                 175
   Ile Val Arg Leu Val Gly Glu Ala Lys Arg Pro Val Leu Tyr Val Gly
               180                 185                 190
   Gly Gly Cys Leu Asn Ser Asp Asp Glu Leu Arg Arg Phe Val Glu Leu
           195                 200                 205
   Thr Gly Ile Pro Val Ala Ser Thr Leu Met Gly Leu Gly Ala Tyr Pro
       210                 215                 220
   Ala Ser Ser Asp Leu Ser Leu His Met Leu Gly Met His Gly Thr Val
   225                 230                 235                 240
   Tyr Ala Asn Tyr Ala Val Asp Lys Ser Asp Leu Leu Leu Ala Phe Gly
                   245                 250                 255
   Val Arg Phe Asp Asp Arg Val Thr Gly Lys Leu Glu Ala Phe Ala Ser
               260                 265                 270
   Arg Ala Lys Ile Val His Ile Asp Ile Asp Pro Ala Glu Ile Gly Lys
           275                 280                 285
   Asn Lys Gln Pro His Val Ser Ile Cys Gly Asp Ile Lys Val Ala Leu
       290                 295                 300
   Gln Gly Leu Asn Lys Ile Leu Glu Glu Lys Asn Ser Val Thr Asn Leu
   305                 310                 315                 320
   Asp Phe Ser Thr Trp Arg Lys Glu Leu Asp Glu Gln Lys Met Lys Phe
                   325                 330                 335
   Pro Leu Ser Phe Lys Thr Phe Gly Glu Ala Ile Pro Pro Gln Tyr Ala
               340                 345                 350
   Ile Gln Val Leu Asp Glu Leu Thr Gly Gly Asn Ala Ile Ile Ser Thr
           355                 360                 365
   Gly Val Gly Gln His Gln Met Trp Ala Ala Gln Phe Tyr Lys Tyr Asn
       370                 375                 380
   Lys Pro Arg Gln Trp Leu Thr Ser
   385                 390
<210> 5
   <211> 2156
   <212> DNA
   <213> Xanthium sp.
<220>
   <221> CDS
   <222> (111)...(2057)
<221> misc_feature
   <222> (0)...(0)
   <223> GenBank Accession No. U16280
<400> 5
   gaacaacagc cacatgtttc tggaccatcg tcgttcacac ctattttaat cagataaaca 60
   aagtacaaac ataacataac ataaccctag tacataacac acattcaaca atg gcg   116
                                                          Met Ala
                                                           1
   gcc atc cct cat aca aac cct tcc atc acc acc aaa cca ccc tca tct   164
   Ala Ile Pro His Thr Asn Pro Ser Ile Thr Thr Lys Pro Pro Ser Ser
           5                   10                  15
   cca cca cgt ccc acc ttc ctc gcc cgt ttc aca ttc cca ata acc tcc   212
   Pro Pro Arg Pro Thr Phe Leu Ala Arg Phe Thr Phe Pro Ile Thr Ser
        20                  25                  30
   act tcc cat aaa cga cac cgt ctc cac atc tcc aac gtc ctc tcc gac   260
   Thr Ser His Lys Arg His Arg Leu His Ile Ser Asn Val Leu Ser Asp
    35                  40                  45                  50
   tcc aaa ccc acc atc acc cat tca cca tta cca acc aaa tca ttt atc   308
   Ser Lys Pro Thr Ile Thr His Ser Pro Leu Pro Thr Lys Ser Phe Ile
                   55                  60                  65
   tcc cgt tac gct cca gac caa cca aga aaa ggc gct gat gtt ctc gtc     356
   Ser Arg Tyr Ala Pro Asp Gln Pro Arg Lys Gly Ala Asp Val Leu Val
               70                  75                  80
   gaa gct ctg gaa cgt gaa ggc gtt aca gac gtc ttc gct tac cca ggt     404
   Glu Ala Leu Glu Arg Glu Gly Val Thr Asp Val Phe Ala Tyr Pro Gly
           85                  90                  95
   ggt gcc tcc atg gag atc cac caa gct ctc acg cgc tca acc acc atc     452
   Gly Ala Ser Met Glu Ile His Gln Ala Leu Thr Arg Ser Thr Thr Ile
       100                 105                 110
   cgc aac gtt ctc cca cgt cac gaa cag ggc ggc gtc ttt gct gcc gaa     500
   Arg Asn Val Leu Pro Arg His Glu Gln Gly Gly Val Phe Ala Ala Glu
   115                 120                 125                 130
   ggc tac gca cgt gcc tcc ggt ctt ccc ggc gtc tgt att gca acc tct     548
   Gly Tyr Ala Arg Ala Ser Gly Leu Pro Gly Val Cys Ile Ala Thr Ser
                   135                 140                 145
   ggt cct gga gct acg aac cta gta agt ggt ctt gct gat gct tta tta     596
   Gly Pro Gly Ala Thr Asn Leu Val Ser Gly Leu Ala Asp Ala Leu Leu
               150                 155                 160
   gac agt gtt cca atg gtt gct att act ggt caa gtt ccc agg aga atg     644
   Asp Ser Val Pro Met Val Ala Ile Thr Gly Gln Val Pro Arg Arg Met
           165                 170                 175
   att gga aca gat gcg ttt caa gaa acc cct att gtt gag gta aca cgt     692
   Ile Gly Thr Asp Ala Phe Gln Glu Thr Pro Ile Val Glu Val Thr Arg
       180                 185                 190
   tcc att act aag cat aat tat tta gtt ttg gat gtc gag gat att ccc     740
   Ser Ile Thr Lys His Asn Tyr Leu Val Leu Asp Val Glu Asp Ile Pro
   195                 200                 205                 210
   agg att gtt agg gaa gct ttt tat ctt gcg tct tct ggt cga ccc gga     788
   Arg Ile Val Arg Glu Ala Phe Tyr Leu Ala Ser Ser Gly Arg Pro Gly
                   215                 220                 225
   ccg gtt tta att gat gta cct aag gat ata cag cag cag ttg gta gtg     836
   Pro Val Leu Ile Asp Val Pro Lys Asp Ile Gln Gln Gln Leu Val Val
               230                 235                 240
   cct aaa tgg gat gag cct att agg tta cct ggg tat ttg tct agg ttc     884
   Pro Lys Trp Asp Glu Pro Ile Arg Leu Pro Gly Tyr Leu Ser Arg Phe
           245                 250                 255
   cct aaa acg gag aat aat ggg cag ttg gaa cag att gtt agg ttg gtg     932
   Pro Lys Thr Glu Asn Asn Gly Gln Leu Glu Gln Ile Val Arg Leu Val
       260                 265                 270
   agt gag gcc aag agg ccg gtt ttg tat gtg ggg ggt ggg tgt ttg aat     980
   Ser Glu Ala Lys Arg Pro Val Leu Tyr Val Gly Gly Gly Cys Leu Asn
   275                 280                 285                 290
   tcg gga gat gag ttg agg cgg ttt gtg gag ctt acg ggg ata ccg gtt     1028
   Ser Gly Asp Glu Leu Arg Arg Phe Val Glu Leu Thr Gly Ile Pro Val
                   295                 300                 305
   gcg agt acg ttg atg ggg ctt gga gcg tac cct gct tct agt gat ttg     1076
   Ala Ser Thr Leu Met Gly Leu Gly Ala Tyr Pro Ala Ser Ser Asp Leu
               310                 315                 320
   tcg ctg cat atg ctt ggg atg cat ggg acc gtt tat gcg aat tat gcg    1124
   Ser Leu His Met Leu Gly Met His Gly Thr Val Tyr Ala Asn Tyr Ala
           325                 330                 335
   gtt gat aag agt gat ttg ttg ctt gcg ttt ggg gta agg ttt gat gac    1172
   Val Asp Lys Ser Asp Leu Leu Leu Ala Phe Gly Val Arg Phe Asp Asp
       340                 345                 350
   cgt gtg acg ggg aag ctt gag gct ttt gct agc aga gct aag att gtt    1220
   Arg Val Thr Gly Lys Leu Glu Ala Phe Ala Ser Arg Ala Lys Ile Val
   355                 360                 365                 370
   cat att gat att gat tct gcg gaa att ggg aag aat aag cag cct cat    1268
   His Ile Asp Ile Asp Ser Ala Glu Ile Gly Lys Asn Lys Gln Pro His
                   375                 380                 385
   gtg tcg att tgt ggt gat att aag gtc gcg tta cag ggt ctg aac aag    1316
   Val Ser Ile Cys Gly Asp Ile Lys Val Ala Leu Gln Gly Leu Asn Lys
               390                 395                 400
   att ttg gag gta aag aat tcg gtg act aat ctt gat ttc tcg aac tgg    1364
   Ile Leu Glu Val Lys Asn Ser Val Thr Asn Leu Asp Phe Ser Asn Trp
           405                 410                 415
   agg aag gaa ttg gat gag caa aag gtt aag tat ccg ttg agt ttt aaa    1412
   Arg Lys Glu Leu Asp Glu Gln Lys Val Lys Tyr Pro Leu Ser Phe Lys
       420                 425                 430
   aca ttt ggc gaa gct att cct ccg cag tat gcc att caa gtg ctt gat    1460
   Thr Phe Gly Glu Ala Ile Pro Pro Gln Tyr Ala Ile Gln Val Leu Asp
   435                 440                 445                 450
   gag tta acg ggt ggg aat gcg att att agc act ggg gtc ggg cag cat    1508
   Glu Leu Thr Gly Gly Asn Ala Ile Ile Ser Thr Gly Val Gly Gln His
                   455                 460                 465
   cag atg tgg gct gct cag ttt tac aaa tac aac aag cct aga caa tgg    1556
   Gln Met Trp Ala Ala Gln Phe Tyr Lys Tyr Asn Lys Pro Arg Gln Trp
               470                 475                 480
   ctg acg tca ggt gga cta ggg gcg atg ggt ttt ggg ttg ccc gct gct    1604
   Leu Thr Ser Gly Gly Leu Gly Ala Met Gly Phe Gly Leu Pro Ala Ala
           485                 490                 495
   atc ggg gcc gct gtt gca aga cct gat gcg gta gta gtt gat atc gat    1652
   Ile Gly Ala Ala Val Ala Arg Pro Asp Ala Val Val Val Asp Ile Asp
       500                 505                 510
   ggt gat gga agc ttt ata atg aac gtt caa gag tta gcc aca atc cgt    1700
   Gly Asp Gly Ser Phe Ile Met Asn Val Gln Glu Leu Ala Thr Ile Arg
   515                 520                 525                 530
   gtt gaa aat ctt cct gtt aag att ttg tta ctt aac aat cag cat ttg    1748
   Val Glu Asn Leu Pro Val Lys Ile Leu Leu Leu Asn Asn Gln His Leu
                   535                 540                 545
   ggt atg gtg gtt cag tgg gag gat cgg ttt tac aag gcg aat cgg gct    1796
   Gly Met Val Val Gln Trp Glu Asp Arg Phe Tyr Lys Ala Asn Arg Ala
               550                 555                 560
   cat acc tac tta gga aat ccg tca aaa gag tct gaa ata ttc cct aac    1844
   His Thr Tyr Leu Gly Asn Pro Ser Lys Glu Ser Glu Ile Phe Pro Asn
           565                 570                 575
   atg ttg aag ttt gct gaa gcg tgt gat atc cca gct gcc cga gtg acc    1892
   Met Leu Lys Phe Ala Glu Ala Cys Asp Ile Pro Ala Ala Arg Val Thr
       580                 585                 590
   cgg aag gca gat cta cga gca gct att cag aag atg ttg gat aca ccg    1940
   Arg Lys Ala Asp Leu Arg Ala Ala Ile Gln Lys Met Leu Asp Thr Pro
   595                 600                 605                 610
   ggg cct tac ttg ttg gat gtg atc gtg ccc cat caa gaa cat gtg ttg    1988
   Gly Pro Tyr Leu Leu Asp Val Ile Val Pro His Gln Glu His Val Leu
                   615                 620                 625
   ccc atg atc ccg gct ggt gga ggt ttc atg gat gtg atc acc gaa ggc    2036
   Pro Met Ile Pro Ala Gly Gly Gly Phe Met Asp Val Ile Thr Glu Gly
               630                 635                 640
   gac ggc aga atg aaa tat tga gcttcaatgt cacatatagt gtgttctgta       2087
   Asp Gly Arg Met Lys Tyr  *
           645
   agcagtttgt cggttatgaa gttaaatgtt ttgttgtgta atttcgttcc tggttaaaaa 2147
   atcaagctt                                                          2156
<210> 6
   <211> 648
   <212> PRT
   <213> Xanthium sp.
<400> 6
   Met Ala Ala Ile Pro His Thr Asn Pro Ser Ile Thr Thr Lys Pro Pro
   1 5 10 15
   Ser Ser Pro Pro Arg Pro Thr Phe Leu Ala Arg Phe Thr Phe Pro Ile
   20 25 30
   Thr Ser Thr Ser His Lys Arg His Arg Leu His Ile Ser Asn Val Leu
   35 40 45
   Ser Asp Ser Lys Pro Thr Ile Thr His Ser Pro Leu Pro Thr Lys Ser
   50 55 60
   Phe Ile Ser Arg Tyr Ala Pro Asp Gln Pro Arg Lys Gly Ala Asp Val
   65 70 75 80
   Leu Val Glu Ala Leu Glu Arg Glu Gly Val Thr Asp Val Phe Ala Tyr
   85 90 95
   Pro Gly Gly Ala Ser Met Glu Ile His Gln Ala Leu Thr Arg Ser Thr
   100 105 110
   Thr Ile Arg Asn Val Leu Pro Arg His Glu Gln Gly Gly Val Phe Ala
   115 120 125
   Ala Glu Gly Tyr Ala Arg Ala Ser Gly Leu Pro Gly Val Cys Ile Ala
   130 135 140
   Thr Ser Gly Pro Gly Ala Thr Asn Leu Val Ser Gly Leu Ala Asp Ala
   145 150 155 160
   Leu Leu Asp Ser Val Pro Met Val Ala Ile Thr Gly Gln Val Pro Arg
   165 170 175
   Arg Met Ile Gly Thr Asp Ala Phe Gln Glu Thr Pro Ile Val Glu Val
   180 185 190
   Thr Arg Ser Ile Thr Lys His Asn Tyr Leu Val Leu Asp Val Glu Asp
   195 200 205
   Ile Pro Arg Ile Val Arg Glu Ala Phe Tyr Leu Ala Ser Ser Gly Arg
   210 215 220
   Pro Gly Pro Val Leu Ile Asp Val Pro Lys Asp Ile Gln Gln Gln Leu
   225 230 235 240
   Val Val Pro Lys Trp Asp Glu Pro Ile Arg Leu Pro Gly Tyr Leu Ser
   245 250 255
   Arg Phe Pro Lys Thr Glu Asn Asn Gly Gln Leu Glu Gln Ile Val Arg
   260 265 270
   Leu Val Ser Glu Ala Lys Arg Pro Val Leu Tyr Val Gly Gly Gly Cys
           275                 280                 285
   Leu Asn Ser Gly Asp Glu Leu Arg Arg Phe Val Glu Leu Thr Gly Ile
       290                 295                 300
   Pro Val Ala Ser Thr Leu Met Gly Leu Gly Ala Tyr Pro Ala Ser Ser
   305                 310                 315                 320
   Asp Leu Ser Leu His Met Leu Gly Met His Gly Thr Val Tyr Ala Asn
                   325                 330                 335
   Tyr Ala Val Asp Lys Ser Asp Leu Leu Leu Ala Phe Gly Val Arg Phe
               340                 345                 350
   Asp Asp Arg Val Thr Gly Lys Leu Glu Ala Phe Ala Ser Arg Ala Lys
           355                 360                 365
   Ile Val His Ile Asp Ile Asp Ser Ala Glu Ile Gly Lys Asn Lys Gln
       370                 375                 380
   Pro His Val Ser Ile Cys Gly Asp Ile Lys Val Ala Leu Gln Gly Leu
   385                 390                 395                 400
   Asn Lys Ile Leu Glu Val Lys Asn Ser Val Thr Asn Leu Asp Phe Ser
                   405                 410                 415
   Asn Trp Arg Lys Glu Leu Asp Glu Gln Lys Val Lys Tyr Pro Leu Ser
               420                 425                 430
   Phe Lys Thr Phe Gly Glu Ala Ile Pro Pro Gln Tyr Ala Ile Gln Val
           435                 440                 445
   Leu Asp Glu Leu Thr Gly Gly Asn Ala Ile Ile Ser Thr Gly Val Gly
       450                 455                 460
   Gln His Gln Met Trp Ala Ala Gln Phe Tyr Lys Tyr Asn Lys Pro Arg
   465                 470                 475                 480
   Gln Trp Leu Thr Ser Gly Gly Leu Gly Ala Met Gly Phe Gly Leu Pro
                   485                 490                 495
   Ala Ala Ile Gly Ala Ala Val Ala Arg Pro Asp Ala Val Val Val Asp
               500                 505                 510
   Ile Asp Gly Asp Gly Ser Phe Ile Met Asn Val Gln Glu Leu Ala Thr
           515                 520                 525
   Ile Arg Val Glu Asn Leu Pro Val Lys Ile Leu Leu Leu Asn Asn Gln
       530                 535                 540
   His Leu Gly Met Val Val Gln Trp Glu Asp Arg Phe Tyr Lys Ala Asn
   545                 550                 555                 560
   Arg Ala His Thr Tyr Leu Gly Asn Pro Ser Lys Glu Ser Glu Ile Phe
                   565                 570                 575
   Pro Asn Met Leu Lys Phe Ala Glu Ala Cys Asp Ile Pro Ala Ala Arg
               580                 585                 590
   Val Thr Arg Lys Ala Asp Leu Arg Ala Ala Ile Gln Lys Met Leu Asp
           595                 600                 605
   Thr Pro Gly Pro Tyr Leu Leu Asp Val Ile Val Pro His Gln Glu His
       610                 615                 620
   Val Leu Pro Met Ile Pro Ala Gly Gly Gly Phe Met Asp Val Ile Thr
   625                 630                 635                 640
   Glu Gly Asp Gly Arg Met Lys Tyr
                   645
<210> 7
   <211> 19
   <212> DNA
   <213> Artificial Sequence
<220>
   <223> Primer HA1U409
<400> 7
   cagacgtgtt ggtggaagc 19
<210> 8
   <211> 21
   <212> DNA
   <213> Artificial Sequence
<220>
   <223> Primer Ha1L1379
<400> 8
   ctgtaacgcg accttaatat c 21
<210> 9
   <211> 22
   <212> DNA
   <213> Artificial Sequence
<220>
   <223> Primer HA1U1313
<400> 9
   tgctgaaatt gggaagaata ag 22
<210> 10
   <211> 19
   <212> DNA
   <213> Artificial Sequence
<220>
   <223> Primer HA1L2131
<400> 10
   tttcgttctg ccatcaccc 19
<210> 11
   <211> 1968
   <212> DNA
   <213> Helianthus annus
<220>
   <221> CDS
   <222> (1)...(1965)
<221> misc_feature
   <222> (0)...(0)
   <223> GenBank Accession No. AY541451
<400> 11
   atg gcg gct cct ccc aac cct tcc atc tcc ttc aaa cca ccg tca ccc    48
   Met Ala Ala Pro Pro Asn Pro Ser Ile Ser Phe Lys Pro Pro Ser Pro
    1               5                  10                  15
   gcc gcc gca ctg cca cca cgc tcc gcc ttc ctc ccc cgt ttc gca tta    96
   Ala Ala Ala Leu Pro Pro Arg Ser Ala Phe Leu Pro Arg Phe Ala Leu
               20                  25                  30
   ccc atc act tcc act acc caa aaa cga cac cgt ctt cac atc tcc aat   144
   Pro Ile Thr Ser Thr Thr Gln Lys Arg His Arg Leu His Ile Ser Asn
           35                  40                  45
   gtt ctc tcc gac tcc aaa tcc acc acc acc acc acc acc acc act caa   192
   Val Leu Ser Asp Ser Lys Ser Thr Thr Thr Thr Thr Thr Thr Thr Gln
       50                  55                  60
   cga ccg tta ccg gtg cag cct ttt gtc tcc cgt tac gcg cca gat caa   240
   Arg Pro Leu Pro Val Gln Pro Phe Val Ser Arg Tyr Ala Pro Asp Gln
   65                  70                  75                  80
   ccg aga aaa ggc gca gac gtg ttg gtg gaa gct ctg gaa cgg gaa ggt   288
   Pro Arg Lys Gly Ala Asp Val Leu Val Glu Ala Leu Glu Arg Glu Gly
                   85                  90                  95
   gtc acc gac gtc ttc gcc tac ccc ggc ggc gcg tca atg gag atc cac    336
   Val Thr Asp Val Phe Ala Tyr Pro Gly Gly Ala Ser Met Glu Ile His
               100                 105                 110
   caa gct ctc acg cgc tca agc act atc cgc aat gtg ctc ccc cgt cac    384
   Gln Ala Leu Thr Arg Ser Ser Thr Ile Arg Asn Val Leu Pro Arg His
           115                 120                 125
   gaa cag ggc ggc gtg ttc gcc gcc gaa ggc tac gcg cgc gcc tcc ggt    432
   Glu Gln Gly Gly Val Phe Ala Ala Glu Gly Tyr Ala Arg Ala Ser Gly
       130                 135                 140
   ctt ccc ggc gtg tgt atc gcc act tcc ggt ccc gga gct acg aac cta    480
   Leu Pro Gly Val Cys Ile Ala Thr Ser Gly Pro Gly Ala Thr Asn Leu
   145                 150                 155                 160
   gtt agt ggt ctt gct gac gcg ctg tta gac agt gtc ccc atg gtg gca    528
   Val Ser Gly Leu Ala Asp Ala Leu Leu Asp Ser Val Pro Met Val Ala
                   165                 170                 175
   atc acc ggt caa gtt ccc cgg aga atg atc gga acc gat gcg ttt caa    576
   Ile Thr Gly Gln Val Pro Arg Arg Met Ile Gly Thr Asp Ala Phe Gln
               180                 185                 190
   gaa acc cca att gtt gag gta aca cgt tcg atc act aaa cat aat tat    624
   Glu Thr Pro Ile Val Glu Val Thr Arg Ser Ile Thr Lys His Asn Tyr
           195                 200                 205
   ctt gtg ttg gat gtt gag gat att ccc aga att gtt cgt gag gct ttt    672
   Leu Val Leu Asp Val Glu Asp Ile Pro Arg Ile Val Arg Glu Ala Phe
       210                 215                 220
   tat ctt gcg agt tcg ggt cga ccc ggc ccg gtt ttg ata gat gta ccg    720
   Tyr Leu Ala Ser Ser Gly Arg Pro Gly Pro Val Leu Ile Asp Val Pro
   225                 230                 235                 240
   aaa gat ata cag caa cag tta gtg gtg ccg aaa tgg gat gaa ccg atg    768
   Lys Asp Ile Gln Gln Gln Leu Val Val Pro Lys Trp Asp Glu Pro Met
                   245                 250                 255
   agg tta ccg ggt tat ttg tct aga atg ccg aag cct caa tat gat ggg    816
   Arg Leu Pro Gly Tyr Leu Ser Arg Met Pro Lys Pro Gln Tyr Asp Gly
               260                 265                 270
   cat ttg gaa cag att gtt agg ttg gtg ggg gaa gcg aag agg ccg gtt    864
   His Leu Glu Gln Ile Val Arg Leu Val Gly Glu Ala Lys Arg Pro Val
           275                 280                 285
   ttg tat gtg ggt ggt ggg tgt ttg aat tcg gat gat gag ttg agg cgg    912
   Leu Tyr Val Gly Gly Gly Cys Leu Asn Ser Asp Asp Glu Leu Arg Arg
       290                 295                 300
   ttt gtg gag ctt acg ggg att ccg gtt gcg agt act ttg atg ggg ctc    960
   Phe Val Glu Leu Thr Gly Ile Pro Val Ala Ser Thr Leu Met Gly Leu
   305                 310                 315                 320
   gga gcg tac cct gct tcg agt gat ttg tcg ctt cat atg ctt ggg atg    1008
   Gly Ala Tyr Pro Ala Ser Ser Asp Leu Ser Leu His Met Leu Gly Met
                   325                 330                 335
   cat ggt acg gtt tat gcg aat tat gcg gtt gat aag agt gat ttg ttg    1056
   His Gly Thr Val Tyr Ala Asn Tyr Ala Val Asp Lys Ser Asp Leu Leu
               340                 345                 350
   ctt gcg ttt ggg gtg cgg ttt gat gat cgt gtg acg ggg aag ctt gag    1104
   Leu Ala Phe Gly Val Arg Phe Asp Asp Arg Val Thr Gly Lys Leu Glu
           355                 360                 365
   gcg ttt gct agt agg gcg aag att gtt cat att gat att gat cct gct    1152
   Ala Phe Ala Ser Arg Ala Lys Ile Val His Ile Asp Ile Asp Pro Ala
       370                 375                 380
   gaa att ggg aag aat aag cag cct cat gtg tcg att tgt ggt gat att    1200
   Glu Ile Gly Lys Asn Lys Gln Pro His Val Ser Ile Cys Gly Asp Ile
   385                 390                 395                 400
   aag gtc gcg tta cag ggt ttg aac aag att ttg gag gaa aag aat tcg    1248
   Lys Val Ala Leu Gln Gly Leu Asn Lys Ile Leu Glu Glu Lys Asn Ser
                   405                 410                 415
   gtg act aat ctt gat ttt tcg acc tgg aga aag gaa ttg gat gaa caa    1296
   Val Thr Asn Leu Asp Phe Ser Thr Trp Arg Lys Glu Leu Asp Glu Gln
               420                 425                 430
   aaa atg aag ttc ccg ttg agc ttt aaa acg ttt ggc gaa gcg att cct    1344
   Lys Met Lys Phe Pro Leu Ser Phe Lys Thr Phe Gly Glu Ala Ile Pro
           435                 440                 445
   cca cag tat gct att caa gtt ctt gat gag tta acg ggc ggg aat gca    1392
   Pro Gln Tyr Ala Ile Gln Val Leu Asp Glu Leu Thr Gly Gly Asn Ala
       450                 455                 460
   att att agc acc ggt gtc ggg caa cat cag atg tgg gct gct cag ttt    1440
   Ile Ile Ser Thr Gly Val Gly Gln His Gln Met Trp Ala Ala Gln Phe
   465                 470                 475                 480
   tac aaa tac aac aaa cct aga caa tgg ctg acg tcg ggc ggg cta ggg    1488
   Tyr Lys Tyr Asn Lys Pro Arg Gln Trp Leu Thr Ser Gly Gly Leu Gly
                   485                 490                 495
   gca atg ggt ttc ggc ctg ccc gct gct atc ggg gcg gcc gtt gca aga    1536
   Ala Met Gly Phe Gly Leu Pro Ala Ala Ile Gly Ala Ala Val Ala Arg
               500                 505                 510
   cct gat gcg gta gta gtt gac atc gac ggt gac gga agc ttt atg atg    1584
   Pro Asp Ala Val Val Val Asp Ile Asp Gly Asp Gly Ser Phe Met Met
           515                 520                 525
   aat gtt caa gag tta gcc aca atc cgt gtt gaa aat ctg ccg gtt aag    1632
   Asn Val Gln Glu Leu Ala Thr Ile Arg Val Glu Asn Leu Pro Val Lys
       530                 535                 540
   att tta tta ctt aac aac cag cat ttg ggt atg gtg gtt cag tgg gag    1680
   Ile Leu Leu Leu Asn Asn Gln His Leu Gly Met Val Val Gln Trp Glu
   545                 550                 555                 560
   gat cgg ttt tac aag gcg aat cgg gct cat acc tac tta gga aac ccg    1728
   Asp Arg Phe Tyr Lys Ala Asn Arg Ala His Thr Tyr Leu Gly Asn Pro
                   565                 570                 575
   tca aaa gag tcg gaa ata ttc cct aac atg gtg aag ttt gct gaa gcc    1776
   Ser Lys Glu Ser Glu Ile Phe Pro Asn Met Val Lys Phe Ala Glu Ala
               580                 585                 590
   tgt gat atc ccg gct gct cga gtg acc caa aag gcg gat cta cga gca    1824
   Cys Asp Ile Pro Ala Ala Arg Val Thr Gln Lys Ala Asp Leu Arg Ala
           595                 600                 605
   gct att cag aag atg ttg gat aca ccc ggg cct tac ttg ttg gat gtg    1872
   Ala Ile Gln Lys Met Leu Asp Thr Pro Gly Pro Tyr Leu Leu Asp Val
       610                 615                 620
   att gtg ccg cat caa gaa cac gtg ttg ccc atg atc ccg gct ggc gga      1920
   Ile Val Pro His Gln Glu His Val Leu Pro Met Ile Pro Ala Gly Gly
   625                 630                 635                 640
   ggt ttc tcg gat gtg atc acc gag ggt gat ggc aga acg aaa tat          1965
   Gly Phe Ser Asp Val Ile Thr Glu Gly Asp Gly Arg Thr Lys Tyr
                   645                 650                 655
   tga                                                                  1968
<210> 12
   <211> 655
   <212> PRT
   <213> Helianthus annus
<400> 12
   Met Ala Ala Pro Pro Asn Pro Ser Ile Ser Phe Lys Pro Pro Ser Pro
   1               5                   10                  15
   Ala Ala Ala Leu Pro Pro Arg Ser Ala Phe Leu Pro Arg Phe Ala Leu
               20                  25                  30
   Pro Ile Thr Ser Thr Thr Gln Lys Arg His Arg Leu His Ile Ser Asn
           35                  40                  45
   Val Leu Ser Asp Ser Lys Ser Thr Thr Thr Thr Thr Thr Thr Thr Gln
       50                  55                  60
   Arg Pro Leu Pro Val Gln Pro Phe Val Ser Arg Tyr Ala Pro Asp Gln
   65                  70                  75                  80
   Pro Arg Lys Gly Ala Asp Val Leu Val Glu Ala Leu Glu Arg Glu Gly
                   85                  90                  95
   Val Thr Asp Val Phe Ala Tyr Pro Gly Gly Ala Ser Met Glu Ile His
               100                 105                 110
   Gln Ala Leu Thr Arg Ser Ser Thr Ile Arg Asn Val Leu Pro Arg His
           115                 120                 125
   Glu Gln Gly Gly Val Phe Ala Ala Glu Gly Tyr Ala Arg Ala Ser Gly
       130                 135                 140
   Leu Pro Gly Val Cys Ile Ala Thr Ser Gly Pro Gly Ala Thr Asn Leu
   145                 150                 155                 160
   Val Ser Gly Leu Ala Asp Ala Leu Leu Asp Ser Val Pro Met Val Ala
                   165                 170                 175
   Ile Thr Gly Gln Val Pro Arg Arg Met Ile Gly Thr Asp Ala Phe Gln
               180                 185                 190
   Glu Thr Pro Ile Val Glu Val Thr Arg Ser Ile Thr Lys His Asn Tyr
           195                 200                 205
   Leu Val Leu Asp Val Glu Asp Ile Pro Arg Ile Val Arg Glu Ala Phe
       210                 215                 220
   Tyr Leu Ala Ser Ser Gly Arg Pro Gly Pro Val Leu Ile Asp Val Pro
   225                 230                 235                 240
   Lys Asp Ile Gln Gln Gln Leu Val Val Pro Lys Trp Asp Glu Pro Met
                   245                 250                 255
   Arg Leu Pro Gly Tyr Leu Ser Arg Met Pro Lys Pro Gln Tyr Asp Gly
               260                 265                 270
   His Leu Glu Gln Ile Val Arg Leu Val Gly Glu Ala Lys Arg Pro Val
           275                 280                 285
   Leu Tyr Val Gly Gly Gly Cys Leu Asn Ser Asp Asp Glu Leu Arg Arg
       290                 295                 300
   Phe Val Glu Leu Thr Gly Ile Pro Val Ala Ser Thr Leu Met Gly Leu
   305                 310                 315                 320
   Gly Ala Tyr Pro Ala Ser Ser Asp Leu Ser Leu His Met Leu Gly Met
                   325                 330                 335
   His Gly Thr Val Tyr Ala Asn Tyr Ala Val Asp Lys Ser Asp Leu Leu
               340                 345                 350
   Leu Ala Phe Gly Val Arg Phe Asp Asp Arg Val Thr Gly Lys Leu Glu
           355                 360                 365
   Ala Phe Ala Ser Arg Ala Lys Ile Val His Ile Asp Ile Asp Pro Ala
       370                 375                 380
   Glu Ile Gly Lys Asn Lys Gln Pro His Val Ser Ile Cys Gly Asp Ile
   385                 390                 395                 400
   Lys Val Ala Leu Gln Gly Leu Asn Lys Ile Leu Glu Glu Lys Asn Ser
                   405                 410                 415
   Val Thr Asn Leu Asp Phe Ser Thr Trp Arg Lys Glu Leu Asp Glu Gln
               420                 425                 430
   Lys Met Lys Phe Pro Leu Ser Phe Lys Thr Phe Gly Glu Ala Ile Pro
           435                 440                 445
   Pro Gln Tyr Ala Ile Gln Val Leu Asp Glu Leu Thr Gly Gly Asn Ala
       450                 455                 460
   Ile Ile Ser Thr Gly Val Gly Gln His Gln Met Trp Ala Ala Gln Phe
   465                 470                 475                 480
   Tyr Lys Tyr Asn Lys Pro Arg Gln Trp Leu Thr Ser Gly Gly Leu Gly
                   485                 490                 495
   Ala Met Gly Phe Gly Leu Pro Ala Ala Ile Gly Ala Ala Val Ala Arg
               500                 505                 510
   Pro Asp Ala Val Val Val Asp Ile Asp Gly Asp Gly Ser Phe Met Met
           515                 520                 525
   Asn Val Gln Glu Leu Ala Thr Ile Arg Val Glu Asn Leu Pro Val Lys
       530                 535                 540
   Ile Leu Leu Leu Asn Asn Gln His Leu Gly Met Val Val Gln Trp Glu
   545                 550                 555                 560
   Asp Arg Phe Tyr Lys Ala Asn Arg Ala His Thr Tyr Leu Gly Asn Pro
                   565                 570                 575
   Ser Lys Glu Ser Glu Ile Phe Pro Asn Met Val Lys Phe Ala Glu Ala
               580                 585                 590
   Cys Asp Ile Pro Ala Ala Arg Val Thr Gln Lys Ala Asp Leu Arg Ala
           595                 600                 605
   Ala Ile Gln Lys Met Leu Asp Thr Pro Gly Pro Tyr Leu Leu Asp Val
       610                 615                 620
   Ile Val Pro His Gln Glu His Val Leu Pro Met Ile Pro Ala Gly Gly
   625                 630                 635                 640
   Gly Phe Ser Asp Val Ile Thr Glu Gly Asp Gly Arg Thr Lys Tyr
                   645                 650                 655
<210> 13
   <211> 2013
   <212> DNA
   <213> Arabidopsis thaliana
<220>
   <221> CDS
   <222> (1)...(2013)
<400> 13
   atg gcg gcg gca aca aca aca aca aca aca tct tct tcg atc tcc ttc   48
   Met Ala Ala Ala Thr Thr Thr Thr Thr Thr Ser Ser Ser Ile Ser Phe
    1               5                  10                  15
   tcc acc aaa cca tct cct tcc tcc tcc aaa tca cca tta cca atc tcc   96
   Ser Thr Lys Pro Ser Pro Ser Ser Ser Lys Ser Pro Leu Pro Ile Ser
               20                  25                  30
   aga ttc tcc ctc cca ttc tcc cta aac ccc aac aaa tca tcc tcc tcc   144
   Arg Phe Ser Leu Pro Phe Ser Leu Asn Pro Asn Lys Ser Ser Ser Ser
           35                  40                  45
   tcc cgc cgc cgc ggt atc aaa tcc agc tct ccc tcc tcc atc tcc gcc   192
   Ser Arg Arg Arg Gly Ile Lys Ser Ser Ser Pro Ser Ser Ile Ser Ala
       50                  55                  60
   gtg ctc aac aca acc acc aat gtc aca acc act ccc tct cca acc aaa   240
   Val Leu Asn Thr Thr Thr Asn Val Thr Thr Thr Pro Ser Pro Thr Lys
    65                 70                  75                  80
   cct acc aaa ccc gaa aca ttc atc tcc cga ttc gct cca gat caa ccc     288
   Pro Thr Lys Pro Glu Thr Phe Ile Ser Arg Phe Ala Pro Asp Gln Pro
                   85                  90                  95
   cgc aaa ggc gct gat atc ctc gtc gaa gct tta gaa cgt caa ggc gta     336
   Arg Lys Gly Ala Asp Ile Leu Val Glu Ala Leu Glu Arg Gln Gly Val
               100                 105                 110
   gaa acc gta ttc gct tac cct gga ggt gca tca atg gag att cac caa     384
   Glu Thr Val Phe Ala Tyr Pro Gly Gly Ala Ser Met Glu Ile His Gln
           115                 120                 125
   gcc tta acc cgc tct tcc tca atc cgt aac gtc ctt cct cgt cac gaa     432
   Ala Leu Thr Arg Ser Ser Ser Ile Arg Asn Val Leu Pro Arg His Glu
       130                 135                 140
   caa gga ggt gta ttc gca gca gaa gga tac gct cga tcc tca ggt aaa     480
   Gln Gly Gly Val Phe Ala Ala Glu Gly Tyr Ala Arg Ser Ser Gly Lys
   145                 150                 155                 160
   cca ggt atc tgt ata gcc act tca ggt ccc gga gct aca aat ctc gtt     528
   Pro Gly Ile Cys Ile Ala Thr Ser Gly Pro Gly Ala Thr Asn Leu Val
                   165                 170                 175
   agc gga tta gcc gat gcg ttg tta gat agt gtt cct ctt gta gca atc     576
   Ser Gly Leu Ala Asp Ala Leu Leu Asp Ser Val Pro Leu Val Ala Ile
               180                 185                 190
   aca gga caa gtc cct cgt cgt atg att ggt aca gat gcg ttt caa gag     624
   Thr Gly Gln Val Pro Arg Arg Met Ile Gly Thr Asp Ala Phe Gln Glu
           195                 200                 205
   act ccg att gtt gag gta acg cgt tcg att acg aag cat aac tat ctt     672
   Thr Pro Ile Val Glu Val Thr Arg Ser Ile Thr Lys His Asn Tyr Leu
       210                 215                 220
   gtg atg gat gtt gaa gat atc cct agg att att gag gaa gct ttc ttt     720
   Val Met Asp Val Glu Asp Ile Pro Arg Ile Ile Glu Glu Ala Phe Phe
   225                 230                 235                 240
   tta gct act tct ggt aga cct gga cct gtt ttg gtt gat gtt cct aaa     768
   Leu Ala Thr Ser Gly Arg Pro Gly Pro Val Leu Val Asp Val Pro Lys
                   245                 250                 255
   gat att caa caa cag ctt gcg att cct aat tgg gaa cag gct atg aga     816
   Asp Ile Gln Gln Gln Leu Ala Ile Pro Asn Trp Glu Gln Ala Met Arg
               260                 265                 270
   tta cct ggt tat atg tct agg atg cct aaa cct ccg gaa gat tct cat     864
   Leu Pro Gly Tyr Met Ser Arg Met Pro Lys Pro Pro Glu Asp Ser His
           275                 280                 285
   ttg gag cag att gtt agg ttg att tct gag tct aag aag cct gtg ttg     912
   Leu Glu Gln Ile Val Arg Leu Ile Ser Glu Ser Lys Lys Pro Val Leu
       290                 295                 300
   tat gtt ggt ggt ggt tgt ttg aat tct agc gat gaa ttg ggt agg ttt     960
   Tyr Val Gly Gly Gly Cys Leu Asn Ser Ser Asp Glu Leu Gly Arg Phe
   305                 310                 315                 320
   gtt gag ctt acg ggg atc cct gtt gcg agt acg ttg atg ggg ctg gga    1008
   Val Glu Leu Thr Gly Ile Pro Val Ala Ser Thr Leu Met Gly Leu Gly
                   325                 330                 335
   tct tat cct tgt gat gat gag ttg tcg tta cat atg ctt gga atg cat    1056
   Ser Tyr Pro Cys Asp Asp Glu Leu Ser Leu His Met Leu Gly Met His
               340                 345                 350
   ggg acg gtg tat gcg aat tac gct gtg gag cat agt gat ttg ttg ttg    1104
   Gly Thr Val Tyr Ala Asn Tyr Ala Val Glu His Ser Asp Leu Leu Leu
           355                 360                 365
   gcg ttt ggg gtg agg ttt gat gat cgc gtc acg ggt aag ctt gag gct    1152
   Ala Phe Gly Val Arg Phe Asp Asp Arg Val Thr Gly Lys Leu Glu Ala
       370                 375                 380
   ttt gct agt agg gct aag att gtt cat att gat att gac tct gct gag    1200
   Phe Ala Ser Arg Ala Lys Ile Val His Ile Asp Ile Asp Ser Ala Glu
   385                 390                 395                 400
   att ggg aag aat aag act cct cat gtg tct gtg tgt ggt gat gtc aag    1248
   Ile Gly Lys Asn Lys Thr Pro His Val Ser Val Cys Gly Asp Val Lys
                   405                 410                 415
   ctg gct ttg caa ggg atg aat aag gtt ctt gag aac cga gct gag gag    1296
   Leu Ala Leu Gln Gly Met Asn Lys Val Leu Glu Asn Arg Ala Glu Glu
               420                 425                 430
   ctt aag ctt gat ttt gga gtt tgg agg aat gag ttg aac gta cag aaa    1344
   Leu Lys Leu Asp Phe Gly Val Trp Arg Asn Glu Leu Asn Val Gln Lys
           435                 440                 445
   cag aag ttt ccg ttg agc ttt aag acg ttt ggg gaa gct att cct cca    1392
   Gln Lys Phe Pro Leu Ser Phe Lys Thr Phe Gly Glu Ala Ile Pro Pro
       450                 455                 460
   cag tat gcg att aag gtc ctt gat gag ttg act gat gga aaa gcc ata    1440
   Gln Tyr Ala Ile Lys Val Leu Asp Glu Leu Thr Asp Gly Lys Ala Ile
   465                 470                 475                 480
   ata agt act ggt gtc ggg caa cat caa atg tgg gcg gcg cag ttc tac    1488
   Ile Ser Thr Gly Val Gly Gln His Gln Met Trp Ala Ala Gln Phe Tyr
                   485                 490                 495
   aat tac aag aag cca agg cag tgg cta tca tca gga ggc ctt gga gct    1536
   Asn Tyr Lys Lys Pro Arg Gln Trp Leu Ser Ser Gly Gly Leu Gly Ala
               500                 505                 510
   atg ggt ttt gga ctt cct gct gcc att gga gcg tct gtt gct aac cct    1584
   Met Gly Phe Gly Leu Pro Ala Ala Ile Gly Ala Ser Val Ala Asn Pro
           515                 520                 525
   gat gca ata gtt gtg gat att gac gga gat gga agc ttt ata atg aat    1632
   Asp Ala Ile Val Val Asp Ile Asp Gly Asp Gly Ser Phe Ile Met Asn
       530                 535                 540
   gtg caa gag ctg gcc aca atc cgt gta gag caa ctt cca gtg aag ata    1680
   Val Gln Glu Leu Ala Thr Ile Arg Val Glu Gln Leu Pro Val Lys Ile
   545                 550                 555                 560
   ctc tta tta aac aac cag cat ctt ggc atg gtt atg caa tgg gaa gat    1728
   Leu Leu Leu Asn Asn Gln His Leu Gly Met Val Met Gln Trp Glu Asp
                   565                 570                 575
   cgg ttc tac aag gct aac cga gct cac aca ttt ctc ggg gat ccg gct    1776
   Arg Phe Tyr Lys Ala Asn Arg Ala His Thr Phe Leu Gly Asp Pro Ala
               580                 585                 590
   cag gag gac gag ata ttc ccg aac atg ttg ctg ttt gca gca gct tgc    1824
   Gln Glu Asp Glu Ile Phe Pro Asn Met Leu Leu Phe Ala Ala Ala Cys
           595                 600                 605
   ggg att cca gcg gcg agg gtg aca aag aaa gca gat ctc cga gaa gct    1872
   Gly Ile Pro Ala Ala Arg Val Thr Lys Lys Ala Asp Leu Arg Glu Ala
       610                 615                 620
   att cag aca atg ctg gat aca cca gga cct tac ctg ttg gat gtg att    1920
   Ile Gln Thr Met Leu Asp Thr Pro Gly Pro Tyr Leu Leu Asp Val Ile
   625                 630                 635                 640
   tgt ccg cac caa gaa cat gtg ttg ccg atg atc ccg agt ggt ggc act    1968
   Cys Pro His Gln Glu His Val Leu Pro Met Ile Pro Ser Gly Gly Thr
                   645                 650                 655
   ttc aac gat gtc ata acg gaa gga gat ggc cgg att aaa tac tga        2013
   Phe Asn Asp Val Ile Thr Glu Gly Asp Gly Arg Ile Lys Tyr  *
               660                 665                 670
<210> 14
   <211> 670
   <212> PRT
   <213> Arabidopsis thaliana
<400> 14
   Met Ala Ala Ala Thr Thr Thr Thr Thr Thr Ser Ser Ser Ile Ser Phe
    1               5                  10                  15
   Ser Thr Lys Pro Ser Pro Ser Ser Ser Lys Ser Pro Leu Pro Ile Ser
               20                  25                  30
   Arg Phe Ser Leu Pro Phe Ser Leu Asn Pro Asn Lys Ser Ser Ser Ser
           35                  40                  45
   Ser Arg Arg Arg Gly Ile Lys Ser Ser Ser Pro Ser Ser Ile Ser Ala
       50                  55                  60
   Val Leu Asn Thr Thr Thr Asn Val Thr Thr Thr Pro Ser Pro Thr Lys
   65                  70                  75                  80
   Pro Thr Lys Pro Glu Thr Phe Ile Ser Arg Phe Ala Pro Asp Gln Pro
                   85                  90                  95
   Arg Lys Gly Ala Asp Ile Leu Val Glu Ala Leu Glu Arg Gln Gly Val
               100                 105                 110
   Glu Thr Val Phe Ala Tyr Pro Gly Gly Ala Ser Met Glu Ile His Gln
           115                 120                 125
   Ala Leu Thr Arg Ser Ser Ser Ile Arg Asn Val Leu Pro Arg His Glu
       130                 135                 140
   Gln Gly Gly Val Phe Ala Ala Glu Gly Tyr Ala Arg Ser Ser Gly Lys
   145                 150                 155                 160
   Pro Gly Ile Cys Ile Ala Thr Ser Gly Pro Gly Ala Thr Asn Leu Val
                   165                 170                 175
   Ser Gly Leu Ala Asp Ala Leu Leu Asp Ser Val Pro Leu Val Ala Ile
               180                 185                 190
   Thr Gly Gln Val Pro Arg Arg Met Ile Gly Thr Asp Ala Phe Gln Glu
           195                 200                 205
   Thr Pro Ile Val Glu Val Thr Arg Ser Ile Thr Lys His Asn Tyr Leu
       210                 215                 220
   Val Met Asp Val Glu Asp Ile Pro Arg Ile Ile Glu Glu Ala Phe Phe
   225                 230                 235                 240
   Leu Ala Thr Ser Gly Arg Pro Gly Pro Val Leu Val Asp Val Pro Lys
                   245                 250                 255
   Asp Ile Gln Gln Gln Leu Ala Ile Pro Asn Trp Glu Gln Ala Met Arg
               260                 265                 270
   Leu Pro Gly Tyr Met Ser Arg Met Pro Lys Pro Pro Glu Asp Ser His
           275                 280                 285
   Leu Glu Gln Ile Val Arg Leu Ile Ser Glu Ser Lys Lys Pro Val Leu
       290                 295                 300
   Tyr Val Gly Gly Gly Cys Leu Asn Ser Ser Asp Glu Leu Gly Arg Phe
   305                 310                 315                 320
   Val Glu Leu Thr Gly Ile Pro Val Ala Ser Thr Leu Met Gly Leu Gly
                   325                 330                 335
   Ser Tyr Pro Cys Asp Asp Glu Leu Ser Leu His Met Leu Gly Met His
               340                 345                 350
   Gly Thr Val Tyr Ala Asn Tyr Ala Val Glu His Ser Asp Leu Leu Leu
           355                 360                 365
   Ala Phe Gly Val Arg Phe Asp Asp Arg Val Thr Gly Lys Leu Glu Ala
       370                 375                 380
   Phe Ala Ser Arg Ala Lys Ile Val His Ile Asp Ile Asp Ser Ala Glu
   385                 390                 395                 400
   Ile Gly Lys Asn Lys Thr Pro His Val Ser Val Cys Gly Asp Val Lys
                   405                 410                 415
   Leu Ala Leu Gln Gly Met Asn Lys Val Leu Glu Asn Arg Ala Glu Glu
               420                 425                 430
   Leu Lys Leu Asp Phe Gly Val Trp Arg Asn Glu Leu Asn Val Gln Lys
           435                 440                 445
   Gln Lys Phe Pro Leu Ser Phe Lys Thr Phe Gly Glu Ala Ile Pro Pro
       450                 455                 460
   Gln Tyr Ala Ile Lys Val Leu Asp Glu Leu Thr Asp Gly Lys Ala Ile
   465                 470                 475                 480
   Ile Ser Thr Gly Val Gly Gln His Gln Met Trp Ala Ala Gln Phe Tyr
                   485                 490                 495
   Asn Tyr Lys Lys Pro Arg Gln Trp Leu Ser Ser Gly Gly Leu Gly Ala
               500                 505                 510
   Met Gly Phe Gly Leu Pro Ala Ala Ile Gly Ala Ser Val Ala Asn Pro
           515                 520                 525
   Asp Ala Ile Val Val Asp Ile Asp Gly Asp Gly Ser Phe Ile Met Asn
       530                 535                 540
   Val Gln Glu Leu Ala Thr Ile Arg Val Glu Gln Leu Pro Val Lys Ile
   545                 550                 555                 560
   Leu Leu Leu Asn Asn Gln His Leu Gly Met Val Met Gln Trp Glu Asp
                   565                 570                 575
   Arg Phe Tyr Lys Ala Asn Arg Ala His Thr Phe Leu Gly Asp Pro Ala
               580                 585                 590
   Gln Glu Asp Glu Ile Phe Pro Asn Met Leu Leu Phe Ala Ala Ala Cys
           595                 600                 605
   Gly Ile Pro Ala Ala Arg Val Thr Lys Lys Ala Asp Leu Arg Glu Ala
       610                 615                 620
   Ile Gln Thr Met Leu Asp Thr Pro Gly Pro Tyr Leu Leu Asp Val Ile
   625                 630                 635                 640
   Cys Pro His Gln Glu His Val Leu Pro Met Ile Pro Ser Gly Gly Thr
                   645                 650                 655
   Phe Asn Asp Val Ile Thr Glu Gly Asp Gly Arg Ile Lys Tyr
               660                 665                 670